(19) Europäisches Patentamt
European Patent Office
Office européen des brevets

(11) EP 0 521 422 B1

(12) **EUROPÄISCHE PATENTSCHRIFT**

(45) Veröffentlichungstag und Bekanntmachung des Hinweises auf die Patenterteilung:
**08.05.1996 Patentblatt 1996/19**

(51) Int. Cl.$^6$: **C07D 221/26**, A61K 31/435

(21) Anmeldenummer: **92110923.7**

(22) Anmeldetag: **27.06.1992**

# (54) Benzomorphane und ihre Verwendung als Arzneimittel

Benzomorphanes and their use as pharmaceuticals

Benzomorphanes et leur utilisation comme médicaments

(84) Benannte Vertragsstaaten:
**AT BE CH DE DK ES FR GB GR IT LI LU NL PT SE**

(30) Priorität: **02.07.1991 DE 4121821**

(43) Veröffentlichungstag der Anmeldung:
**07.01.1993 Patentblatt 1993/01**

(73) Patentinhaber:
- **BOEHRINGER INGELHEIM KG**
  **D-55216 Ingelheim (DE)**
  Benannte Vertragsstaaten:
  **BE CH DE DK ES FR GR IT LI LU NL PT SE AT**
- **BOEHRINGER INGELHEIM INTERNATIONAL GmbH**
  **D-55216 Ingelheim am Rhein (DE)**
  Benannte Vertragsstaaten:
  **GB**

(72) Erfinder:
- **Carter, Adrian, Dr.**
  **W-6530 Bingen (DE)**
- **Ensinger, Helmut, Dr., Dipl.-Chem.**
  **W-6507 Ingelheim am Rhein (DE)**
- **Grauert, Matthias, Dr.,Dipl.-Chem.**
  **W-6507 Ingelheim am Rhein (DE)**
- **Kuhn, Franz Josef, Dr.**
  **W-6535 Gau-Algesheim (DE)**
- **Merz, Herbert, Dr., Dipl.-Chem.**
  **W-6507 Ingelheim am Rheim (DE)**
- **Müller, Enzio, Prof. Dr.**
  **W-6530 Bingen (DE)**
- **Stransky, Werner, Dr., Dipl.-Chem.**
  **W-6535 Gau-Algesheim (DE)**
- **Streller, Ilse, Dr.**
  **W-6534 Stromberg (DE)**

(56) Entgegenhaltungen:
**EP-A- 0 004 960**
**EP-A- 0 006 449**
**EP-A- 0 028 717**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.11.5/3.4

## Beschreibung

Die Erfindung betrifft neue Benzomorphane, Verfahren zu ihrer Herstellung sowie ihre Verwendung als Arzneimittel. Die erfindungsgemäßen Benzomorphane entsprechen der allgemeinen Formel I:

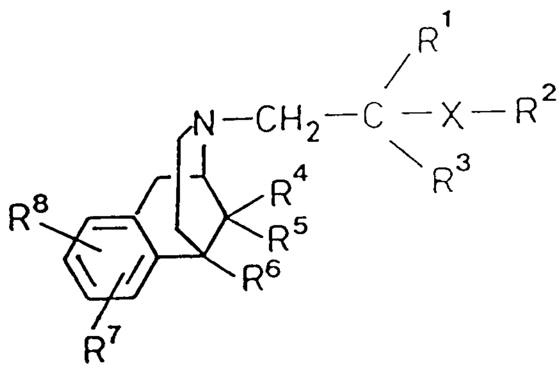

(I)

worin

X Sauerstoff oder Schwefel;

$R^1$ $C_1$-$C_8$-Alkyl, $C_3$-$C_6$-Alkenyl, $C_3$-$C_6$-Alkinyl, einen aromatischen Rest mit 6 bis 10 Kohlerstoffatomen - auch in Zusammensetzungen - wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;

$R^2$ Wasserstoff, $C_1$-$C_8$-Alkyl, $C_3$-$C_6$-Alkenyl, $C_3$-$C_6$-Alkinyl, einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;
einen über eine Alkylenkette gebundenen Arylrest mit 7 bis 14 Kohlenstoffatomen, wobei der Aromat mit einer oder mehreren Niederalkylgruppe(n), Alkoxygruppe(n), Nitrogruppe(n), Aminogruppe(n) und/oder einem oder mehreren Halogenatome(n) - untereinander gleich oder verschieden - substituiert sein kann;

$R^3$ Wasserstoff, $C_1$-$C_6$-Alkyl;

$R^4$ $C_1$-$C_8$-Alkyl;

$R^5$ $C_1$-$C_8$-Alkyl;

$R^6$ $C_1$-$C_8$-Alkyl, einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;

$R^7$ und $R^8$ unabhängig voneinander
Wasserstoff, $C_1$-$C_8$-Alkyl, Halogen,
-OH, $C_1$-$C_8$-Alkoxy,
einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann,
-CN, $-NO_2$,
$NH_2$, -NH($C_1$-$C_8$-Alkyl),
-N($C_1$-$C_8$-Alkyl$)_2$, wobei die
Alkylreste gleich oder verschieden sein können,

-NH-Acyl oder -N-Acyl-($C_1$-$C_8$-Alkyl), worin Acyl für Benzoyl oder für einen Alkylcarbonylrest mit einem geradkettigen oder verzweigten Niederalkylrest mit 1 bis 6 Kohlenstoffatom(en), wobei der Alkylrest gegebenenfalls mit einem oder mehreren Halogenatom(en), die untereinander gleich oder verschieden sein können, substituiert sein kann,

bedeuten können, deren Stereoisomere sowie deren Säureadditionssalze, mit der Maßgabe, daß - im Falle

| | |
|---|---|
| X | Sauerstoff; |
| $R^1$ | $C_1$-$C_3$-Alkyl, $C_3$-Alkenyl, $C_3$-Alkinyl; |
| $R^2$ | Wasserstoff, $C_1$-$C_4$-Alkyl, $C_3$- und $C_4$-Alkenyl; |
| $R^3$ | Wasserstoff, $C_1$-$C_3$-Alkyl; |
| $R^4$ | Methyl; |
| $R^5$ | Methyl; |
| $R^6$ | $C_1$-$C_4$-Alkyl oder Phenyl |

und einer der beiden Substituenten $R^7$ oder $R^8$ Wasserstoff bedeuten - der verbleibende Substituent $R^7$ oder $R^8$ in der 2'-Position nicht die Bedeutung von Wasserstoff, Hydroxy, $C_1$-$C_3$-Alkoxy oder O-Acyl haben darf.

Bevorzugt sind Verbindungen der allgemeinen Formel I, worin

| | |
|---|---|
| X | Sauerstoff oder Schwefel; |
| $R^1$ | Methyl, Ethyl, Propyl, Isopropyl, Phenyl; |
| $R^2$ | Methyl, Ethyl, Propyl, Isopropyl, Allyl, Propargyl, Phenyl, Benzyl; |
| $R^3$ | Wasserstoff, $C_1$-$C_4$-Alkyl; |
| $R^4$ | Methyl, Ethyl, Propyl, Isopropyl; |
| $R^5$ | Methyl, Ethyl, Propyl, Isopropyl; |
| $R^6$ | Methyl, Ethyl, Propyl, Isopropyl, Phenyl; |
| $R^7$ | Fluor, Chlor, Hydroxy, Niederalkyl, $C_1$-$C_3$-Alkoxy, einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann; |
| $R^8$ | Wasserstoff, Niederalkyl, Hydroxy oder $C_1$-$C_8$-Alkoxy |

bedeuten können, deren Stereoisomere sowie deren Säureadditionssalze.

Besonders bevorzugt sind Verbindungen der allgemeinen Formel I, worin

| | |
|---|---|
| X | Sauerstoff, |
| $R^1$ | Methyl, Ethyl; |
| $R^2$ | Methyl; Ethyl; |
| $R^3$ | Wasserstoff; |
| $R^4$ | Methyl, Ethyl; |
| $R^5$ | Methyl, Ethyl; |

$R^6$ Methyl, Ethyl;

$R^7$ Hydroxy, Methyl, Methoxy,
einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann;

$R^8$ Wasserstoff, Methyl, Ethyl, Hydroxy oder $C_1$-$C_3$-Alkoxy

bedeuten können, deren Stereoisomere sowie deren Säureadditionssalze.

Ganz besonders bevorzugt sind Verbindungen der allgemeinen Formel I, worin

X Sauerstoff;

$R^1$ Methyl;

$R^2$ Methyl;

$R^3$ Wasserstoff;

$R^4$ Methyl;

$R^5$ Methyl;

$R^6$ Methyl;

$R^7$ Hydroxy, Methyl, Methoxy, Acetoxy;

$R^8$ Wasserstoff, Methyl, Hydroxy, Methoxy oder Ethoxy

bedeuten können und wobei sich der Substituent $R^7$ in 3'-Stellung und der Substituent $R^8$ in 2'-Stellung befindet und das 2"-Kohlenstoffatom eine R-Konfiguration aufweist sowie deren Säureadditionssalze.

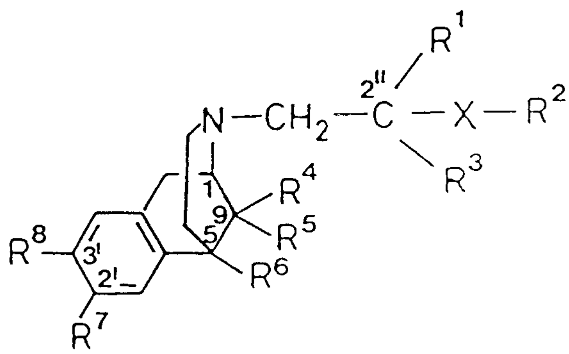

Gegenstand der Erfindung sind die einzelnen Isomeren, deren Mischungen sowie die entsprechenden physiologisch geeigneten Säureadditionssalze mit anorganischen oder organischen Säuren. Bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Soweit nicht im einzelnen abweichende Angaben gemacht werden, werden die allgemeinen Definitionen im folgenden Sinn gebraucht:

$C_1$-$C_6$-Alkyl bzw. $C_1$-$C_8$-Alkyl steht im allgemeinen für einen verzweigten oder unverzweigten Kohlenwasserstoffrest mit 1 bis 6 bzw. 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

Methyl, Ethyl, Propyl, 1-Methylethyl (Isopropyl), Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbuty, 1-Ethylbuty, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 3 Kohlenstoffatomen, wie Methyl, Ethyl, Propyl, Isopropyl.

Alkenyl steht im allgemeinen für einen geradkettigen oder verzweigten Kohlenwasserstoffrest mit 3 bis 6 Kohlenstoffatomen und mit einer oder mehreren, bevorzugt mit einer Doppelbindung, der gegebenenfalls mit einem Halogenatom oder mehreren Halogenatomen - vorzugsweise Fluor - substituiert sein kann, die untereinander gleich oder verschieden sein können.

Als Beispiele seien genannt:
2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl.

Bevorzugt ist der Allylrest.

Alkinyl steht im allgemeinen für einen geradkettigen oder verzweigten Kohlenwasserstoffrest mit 3 bis 6 Kohlenstoffatomen und mit einer oder mehreren Dreifachbindung(en).

Bevorzugt ist ein Niederalkinylrest (Propargyl) mit 3 Kohlenstoffatomen und einer Dreifachbindung, der gegebenenfalls mit einem Halogenatom - vorzugsweise Fluor - oder mehreren Halogenatomen substituiert sein kann, die untereinander gleich oder verschieden sein können.

Acyl steht im allgemeinen für Benzoyl oder für Alkylcarbonylreste - wie geradkettiges oder verzweigtes Niederalkyl mit 1 bis etwa 6 Kohlenstoffatom(en), die über eine Carbonylgruppe gebunden sind, wobei der Alkylrest gegebenenfalls mit einem oder mehreren Halogenatom(en), die untereinander gleich oder verschieden sein können, substituiert sein kann. Bevorzugt sind Alkylreste mit bis zu 4 Kohlenstoffatomen. Als Beispiele seien genannt: Acetyl, Trifluoracetyl, Ethylcarbonyl, Propylcarbonyl, Isopropylcarbonyl, Butylcarbonyl sowie Isobutylcarbonyl. Besonders bevorzugt ist der Acetylrest.

Acyloxy steht für eine über ein Sauerstoff gebundene Acylgruppe, wobei Acyl die o.a. Bedeutung besitzt.

Aryl steht im allgemeinen für einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden - substituiert sein kann. Bevorzugter Arylrest ist Phenyl.

Aralkyl steht im allgemeinen für einen über eine Alkylenkette gebundenen Arylrest mit 7 bis 14 Kohlenstoffatomen, wobei der Aromat mit einer oder mehreren Niederalkylgruppe(n), Alkoxygruppe(n), Nitrogruppe(n), Aminogruppe(n) und/oder einem oder mehreren Halogenatom(en) - untereinander gleich oder verschieden - substituiert sein kann. Bevorzugt werden Aralkylreste mit 1 bis 6 Kohlenstoffatom(en) im aliphatischen Teil und 6 Kohlenstoffatomen im aromatischen Teil. Als bevorzugte Aralkylreste seien - sofern nicht anders angegeben - Benzyl, Phenethyl und Phenylpropyl genannt.

Alkoxy steht im allgemeinen für einen über ein Sauerstoffatom gebundenen geradkettigen oder verzweigten Kohlenwasserstoffrest mit 1 bis 8 Kohlenstoffatom(en). Bevorzugt ist ein Niederalkoxyrest mit 1 bis 3 Kohlenstoffatom(en). - Besonders bevorzugt ist die Methoxygruppe.

Amino steht - sofern nicht anders angegeben - für eine $NH_2$-Funktion, die gegebenenfalls durch eine oder zwei $C_1$-$C_8$-Alkyl-, Aryl- oder Aralkylreste - gleich oder verschieden - substituiert sein kann.

Alkylamino steht zum Beispiel für Methylamino, Ethylamino, Propylamino, 1-Methylethylamino, Butylamino, 1-Methylpropylamino, 2-Methylpropylamino oder 1,1-Dimethylethylamino.

Dialkylamino steht beispielsweise für Dimethylamino, Diethylamino, Dipropylamino, Dibutylamino, Di-(1-methylethyl)amino, Di-(1-methylpropyl)amino, Di-2-methylpropylamino, Ethylmethylamino, Methylpropylamino.

Halogen steht - sofern nicht anders angegeben - in erster Linie für Fluor, Chlor und Brom und in zweiter Linie für Jod.

Einige der unter die allgemeine Formel I fallenden Benzomorphanderivate sind aus der europäischen Patentanmeldung Nr. 0 004 960 bekannt. Diese Offenlegungsschrift betrifft bestimmte 6,7-Benzomorphanderivate haben eine analgetische Eigenschaft und/oder die Eigenschaften eines Morphin-Antagonisten.

1. Pharmakologische Charakterisierung

Es ist bekannt, daß nach systemischer Applikation von Glutamat Neuronen im Gehirn von Mäusen zerstört werden [S.M. Rothman und T.W. Olney, Trends in Neurosciences 10 (1987) 299]. Dieser Befund läßt unter anderem den Schluß

zu, daß Glutamat eine Rolle bei neurodegenerativen Erkrankungen spielt [R. Schwarcz und B. Meldrum, The Lancet 11 (1985) 140].

Weiterhin sind Substanzen wie z.B. Quisqualinsäure, Kainsäure, Ibotensäure, Glutaminsäure und N-Methyl-D-asparaginsäure (NMDA) als exogene bzw. endogene Neurotoxine bekannt. Hinsichtlich der Neurotoxizität wirken diese Substanzen bezüglich der einzelnen Zelltypen selektiv, so daß bei Tieren durch spezifische Gehirnläsionen Funktionsausfälle induziert werden können. Diese sind mit jenen vergleichbar, welche im Zusammenhang mit Epilepsie und anderen neurodegenerativen Erkrankungen - wie zum Beispiel Morbus Huntington und Morbus Alzheimer - auftreten.

Ferner haben in vivo und in vitro Versuche gezeigt, daß die im Gehirn infolge von Hypoglykämie, Hypoxie, Anoxie und Ischämie auftretenden Zellschäden und Funktionsausfälle z.T. auf einer erhöhten synaptischen Aktivität beruhen, wobei die glutamaterge Synapse von besonderer Bedeutung ist. Substanzen und Ionen, welche die Aktivität des Glutamat-Rezeptors und des mit diesem Rezeptor verbundenen Ionenkanals hemmen - wie z.B. kompetitive und nicht-kompetitive Antagonisten excitatorischer Aminosäuren sowie Magnesium-Ionen ($Mg^{2+}$) - schützen Gehirnzellen vor hypoxischen bzw. ischämischen Schäden. Diese Befunde zeigen, daß der Glutamat-Rezeptor eine wichtige Rolle bei der Vermittlung des ischämischen Schadens spielt.

Biochemische und elektrophysiologische Studien lehren, daß der Rezeptor-Ionenkanal gegenüber Schwankungen der Magnesiumkonzentration hochempfindlich ist. Bei einem Abfallen der Magnesiumkonzentration kommt es im Hippocampus zu spontanen epileptischen Nachentladungen, die durch Antagonisten excitatorischer Aminosäuren gehemmt werden können.

Überraschenderweise wurde nun gefunden, daß Benzomorphane der allgemeinen Formel I den NMDA-Kanal blokkieren und eine neuroprotektive Wirkung aufweisen.

Die Herstellung dieser Benzomorphanderivate ist aus der DE-PS 21 05 743 und der DE-OS 28 28 039 sowie aus der Literatur [H. Merz und K. Stockhaus, J. Med. Chem. 22 (1979) 1475] bekannt. Ebenso ist bereits bekannt, daß derartige Verbindungen eine analgetische Wirksamkeit besitzen und als suchtfreie Analgetika sowie als Antitussiva therapeutisch angewandt werden können (DE-PS 21 05 743).

Als Testsystem für den Nachweis der NMDA-antagonistischen Wirkung der Benzomorphanderivate dient der Hippocampusschnitt. Über Mikroelektroden werden die Schäfferkollaterale des sich in einer Perfusionskammer befindenden Hippocampusschnittes stimuliert und die auftretenden Summenpotentiale extrazellulär an den Pyramidenzellen der CAI-Region abgeleitet [H.L. Haas, B. Schaerer und M. Vosmansky, J. Neuroscience Meth. 1 (1979) 323].

Die neuroprotektive Wirkung der Benzomorphanderivate, die unter die allgemeine Formel I fallen, wurde außerdem auch in Bezug auf Proteinsynthese und Neurotransmitter-Freisetzung im Hippocampusschnitt nachgewiesen.

Rezeptorbindungstests zeigen weiterhin, daß die offenbarten Benzomorphanderivate nicht-kompetitive Glutamat-Rezptorantagonisten sind.

Weiterhin wurde die neuroprotektive Wirkung der Benzomorphanderivate der allgemeinen Formel I an der Maus - in vivo - durch Hemmung der N-Methyl-D-asparaginsäure induzierten Letalität nachgewiesen [J.D. Leander et al., Brain Research 448 (1988) 115] und durch Hemmung des - durch Ischämie verursachten neuronalen Zelltodes an der Maus und Wüstenspringmaus (Gerbil).

Diese Ergebnisse liefern einen Beleg dafür, daß die Benzomorphanderivate der allgemeinen Formel I bei neurodegenerativen Erkrankungen sowie Gehirnischämie verschiedener Genese eingesetzt werden können. Hierunter fallen beispielsweise:
Status epileptikus, Hypoglykämie, Hypoxie, Anoxie, Gehirntrauma, Gehirnoedem, amyotrope laterale Sklerose, Huntington's Disease, Morbus Alzheimer, Hypotonie, Herzinfarkt, Gehirn-Schlaganfall und perinatale Asphyxie.

2. Formulierung

Die Benzomorphanderivate der allgemeinen Formel I sowie ihre Säureadditionssalze mit pharmakologisch unbedenklichen Säuren können in bekannter Weise in die üblichen Formulierungen - wie Tabletten, Dragees, Pillen, Granulate, Aerosole, Siruppe, Emulsionen, Suspensionen und Lösungen unter Verwendung inerter pharmazeutisch geeigneter Trägerstoffe oder Lösungsmittel überführt werden. Hierbei soll der Anteil der pharmazeutisch wirksamen Verbindung(en) jeweils im Bereich von 0,5 bis 90 Gew.-% der Gesamtzusammensetzung liegen, d.h. in Mengen, die ausreichend sind, um den unten angegebenen Dosierungsbereich zu erreichen.

Die Formulierungen werden zum Beispiel durch Verstrecken der Wirkstoffe mit Lösungsmitteln und/oder Trägerstoffen, gegebenenfalls unter Verwendung von Emulgiermitteln und/oder Dispergiermitteln hergestellt, wobei beispielsweise bei der Verwendung von Wasser als Verdünnungsmittel gegebenenfalls organische Lösungsmittel als Lösungsvermittler bzw. Hilfslösungsmittel eingesetzt werden können.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise parental - insbesondere auf dem Wege der Infusion - intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die orale Anwendung liegt bei 1 - 300 mg vorzugsweise zwischen 5 und 150 mg.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ferner können die Verbindungen der allgemeinen Formel I bzw. deren Säureadditionssalze auch mit andersartigen Wirkstoffen kombiniert werden.

<u>Formulierungsbeispiele</u>

<u>Tabletten</u>

1. Die Tablette enthält folgende Bestandteile:

| | |
|---|---|
| Wirkstoff gemäß Formel I | 0,020 Teile |
| Stearinsäure | 0,010 Teile |
| Dextrose | 1,890 Teile |
| gesamt | 1,920 Teile |

<u>Herstellung</u>

Die Stoffe werden in bekannter Weise zusammengemischt und die Mischung zu Tabletten verpreßt, von denen jede 1,92 g wiegt und 20 mg Wirkstoff enthält.

<u>Amupullenlösung</u>

Zusammensetzung

| | |
|---|---|
| Wirkstoff gemäß Formel I | 1,0 mg |
| Natriumchlorid | 45,0 mg |
| Aqua pro inj. | ad 5,0 ml |

<u>Herstellung:</u>

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 10 mg Wirkstoff.

Suppositorien

Jedes Zäpfchen enthält:

| | |
|---|---|
| Wirkstoff gemäß Formel I | 1,0 Teile |
| Kakaobutter (Schmp.: 36-37°C) | 1200,0 Teile |
| Carnaubawachs | 5,0 Teile |

Herstellung

Kakaobutter und Carnaubawachs werden zusammengeschmolzen. Bei 45°C gibt man den Wirkstoff hinzu und rührt bis eine komplette Dispersion entstanden ist. Die Mischung wird in Form entsprechender Größe gegoßen und die Zäpfchen zweckmäßig verpackt.

3. Syntheseverfahren

Die Herstellung der den verschiedenen Syntheseverfahren als Ausgangsmaterial dienenden Bezomorphanderivate vom Typ 2 ist entweder aus dem Stand der Technik bekannt [DE-A 20 27 077, CA 74 (1971) 125482x; EP-B 4960, CA 93 (1980) 4941 f] oder wird in den folgenden Beispielen beschrieben.

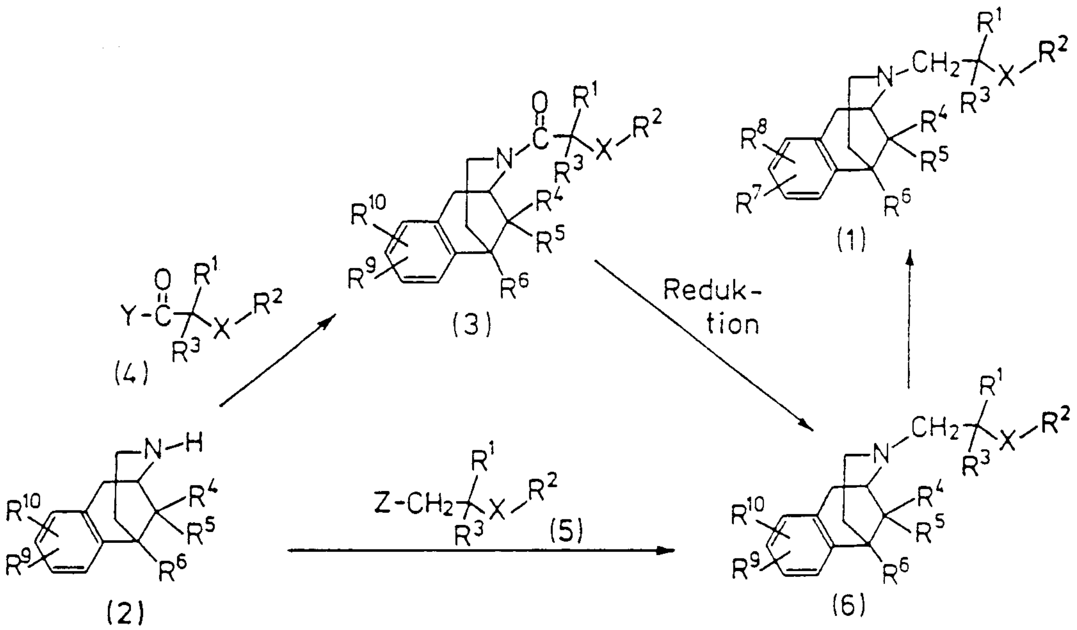


Dabei sind die Substituenten $R^9$ und $R^{10}$ entweder mit den gewünschten Substituenten $R^7$ und $R^8$ identisch oder werden auf einer späteren Synthesestufe in diese überführt.

3.1a

Die Einführung eines gewünschten Substituenten an der Aminofunktion des Benzomorphan-Stickstoffs kann zum einen auf dem Wege der Acylierung mit einem geeignet aktivierten Carbonsäurederivat erfolgen. Entsprechende Carbonsäurederivate vom Typ 4 sind aus dem Stand der Technik bekannt oder sind auf dem Wege gängiger Syntheseverfahren leicht zugänglich.

Für die Acylierung selbst stehen wiederum eine Vielzahl von Verfahren zur Auswahl [C. Ferri, Reaktionen der organischen Synthese, Georg Thieme Verlag Stuttgart, 1978, S. 222 ff. und zit. Lit. J. March, Advanced Organic Chemistry, 3rd Ed., John Wiley and Sons, New York 1985, S. 370 ff und zit. Lit.; R.C. Larock,

Comprehensive Organic Transformations - A Guide to Functional Group Preparations, VCH Verlagsgesellschaft, D-6940 Weinheim 1989, S. 963 ff. und zit. Lit., wobei Umsetzungen mit Carbonsäurehalogeniden [A.L.J. Beckwith in J. Zabicki, "The Chemistry of Amides", Interscience, New York 1970 S. 73] in einem unter den herrschenden Reaktionsbedingungen im wesentlichen inerten Lösungsmitteln - gegebenenfalls in Gegenwart von säurebindenden Agenzien - bevorzugt werden. Als inerte Lösungsmittel werden im allgemeinen organische Solvenzien eingesetzt, die sich unter den angewandten Rekationsbedingungen nicht verändern, wie Kohlenwasserstoffe - wie z.B. Benzol, Toluol, Xylol - oder Erdölfraktionen oder Ether - wie z.B. Diethylether, Glykoldimethylether (Glyme), Diglykoldimethylether - oder cyclische Ether - wie z.B. Tetrahydrofuran oder Dioxan - oder Halogenkohlenwasserstoffe - wie z.B. Tetrachlorkohlenstoff, Chloroform oder Dichlormethan.

Die Herstellung der gewünschten Carbonsäurehalogenide ist aus dem Stand der Technik bekannt [Houben-Weyl, Methoden der organischen Chemie, Band VIII und Band E5, Georg Thieme Verlag, Stuttgart, 1952 bzw. 1985] soweit sie nicht ohnehin kommerziell erhältlich sind. Zweckmäßigerweise wird dabei das Benzomorphanderivat vom Typ 2 bevorzugt in halogenierten Kohlenwasserstoffen - besonders bevorzugt in Dichlormethan - und in Gegenwart von tertiären Aminen - wie z.B. Triethylamin - mit dem gewünschten Säurehalogenid - besonders bevorzugt mit dem gewünschten Säurechlorid - umgesetzt. Es ist jedoch auch möglich, die Umsetzung - in Anlehnung an die sog. Schotten-Baumann-Variante - in Wasser oder in einem wäßrigen Alkohol in Gegenwart von Alkalihydroxiden oder Alkalicarbonaten durchzuführen [s.z.B. Organikum, Autorenkollektiv, VEB Deutscher Verlag der Wissenschaften, 17. Auflage, Berlin 1988 S. 407]. In Abhängigkeit von den eingesetzten Edukten kann es sich des weiteren als besonders vorteilhaft erweisen, die Acylierung nach der Variante von Einhorn durchzuführen, wobei Pyridin sowohl als säurebindendes Agenz sowie als Reaktionsmedium eingesetzt wird.

Daneben besteht die Möglichkeit, die Acylierungsreaktion mit der jeweiligen freien Carbonsäure durchzuführen [s.z.B.: A.L.J. Beckwith in J. Zabicki, "The Chemistry of Amides", Interscience, New York 1970, S. 105 ff.; J.A. Mitchell und E.E. Reid, J. Am. Chem. Soc. 53 (1931) 1879]. Des weiteren kann es sich als zweckmäßig erweisen, statt der freien Carbonsäure ein gemischtes Anhydrid - z.B. mit einem Kohlensäureester - einzusetzen [C. Ferri, Reaktionen der organischen Synhtese, Georg Thieme Verlag, Stuttgart 1978, S. 222 und zit Lit.; A.L.J. Beckwith in J. Zabicki, "The Chemistry of Amides", Interscience, New York 1970, S. 86; J. March, Advanced Organic Chemistry, 3rd Ed., John Wiley and Sons, New York 1985, S. 371 und zit. Lit., R.C. Larock, Comprehensive Organic Transformations, VCH Publishers, D-6940 Weinheim 1989, S. 981 und zit. Lit.].

Bei der bevorzugten Acylierung mit Carbonsäurehalogeniden - insbesondere Carbonsäurechloriden - kann die Reaktionstemperatur in weiten Grenzen variiert werden, die nach unten durch zu geringe Reaktionsgeschwindigkeiten und nach oben durch das Überhandnehmen von unerwünschten Nebenreaktionen gesteckt werden. Bei der Durchführung haben sich Reaktionstemperaturen im Bereich von -50°C bis 150°C - bevorzugt 0°C bis 75°C - bewährt. Dabei arbeitet man zweckmäßigerweise mit einem geringen Überschuß des Acylierungsmittel in Gegenwart eines - in etwas größerem Überschuß vorhandenem - säurebindenden Mittels, um eine möglichst vollständige Umsetzung der Edukte zu gewährleisten.

3.1b

Um zum gewünschten Amin vom Typ 6 zu gelangen, ist im anschließenden Reaktionsschritt die Reduktion des Säureamids 3 erforderlich.

Derartige Reduktionen von Säureamiden sind aus dem Stand der Technik bekannt und können auf dem Wege der elektrolytischen Reduktion, durch Reduktion mit Alkalimetallen sowie durch katalytische Reduktion [R. Schröter in Houben-Weyl, Methoden der organischen Chemie, Band XI/1, Georg Thieme Verlag, Stuttgart 1957, S. 574] oder mit Diboran bzw. Borwasserstoff-Derivaten [J. Fuhrhop und G. Penzlin, Organic Synthesis - Concepts - Methods - Starting Materials, VCH-Verlagsgesellschaft, Weinheim 1986, S. 90] erfolgen.

Bevorzugt wird die Reduktion mit komplexen Hydriden, wie Alkalibor- oder Alkalialuminiumhydriden bzw. mit deren geeigneten Derivaten - gegebenenfalls in Gegenwart eines Katalysators - [N.G. Gaylord, Reduction with Complex Metal Hydrides, Wiley New York 1965; A. Hàjos, Complex Hydrides, Elsevier New York 1979; V. Bažant, M. čapka, M. černy, V. Chvalovsky, K. Kochloefl, M. Kraus und J. Màlek, Tetrahedron Lett. 9 -(1968) 3303], wobei Lithiumaluminiumhydrid besonders bevorzugt wird.

Als Reaktionsmedien eignen sich hierbei alle inerten organischen Lösungsmittel, die sich unter den gegebenen Reaktionsbedingungen nicht verändern. Hierzu gehören bevorzugt Ether wie z.B. Diethylether, Diisopropylether, tert.-Butylmethylether, Di-n-butylether, Glykoldimethylether (Glyme), Diglykoldimethylether (Diglyme), cyclische Ether - wie z.B. Dioxan - und besonders bevorzugt Tetrahydrofuran, wobei sich die Auswahl des Lösungsmittels unter anderem nach der Art des eingesetzten Reduktionsmittels richtet.

Dabei ist es in der Regel vorteilhaft, derartige Reduktionen in Gegenwart eines Überschusses des Reduktionsmittels, welches vorzugsweise eines der zuvorgenannten komplexen Hydride verkörpert - insbesondere Lithiumalanat - ablaufen zu lassen, der in einem Bereich von 5 bis 100 % - vorzugsweise in einem Bereich zwischen 10 bis 50 % - liegt.

Die Reaktionspartner werden üblicherweise unter Eiskühlung oder bei Raumtemperatur zusammengegeben und anschließend - in Abhängigkeit von der Reaktivität der Edukte - auf Temperaturen im Bereich von 150°C - vorzugsweise bis 75°C - erwärmt.

3.2

Eine weitere Möglichkeit zur Herstellung von Benzomorphanderivaten des Typs 6 besteht in der Umsetzung des Benzomorphanabkömmlings 2 mit geeigneten Alkylierungsmitteln vom Typ 5, wobei in der Formel 5 Z eine bei der Alkylierungsgruppe austretende Fluchtgruppe (Leaving group) darstellt. Bevorzugte Austrittsgruppen sind Halogen - wie z.B. Cl, Br, I - oder O-$SO_2$-Aryl - wie z.B. Tosylat - oder ein Alkylsulfonat O-$SO_2$-Alkyl - wie z.B. oder Methansulfonat - oder Halogenmethansulfonat oder Sulfat. Entsprechende Alkylierungsreagenzien sind entweder kommerziell erhältlich oder deren Herstellung ist aus dem Stand der Technik bekannt.

Als Lösungsmittel eigenen sich alle inerten Lösungsmittel, die sich unter den gegebenen Reaktionsbedingungen im wesentlichen nicht verändern und die selbst nicht als reaktionsfähige Komponenten nicht nachteilig das Reaktionsgeschehen beeiflussen können. Hierzu gehören bevorzugt z.B. Alkohole - wie Methanol, Ethanol, Propanol oder Isopropanol - oder Ether - wie Diethylether, Di-n-butylether, tert.-Butylmethylether, Glykoldimethylether (Glyme), Diethylenglykoldimethylether (Diglyme), Tetrahydrofuran und Dioxan - oder Ketone - wie z.B. Methylethylketon oder Aceton - oder Säureamide - wie z.B. Hexanmethylphosphorsäuretriamid oder Dimethylformamid.

Außerdem ist es möglich, Gemische der genannten Lösungsmittel zu verwenden. Besonders bevorzugt werden Tetrahydrofuran oder Dimethylformamid oder Mischungen dieser beiden Lösungsmittel. Die Reaktion wird vorzugsweise in Gegenwart von säurebindenden Agenzien - wie z.B. Alkali- oder Erdalkalicarbonaten oder -hydrogencarbonaten - durchgeführt.

Die Reaktionstemperatur läßt sich bei der Reaktionsführung in weiten Grenzen variieren, die für die praktische Durchführung nach unten durch zu geringe Reaktionsgeschwindigkeiten und nach oben durch ein Überhandnehmen von Nebenreaktionen gesteckt werden. Geeignete Reaktionstemperaturen liegen in einem Intervall von 0°C bis 150°C und - bevorzugt - zwischen 50°C und 100°C.

3.3

Bei der gegebenenfalls notwendigen Umwandlung von $R^9$ und/oder $R^{10}$ in $R^7$ bzw. $R^8$ handelt es sich um viele verschiedenartige Reaktionen, die sich nicht unter einem einheitlichen Schema zusammenfassen lassen. Derartige Umwandlungseaktionen werden durch die entsprechenden Beispiele belegt.

4. Beispiele

Vorbemerkung: Unter Ether ist - wenn nicht anders angegeben - Diethylether zu

verstehen.

4.1 Verbindungen vom Typ (1) - hergestellt nach Verfahren 3.1

4.1.1 Beispiel 1
(-)-(1R,5S,2"R)-2'-Hydroxy-2-(2-methoxypropyl)-5,9,9-trimethyl-6,7-benzomorphan Hydrochlorid

a) Acylierung

27,8 g (0,12 Mol) (±)-2'-Hydroxy-5,9,9-trimethyl-6,7-benzomorphan [DOS 20 27 077; CA 74, 125482x (1971)] werden in 278 ml absolutem Dichlormethan in Gegenwart von 27,9 g (0,275 Mol) Triethylamin unter Rühren innerhalb von ca. 30 Minuten mit 19,2 g (0,156 Mol) (R)-2-Methoxypropionsäurechlorid versetzt. Die Reaktionstemperatur wird dabei im Bereich von 30-35°C gehalten. Anschließend wird eine Stunde unter Rückfluß gekocht. Dann wird abgekühlt und nacheinander mit 175 ml Wasser, 175 ml 2 N Salzsäure und noch zweimal mit je 175 ml Wasser gewaschen. Nach dem Trocknen mit Natriumsulfat und dem Abfiltrieren des Trockenmittels wird die Lösung i. Vak. eingedampft, zuletzt bei 80°C und vollem Wasserstrahlvakkum. Man erhält das Acylierungsprodukt vom Typ (3) als Eindampfungsrückstand in einer Ausbeute von 42,3 g (rund 100 % d. Th.).

b) Reduktion

Das nach dem oben beschriebenen Verfahren hergestellte Acylierungsprodukt wird in 425 ml absolutem Tetrahydrofuran gelöst. Die Lösung wird unter Rühren und Eiskühlung innerhalb von einer Stunde bei 10-15°C in eine Suspension von 9,1 g (0,24 Mol) Lithiumaluminiumhydrid (Lithiumtetrahydridoalanat) in 300 ml Tetrahydrofuran eingetropft. Anschließend wird die Reaktionsmischung zwei Stunden unter Rühren und Rückfluß gekocht. Dann wird unter Kühlung im Eisbad und Rühren tropfenweise mit 25 ml Wasser versetzt und schließlich nach Zugabe von weiteren 70 ml Wasser und gesättigter Diammoniumtartrat-Lösung (890 ml) im Scheidetrichter geschüttelt. Die abgetrennte Tetrahydrofuran-Phase wird i.Vak. eingedampft und die wäßrige Phase dreimal mit je 175 ml Dichlormethan extrahiert. Der Eindampfungsrückstand der Tetrahydrofuran-Phase wird mit den Dichlormethan-Extrakten gelöst und die Lösung zweimal mit je 100 ml Wasser gewaschen. Nach dem Trocknen mit Natriumsulfat und Abfiltrieren des Trockenmittels wird die Lösung eingedampft, zuletzt bei 80°C und vollem Wasserstrahlvakuum. Es verbleibt ein Rückstand (37 g, rund 100 % d. Th.), der aus einem Gemisch der beiden zu erwartenden diastereomeren Verbindungen besteht. Im Dünnschichtchromatogramm (DC) zeigen diese Diasteroemeren $R_f$-Werte von 0,42 bzw. 0,51 (Kieselgel 60, Chloroform-Methanol-konz. Ammoniak 95:5:0,1).

Die Diastereomeren lassen sich durch Kristallisation der Hydrochloride trennen. Dazu löst man das Basengemisch in 120 ml absolutem Ethanol und säuert die Lösung mit Salzsäure (12 ml 32 %-ige Salzsäure) an. Es kristallisiert sofort die Titelverbindung ($R_f$ = 0,42), die nach dem Abkühlen (Eisbad) abgesaugt, portionsweise mit eiskaltem Ethanol (40 ml) gewaschen und, zuletzt bei 80°C bis zur Gewichtskonstanz getrocknet wird. Die Ausbeute beträgt 15,0 g (73,5 % der theoretisch maximal erhältlichen Menge). Die Substanz schmilzt bei 264°C unter Zersetzung und zeigt eine spezifische optische Drehung von $[\alpha]_D^{25}$ = -116,9° (c = 1, $CH_3OH$). Eine aus einer Mischung aus Methanol und Diethylether umkristallisierte Probe schmilzt unverändert bei 264°C (Zers.) und zeigt eine spezifische Drehung von $[\alpha]_D^{25}$ = -118,6° (c = 1, $CH_3OH$).

4.1.2 Beispiel 2
(+)-(1S,5R,2"R)-2'-Hydroxy-2-(2-methoxypropyl)-5,9,9-trimethyl-6,7-benzomorphan Hydrochlorid

Die in dem Verfahren nach Beispiel 1 anfallende Mutterlauge enthält überwiegend das Diastereomere mit dem $R_f$-Wert 0,51. Sie wird eingedampft und der Rückstand (25 g) mit 40 ml Isopropanol eine Stunde unter Rühren auf Rückflußtemperatur erwärmt. Die dabei entstandene Kristallsuspension wird ca. 12 Stunden bei Raumtemperatur stehengelassen, dann abgesaugt und mit wenig kaltem Isopropanol portionsweise gewaschen. Das Kristallisat besteht in der Hauptsache aus der Titelverbindung neben Resten des Diastereomeren (Beispiel 1). Die Titelverbindung wird in die freie Basenform überführt und säulenchromatographisch gereinigt. Dazu schüttelt man das Kristallisat mit Wasser (75 ml), Dichlormethan (75 ml) und überschüssigem konz. Ammoniak (6 ml, 25 %-ig). Nach dem Trennen der Phasen wird die wäßrige Schicht noch einmal mit 25 ml Dichlormethan extrahiert. Die vereinigten Dichlormethan-Extrakte werden zweimal mit je 25 ml Wasser gewaschen, anschließend mit Natriumsulfat getrocknet nach dem Abfiltrieren des Trokkenmittels eingedampft, zuletzt bei 80°C und vollem Wasserstrahlvakuum. Der Rückstand (15,4 g) wird durch Säulenchromatographie an 2 kg Kieselgel (MN K 60, 230-400 mesh ASTM, von Macherey und Nagel) unter Verwendung von Dichlormethan-Methanol-konz. Ammoniak 95:5:0,1 als Elutionsmittel gereinigt. Die Fraktionen mit der reinen Substanz ergeben nach Eindampfen einen Rückstand (6,8 g), der aus der Base der Titelverbindung besteht. Der Rückstand wird mit 14 ml absolutem Ethanol gelöst und die Lösung mit 10 ml 2,5 N ethanolischer Salzsäure angesäuert. Nach Versetzen mit Diethylether bis eben zur bleibenden Trübung kristallisiert die Titelverbindung. Nach dem Stehenlassen über einen Zeitraum von ca. 12. Stunden im Kühlschrank wird abgesaugt und zuerst mit einer Methanol-Ether-Mischung 1:2, dann mit Ether gewaschen. Nach dem Trocknen, zuletzt bei 80°C, erhält man 6,4 g der Titelverbindung (31,4 % der theoretisch maximal erhältlichen Menge) mit einem Schmelzpunkt von 236° (Zers.) und einem spezifischen Drehwert von $[\alpha]_D^{25}$ = +88,0° (c = 1, $CH_3OH$).

4.1.3 Beispiel 3
(+)-(1S,5R,2"S)-2'-Hydroxy-2-(2-methoxypropyl)-5,9,9-trimethyl-6,7-benzomorphan Hydrochlorid

Ausgehend von 9,25 g (0,040 Mol) (±)-2'-Hydroxy-5,9,9-trimethyl-6,7-benzomorphan und 6,37 g (0,052 Mol) (S)-2-Methoxypropionsäurechlorid erhält man analog Beispiel 1 die Titelverbindung in einer Ausbeute von 5,1 g (75,0 % d. Th.) mit einem $R_f$-Wert von 0,42, einem Schmelzpunkt von 270°C (Zers.) und einem spezifischen Drehwert von $[\alpha]_D^{25}$ = +117° (c = 1, $CH_3OH$).

4.1.4 Beispiel 4
(-)-(1R,5S,2"S)-2'-Hydroxy-2-(2-methoxypropyl)-5,9,9-trimethyl-6,7-benzomorphan Hydrochlorid

Ausgehend von der in Beispiel 3 anfallenden Mutterlauge erhält man analog Beispiel 2 die Titelverbindung in einer Ausbeute von 3,2 g (47,1 % d. Th.) mit einem Schmelzpunkt von 235°C (Zers.) und einem spezifischen Drehwert von $[\alpha]_D^{25}$ = -89,9° (c = 1, $CH_3OH$).

4.1.5 Beispiel 5
(-)-(1R,5S,2"R)-2'-Hydroxy-2-(2-methoxypropyl)-5,9,9-trimethyl-6,7-benzomorphan Hydrochlorid

Setzt man analog Beispiel 1 statt der racemischen Ausgangsverbindung 2,31 g (0,010 Mol) des enantiomerenreinen (-)-2'-Hydroxy-5,9,9-trimethyl-6,7-benzomorphans mit 1,60 g (0,013 Mol) (R)-2-Methoxypropionsäurechlorid um, so erhält man statt eines Gemisches der diastereomeren Basen zunächst die sterisch reine Base der Titelverbindung und aus dieser das Hydrochlorid in einer Ausbeute von 3,0 g (88,4 % d. Th.) mit einem Schmelzpunkt von 264°C (Zers.) und einem spezifischen Drehwert von $[\alpha]_D^{25}$ = -117,5° (c = 1, $CH_3OH$).

4.1.6 Beispiel 6
(-)-(1R,5S,2"S)-2'-Hydroxy-2-(2-methoxypropyl)-5,9,9-trimethyl-6,7-benzomorphan Hydrochlorid

Führt man die in Beispiel 5 beschriebene Synthese mit (S)-2-Methoxypropionsäure durch, so erhält man die Titelverbindung in einer Ausbeute von 2,9 g (85,3 % d. Th.) mit einem Schmelzpunkt von 235°C (Zers.) und einem spezifischen Drehwert von $[\alpha]_D^{25}$ = -89,7° (c = 1, $CH_3OH$).

4.1.7 Beispiel 7
(-)-(1R,5S,2"R)-2'-Hydroxy-2-(2-methoxypropyl)-5,9,9-trimethyl-6,7-benzomorphan Hydrochlorid

Ausgehend von 4,62 g (0,020 Mol) (-)-2'-Hydroxy-5,9,9-trimethyl-6,7-benzomorphan und 3,20 g (0,026 Mol) (R/S)-2-Methoxypropionsäurechlorid erhält man in Analogie zu Beispiel 1 ein Gemisch der zu erwartenden diastereomeren Basen, die analog Beispiel 2 mittels Säulenchromatographie getrennt werden. Die langsamer laufende Substanz ($R_f$ = 0,42) wird wie dort beschrieben als Hydrochlorid kristallisiert. Man erhält die Titelverbindung in einer Ausbeute von 2,1 g (61,8 % d. Th.) mit einem Schmelzpunkt von 264°C (Zers.) und einem spezifischen Drehwert von $[\alpha]_D^{25}$ = -117,9° (c = 1, $CH_3OH$).

4.1.8 Beispiel 8
(-)-(1R,2S,2"S)-2'-Hydroxy-2-(2-methoxypropyl)-5,9,9-trimethyl-6,7-benzomorphan Hydrochlorid

Ausgehend von der in Beispiel 7 abgetrennten, schneller laufenden Substanz ($R_f$ = 0,51) erhält man nach Überführung in das Hydrochlorid analog Beispiel 2 die Titelverbindung in einer Ausbeute von 2,2 g (64,7 % d. Th.) mit einem Schmelzpunkt von 235°C (Zers.) und einem spezifischen Drehwert von $[\alpha]_D^{25}$ = -88,6° (c = 1, $CH_3OH$).

4.1.9 Beispiel 9
(+)-(1S,5R,2"S)-2'-Hydroxy-2-(2-methoxypropyl)-5,9,9-trimethyl-6,7-benzomorphan Hydrochlorid

2,60 g (0,025 Mol) (S)-2-Methoxypropionsäurechlorid werden in 100 ml absolutem Tetrahydrofuran mit 4,04 g (0,025 Mol) 1,1'-Carbonyldiimidazol zwei Stunden bei Raumtemperatur gerührt. Nach Zugabe von 4,62 g (0,020 Mol) (+)-2'-Hydroxy-5,9,9-trimethyl-6,7-benzomorphan wird zwei Tage bei Raumtemperatur weitergerührt. Dann wird eingedampft, der Rückstand anschließend mit 75 ml Dichlormethan aufgenommen und nacheinander mit 2 N HCl und zweimal mit Wasser gewaschen. Die organische Phase wird nach dem Abtrennen mit Natriumsulfat getrocknet und nach dem Abfiltrieren des Trockenmittels i. Vak., zuletzt bei 80°C und vollem Wasserstrahlvakuum, eingedampft. Der Eindampfungsrückstand wird - wie in Beispiel 1 beschrieben - mit Lithiumaluminiumhydrid (Lithiumtetrahydridoalanat) reduziert und das Reduktionsprodukt analog Beispiel 2 als Hydrochlorid kristallisiert. Man erhält die Titelverbindung in einer Ausbeute von 6,9 g (81,3 % d. Th.) mit einem Schmelzpunkt von 264°C (Zers.) und einem spezifischen Drehwert von $[\alpha]_D^{25}$ = +117,4° (c = 1, $CH_3OH$).

4.1.10 Beispiel 10
(-)-(1R,5S,2"R)-9,9-Dimethyl-5-ethyl-2'-hydroxy-2-(2-methoxypropyl)-6,7-benzomorphan Hydrochlorid

Ausgehend von 1,69 g (0,006 Mol) (±)-9,9-Dimethyl-5-ethyl-2'-hydroxy-6,7-benzomorphan Hydrochlorid [DOS 20 27 077; CA 74, 125482x (1971)], 1,82 g (0,018 Mol) Triethylamin und 0,96 g (0,0078 Mol) (R)-2-Methoxypropionsäurechlorid erhält man analog Beispiel 1 ein Gemisch der zu erwartenden diastereomeren Basen (1,7 g), die in Analogie zu Beispiel 2 mittels Säulenchromatographie getrennt werden. Die langsamer laufende Substanz (0,6 g, $R_f$ = 0,40) wird analog Beispiel 2 als Hydrochlorid auskristallisiert. Man erhält dabei die Titelverbindung in einer Ausbeute von 0,6 g (56,6 % d. Th.) mit einem Schmelzpunkt von 275°C (Zers.) und einem spezifischen Drehwert von $[\alpha]_D^{25}$ = -100,5° (c = 1, $CH_3OH$).

4.1.11 Beispiel 11
(+)-(1S,5R,2"R)-9,9-Dimethyl-5-ethyl-2'-hydroxy-2-(2-methoxypropyl)-6,7-benzomorphan Hydrochlorid

Das in Beispiel 10 säulenchromatographisch abgetrennte, schneller laufende Diastereomere (0,7 g, $R_f$ = 0,45) wird analog Beispiel 2 als Hydrochlorid auskristallisiert. Man erhält die Titelverbindung in einer Ausbeute von 0,23 g (21,7 % d. Th.) mit einem Schmelzpunkt von 216°C (Zers.) und einem spezifischen Drehwert von $[\alpha]_D^{25}$ = +62,4° (c = 1, $CH_3OH$).

4.1.12 Beispiel 12
(+)-(1S,5R,2"S)-9,9-Dimethyl-5-ethyl-2'-hydroxy-2-(2-methoxypropyl)-6,7-benzomorphan Hydrochlorid

Ausgehend von 1,69 g (0,006 Mol) (±)-9,9-Dimethyl-5-ethyl-2'-hydroxy-6,7-benzomorphan Hydrochlorid, 1,82 g (0,018 Mol) Triethylamin und 0,96 g (0,0078 Mol) (S)-2-Methoxypropionsäurechlorid erhält man analog Beispiel 1 ein Gemisch der zu erwartenden diastereomeren Basen (1,7 g ), die - analog Beispiel 2 - mittels Säulenchromatographie getrennt werden. Die langsamer laufende Substanz (0,6 g, $R_f$ = 0,40) wird analog Beispiel 2 als Hydrochlorid auskristallisiert. Man erhält die Titelverbindung in einer Ausbeute von 0,6 g (56,6 % d. Th.) mit einem Schmelzpunkt von 275°C (Zers.) und einem spezifischen Drehwert von $[\alpha]_D^{25}$ = +102,5° (c = 1, $CH_3OH$).

4.1.13 Beispiel 13
(-)-(1R,5S,2"S)-9,9-Dimethyl-5-ethyl-2'-hydroxy-2-(2-methoxypropyl)-6,7-benzomorphan Hydrochlorid

Das nach Beispiel 12 säulenchromatographisch abgetrennte, schneller laufende Diastereomere (0,6 g, $R_f$ = 0,45) wird analog Beispiel 2 als Hydrochlorid auskristallisiert. Man erhält die Titelverbindung in einer Ausbeute von 0,65 g (61,2 % d. Th.) mit einem Schmelzpunkt von 219°C (Zers.) und einem spezifischen Drehwert von $[\alpha]_D^{25}$ = -67,6° (c = 1, $CH_3OH$).

4.1.14 Beispiel 14
(-)-9,9-Dimethyl-2'-hydroxy-2-((R)-2-methoxypropyl)-5-phenyl-6,7-benzomorphan

Ausgehend von 1,76 g (6 mMol) (±)-9,9-Dimethyl-2'-hydroxy-5-phenyl-6,7-benzomorphan [EP-B-4960; CA 93, 4941f (1980)] und 0,96 g (7,8 mMol) (R)-2-Methoxypropionsäurechlorid erhält man analog Beispiel 1 ein Gemisch der zu erwartenden diastereomeren Basen (2,1 g) mit $R_f$-Werten von 0,50 und 0,55 (vergl. Beispiel 2). Das Gemisch wird analog Beispiel 2 in Form der Hydrochloride auskristallisiert (2,4 g) und wieder in die entsprechenden freien Basen überführt. (Eindampfungsrückstand 1,8 g). Dieser Basenrückstand wird aus 6 ml Essigsäureethylester und 18 ml Petrolether (80°C) umkristallisiert. Danach erhält man die Titelverbindung in einer Ausbeute von 0,72 g (65,5 % d. Th.) mit einem Schmelzpunkt von 202°C, einem $R_f$-Wert von 0,50 und einem spezifischen Drehwert von $[\alpha]_D^{25}$ = -24,4° (c = 1, $CH_3OH$).

4.1.15 Beispiel 15
(+)-9,9-Dimethyl-2'-hydroxy-2-((R)-2-methoxypropyl)-5-phenyl-6,7-benzomorphan Hydrochlorid

Die nach Beispiel 13 bei der Kristallisation erhaltene Mutterlauge (Essigester/Petrolether) wird eingedampft. Der Rückstand (1,2 g) wird in 5 ml Ethanol gelöst und die Lösung mit 2,5 N ethanolischer Salzsäure angesäuert. Nach Zugabe von Diethylether bis eben zur Trübung kristallisiert Hydrochlorid (0,4 g) als verunreinigtes Rohprodukt. Die Mutterlauge enthält die reine Titelverbindung, die nach deren Eindampfen als Rückstand isoliert wird: Ausbeute 0,75 g (62,0 % d. Th.), Schmelzpunkt 168°C (Zers.), $R_f$ = 0,55 spezifischer Drehwert $[\alpha]_D^{25}$ = +11,6° (c = 1, $CH_3OH$).

4.1.16 Beispiel 16
(-)-(1R,5S,2"R)-3'-Hydroxy-2-(2-methoxypropyl)-5,9,9-trimethyl-6,7-benzomorphan Hydrochlorid

a) Ausgangsverbindung

Das hier und in weiteren Beispielen benötigte (±)-3'-Hydroxy-5,9,9-trimethyl-6,7-benzomorphan ist aus dem Stand der Technik noch nicht bekannt. Es kann beispielsweise auf dem für das bekannte 2'-Hydroxy-Isomere [DOS 20 27 077,; CA 74 (1971), 125482x] angegebenen Syntheseweg hergestellt werden, indem man anstelle des p-Methoxybenzyllithiums das m-Methoxybenzyllithium einsetzt. Beim Ringschluß zum Benzomorphan-System entstehen in letzterem Fall zwei isomere Verbindungen, die als Gemisch weiterumgesetzt werden. Das zuletzt erhaltene Gemisch aus 1'-Hydroxy- und 3'-Hydroxy-5,9,9-trimethyl-6,7-benzomorphan wird durch Flash-Säulenchromatographie an der 100-fachen Menge Kieselgel (MN K 60, 230-400 mesh ASTM, von Macherey und Nagel) unter Verwendung einer Mischung Essigsäureethylester-Methanol-konz. Ammoniak 80:20:5 als Elutionsmittel getrennt. Man erhält danach das (±)-1'-Hydroxy-5,9,9-trimethyl-6,7-benzomorphan ($R_f$ = 0,20) und das (±)-3'-Hydroxy-5,9,9-trimethyl-6,7-benzomorphan ($R_f$ = 0,14) in einem Verhältnis von 1:2 in Form von dickflüssigen Ölen ($R_f$-Werte: DC, Kieselgel 60, unter Verwendung des oben genannten Fließmittels).

b) Umsetzung zur Titelverbindung

Ausgehend von 2,31 g (0,010 Mol) (±)-3'-Hydroxy-5,9,9-trimethyl-6,7-benzomorphan und 1,6 g (0,013 Mol) (R)-2-Methoxypropionsäurechlorid erhält man analog Beispiel 1 ein Gemisch der zu erwartenden diasteromeren Basen, die durch Säulenchromatographie an der 100-fachen Menge Kieselgel analog Beispiel 2 getrennt werden. Die langsamer laufende Substanz ($R_f$ = 0,65) wird - wie dort beschrieben - als Hydrochlorid auskristallisiert. Man erhält die Titelverbindung in einer Ausbeute von 0,86 g (50,6 %) mit einem Schmelzpunkt von 240-242°C (Zers.) und einem spezifischen Drehwert von $[\alpha]_D^{25}$ = -90,9° (c = 1, $CH_3OH$).

4.1.17 Beispiel 17
(+)-(1S,5R,2"R)-3'-Hydroxy-2-(2-methoxypropyl)-5,9,9-trimethyl-6,7-benzomorphan Hydrochlorid

Die in Beispiel 16 abgetrennte, schneller laufende Substanz ($R_f$ = 0,74) wird analog Beispiel 2 als Hydrochlorid auskristallisiert. Man erhält die Titelverbindung in einer Ausbeute von 1,28 g (75,3 % d. Th.) mit einem Schmelzpunkt von 250-251°C und einem spezifischen Drehwert von $[\alpha]_D^{25}$ = +57,1° (c = 1, $CH_3OH$).

4.1.18 Beispiel 18
(+)-(1S,5R,2"S)-3'-Hydroxy-2-(2-methoxypropyl)-5,9,9-trimethyl-6,7-benzomorphan Hydrochlorid

Ausgehend von 2,31 g (0,010 Mol) (±)-3'-Hydroxy-5,9,9-trimethyl-6,7-benzomorphan (siehe Beispiel 16) und 1,60 g (0,013 Mol) (S)-2-Methoxypropionsäurechlorid erhält man analog Beispiel 1 ein Gemisch der zu erwartenden diastereomeren Basen, die durch Chromatographie - analog Beispiel 2 - an der 100-fachen Menge Kieselgel getrennt werden. Die langsamer laufende Substanz ($R_f$ = 0,65) wird - wie dort beschrieben - als Hydrochlorid kristallisiert. Man erhält die Titelverbindung in einer Ausbeute von 0,57 g (33,4 % d. Th.) mit einem Schmelzpunkt von 239-240°C (Zers.) und einem spezifischen Drehwert von $[\alpha]_D^{25}$ = +90,7° (c = 1, $CH_3OH$).

4.1.19 Beispiel 19
(-)-(1R,5S,2"S)-3'-Hydroxy-2-(2-methoxypropyl)-5,9,9-trimethyl-6,7-benzomorphan Hydrochlorid

Die in Beispiel 18 abgetrennte, schneller laufende Substanz ($R_f$ = 0,74) wird analog Beispiel 2 als Hydrochlorid auskristallisiert. Man erhält die Titelverbindung in einer Ausbeute von 0,67 g (39,2 % d. Th.) mit einem Schmelzpunkt von 250-251°C (Zers.) und einem spezifischen Drehwert von $[\alpha]_D^{25}$ = -56,5° (c = 1, $CH_3OH$).

4.1.20 Beispiel 20
(-)-(1R,5R,2"R)-1'-Hydroxy-2-(2-methoxypropyl)-5,9,9-trimethyl-6,7-benzomorphan Hydrochlorid

Ausgehend von 2,31 g (0,010 Mol) (±)-1'-Hydroxy-5,9,9-trimethyl-6,7-benzomorphan (siehe Beispiel 16) und 1,60 g (R)-2-Methoxypropionsäurechlorid erhält man analog Beispiel 1 ein Gemisch der zu erwartenden diastereomeren Basen, die analog Beispiel 2 an der 100-fachen Menge Kieselgel getrennt werden. Die langsamer laufende Substanz ($R_f$ = 0,52) wird analog Beispiel 2 als Hydrochlorid kristallisiert. Man erhält die Titelverbindung in einer Ausbeute von 0,70 g (41,2 % d. Th.) mit einem Schmelzpunkt von 128-135°C (Zers.) und einem spezifischen Drehwert von $[\alpha]_D^{25}$ = -100,5° (c = 1, $CH_3OH$).

4.1.21 Beispiel 21
(+)-(1S,5S,2"R)-1'-Hydroxy-2-(2-methoxypropyl)-5,9,9-trimethyl-6,7-benzomorphan Hydrochlorid

Die in Beispiel 20 abgetrennte, schneller laufende Substanz ($R_f$ = 0,56) wird analog Beispiel 2 als Hydrochlorid kristallisiert. Man erhält die Titelverbindung in einer Ausbeute von 0,40 g (23,6 % d. Th.) mit einem Schmelzpunkt von 88-89°C (Zers.) und einem spezifischen Drehwert von $[\alpha]_D^{25}$ = 50,4° (c = 1, $CH_3OH$).

4.1.22 Beispiel 22
(+)-(1S,5S,2"S)-1'-Hydroxy-2-(2-methoxypropyl)-5,9,9-trimethyl-6,7-benzomorphan Hydrochlorid

Ausgehend von 2,31 g (0,010 Mol) (±)-1'-Hydroxy-5,9,9-trimethyl-6,7-benzomorphan (siehe Beispiel 16) und 1,60 g (0,013 Mol) (S)-2-Methoxy-propionsäurechlorid erhält man analog Beispiel 1 ein Gemisch der zu erwartenden diastereomeren Basen, die analog Beispiel 2 an der 100-fachen Menge Kieselgel getrennt werden. Die langsamer laufende Substanz ($R_f$ = 0,52) wird analog Beispiel 2 als Hydrochlorid auskristallisiert. Man erhält die Titelverbindung in einer Ausbeute von 0,63 g (36,8 % d. Th.) mit einem Schmelzpunkt von 85-90°C und einem spezifischen Drehwert von $[\alpha]_D^{25}$ = +96,2° (c = 1, $CH_3OH$).

4.1.23 Beispiel 23
(-)-(1R,5R,2"S)-1'-Hydroxy-2-(2-methoxypropyl)-5,9,9-trimethyl-6,7-benzomorphan Hydrochlorid

Die in Beispiel 22 abgetrennte, schneller laufende Substanz ($R_f$ = 0,56) wird analog Beispiel 2 als Hydrochlorid kristallisiert. Man erhält die Titelverbindung in einer Ausbeute von 0,50 g (29,4 % d. Th.) mit einem Schmelzpunkt von 90-91°C (Zers.) und einem spezifischen Drehwert von $[\alpha]_D^{25}$ = -64,0° (c = 1, $CH_3OH$).

4.1.24 Beispiel 24
(-)-(1R,5S,2"R)-4'-Hydroxy-2-(2-methoxypropyl)-5,9,9-trimethyl-6,7-benzomorphan Hydrochlorid

a) Herstellung der Ausgangsverbindung

Die Herstellung des hier und in weiteren Beispielen benötigten (±)-4'-Hydroxy-5,9,9-trimethyl-6,7-benzomorphans ist aus dem Stand der Technik noch nicht bekannt. Man kann es beispielsweise auf dem aus dem Stand der Technik bekannten Syntheseweg für das bekannte 2'-Hydroxy-Isomere [DOS 20 27 077, CA 74, 125482x (1971)] herstellen, indem man anstelle des p-Methoxybenzyllithiums o-Methoxybenzyllithium einsetzt. Die gesuchte Verbindung kristallisiert als Base aus Isopropanol-Petrolether mit einem Schmelzpunkt von 227°C. Im Dünnschichtchromatogramm (Kieselgel 60, Chloroform-Methanol-konz. Ammoniak 65:35:3) kann dieser ein $R_f$-Wert von 0,20 zugeordnet werden.

b) Umsetzung zur Titelverbindung

Ausgehend von 2,31 g (0,010 Mol) (±)-4'-Hydroxy-5,9,9-trimethyl-6,7-benzomorphan und 1,60 g (0,01 Mol) (R)-2-Methoxypropionsäurechlorid erhält man analog Beispiel 1 ein Gemisch der zu erwartenden Basen (3,0 g), die analog Beispiel 2 mittels Säulenchromatographie an 450 g Kieselgel getrennt werden. Man erhält dabei 0,9 g der langsamer laufenden Verbindung ($R_f$ = 0,34) und 1,3 g der schneller laufenden Verbindung ($R_f$ = 0,43). Erstere wird analog Beispiel 2 als Hydrochlorid kristallisiert. Dabei erhält man die Titelverbindung in einer Ausbeute von 0,95 g (55,9 % d. Th.) mit einem Schmelzpunkt von 263°C (Zers.) und einem spezifischen Drehwert von $[\alpha]_D^{25}$ = -86,9° (c = $CH_3OH$).

4.1.25 Beispiel 25
(+)-(1S,5R,2"R)-4'-Hydroxy-2-(2-methoxypropyl)-5,9,9-trimethyl-6,7-benzomorphan Hydrochlorid

Die in Beispiel 24 abgetrennte, schneller laufende Verbindung ($R_f$ = 0,43) wird analog Beispiel 2 als Hydrochlorid kristallisiert. Man erhält die Titelverbindung in einer Ausbeute von 1,23 g (72,4 % d. Th.) mit einem Schmelzpunkt von 258°C (Zers.) und einem spezifischen Drehwert von $[\alpha]_D^{25}$ = +51,7°, (c = 1, $CH_3OH$).

4.1.26 Beispiel 26
(+)-(1S,5R,2"S)-4'-Hydroxy-2-(2-methoxypropyl)-5,9,9-trimethyl-6,7-benzomorphan Hydrochlorid

Ausgehend von 2,31 g (0,010 Mol) (±)-4'-Hydroxy-5,9,9-trimethyl-6,7-benzomorphan (siehe Beispiel 24) und 1,60 g (0,013 Mol) (S)-2-Methoxypropionsäurechlorid erhält man analog Beispiel 1 ein Gemisch der zu erwartenden diastereomeren Basen (3,1 g), die analog Beispiel 2 mittels Säulenchromatographie an 450 g Kieselgel getrennt werden. Man erhält dabei 1,0 g der langsamer laufenden Verbindung ($R_f$ = 0,34) und 1,2 g der schneller laufenden Verbindung. Erstere wird analog Beispiel 2 in Form des Hydrochlorids kristallisiert und ergibt die Titelverbindung in einer Ausbeute von 1,15 g (67,7 %) mit einem Schmelzpunkt von 260°C (Zers.) und einem spezifischen Drehwert von $[\alpha]_D^{25}$ = -51,2° (c = 1, $CH_3OH$).

4.1.27 Beispiel 27
(-)-(1R,5S,2"S)-4'-Hydroxy-2-(2-methoxypropyl)-5,9,9-trimethyl-6,7-benzomorphan Hydrochlorid

Die in Beispiel 26 abgetrennte, schneller laufende Substanz ($R_f$ = 0,43) wird analog Beispiel 2 als Hydrochlorid kristallisiert und ergibt die Titelverbindung in einer Ausbeute von 0,95 g (55,9 % d. Th.) mit einem Schmelzpunkt von 261°C (Zers.) und einem spezifischen Drehwert von $[\alpha]_D^{25}$ = +86,1°, (c = 1, $CH_3OH$).

4.1.28 Beispiel 28
(-)-(1R,5S,2"R)-2'-Methoxy-2-(2-methoxypropyl)-5,9,9-trimethyl-6,7-benzomorphan Methansulfonat

Ausgehend von 2,45 g (0,010 Mol) (-)-2'-Methoxy-5,9,9-trimethyl-6,7-benzomorphan [DOS 20 27 077; CA 125482x (1971)] und 1,35 g (0,011 Mol) (R)-2-Methoxypropionsäurechlorid erhält man analog Beispiel 1 die sterisch einheitliche Titelverbindung in Form der freien Base (2,3 g). Diese wird in 5 ml absolutem Ethanol gelöst. Die erhaltene Lösung wird nach Ansäuern mit Methansulfonsäure mit Diethylether bis eben zur Trübung versetzt. Während das Methansulfonat kristallisiert, gibt man unter Rühren nach und nach noch einmal die gleiche Menge Ether zu. Dann wird die Reaktionsmischung 4 Stunden mit einem Eisbad gekühlt, anschließend abgesaugt, dann zunächst mit einer Ethanol-Ether-Mischung 1:2, danach nur mit Ether gewaschen. Nach dem Trocknen, zuletzt bei 80°C, erhält man die Titelverbindung in einer Ausbeute von 3,3 g (79,8 % d. Th.) mit einem Schmelzpunkt von 165-166°C (Zers.) und einem spezifischen Drehwert von $[\alpha]_D^{25}$ = -92,4° (c = 1, $CH_3OH$).

4.1.29 Beispiel 29
(+)-(1S,5R,2"S)-2'-Methoxy-2-(2-methoxypropyl)-5,9,9-trimethyl-6,7-benzomorphan Methansulfonat

Ausgehend von 2,45 g (0,010 Mol) (+)-2'-Methoxy-5,9,9-trimethyl-6,7-benzomorphan [DOS 20 27 077; CA 74, 125482x (1971)] und 1,35 g (0,011 Mol) (S)-2-Methoxypropionsäurechlorid erhält man analog Beispiel 28 die Titelverbindung in einer Ausbeute von 3,1 g (75,0 % d. Th.) mit einem Schmelzpunkt von 165-167°C (Zers.) und einem spezifischen Drehwert von $[\alpha]_D^{25}$ = +92,3° (c = 1, $CH_3OH$).

4.1.30 Beispiel 30
(-)-(1R,5S,2"R)-2-(2-Methoxypropyl)-5,9,9-trimethyl-6,7-benzomorphan Hydrochlorid

a) Herstellung der Ausgangsverbindung

Die Herstellung des hier und in weiteren Beispielen benötigten (±)-5,9,9-Trimethyl-6,7-benzomorphans ist aus dem Stand der Technik noch nicht bekannt. Es kann beispielsweise auf dem für das bekannte 2'-Hydroxy-Derivat angegebenen Syntheseweg [DOS 20 27 077; CA 74 (1971) 125482x] hergestellt werden, wobei man anstelle des p-Methoxybenzyllithiums Benzyllithium einsetzt. Die gesuchte Verbindung kristallisiert als Hydrobromid aus einer Isopropanol-Ether-Mischung mit einem Schmelzpunkt von 259°C (Zers.).

b) Umsetzung zur Titelverbindung

Ausgehend von 2,96 g (0,010 Mol) (±)-5,9,9-Trimethyl-6,7-benzomorphan und 1,35 g (0,011 Mol) (R)-2-Methoxypropionsäurechlorid erhält man analog Beispiel 1 ein Gemisch der zu erwartenden diastereomeren Basen. Diese werden analog Beispiel 2 durch Säulenchromatographie an der 150-fachen Menge Kieselgel getrennt. Die langsamer laufende Substanz ($R_f$ = 0,63) wird - wie dort beschrieben - als Hydrochlorid kristallisiert. Man erhält die Titelverbindung in einer Ausbeute von 1,3 g (76,5 % d. Th.) mit einem Schmelzpunkt von 225°C (Zers.) und einem spezifischen Drehwert von $[\alpha]_D^{25}$ = -101,7° (c = 1, $CH_3OH$).

4.1.31 Beispiel 31
(+)-(1S,5R,2"R)-2-(2-Methoxypropyl)-5,9,9-trimethyl-6,7-benzomorphan Oxalat

Die in Beispiel 30 abgetrennte, schneller laufende Substanz ($R_f$ = 0,67) wird mit 5 ml Ethanol gelöst. Die Lösung wird nach dem Ansäuern mit Oxalsäure mit Diethylether bis eben zur Trübung versetzt. Während der danach einsetzenden Kristallisation gibt man nach und nach noch einmal die Hälfte der ursprünglichen Ethermenge zu und läßt sie ca. 12 Stunden im Kühlschrank. Nach Absaugen, Waschen mit einer Ethanol-Ether-Mischung und Trocknen bei 80°C erhält man die Titelverbindung in einer Ausbeute von 1,1 g (60,4 % d. Th.) mit einem Schmelzpunkt von 135°C (Zers.) und einem spezifischen Drehwert von $[\alpha]_D^{25}$ = +60,2° (c = 1, $CH_3OH$).

4.1.32 Beispiel 32
(-)-(1R,5S,2"R)-2-(2-Methoxypropyl)-2',5,9,9-tetramethyl-6,7-benzomorphan Hydrochlorid

a) Herstellung der Ausgangsverbindung

Das hier und in weiteren Beispielen benötigte (±)-2',5,9,9-Tetramethyl-6,7-benzomorphan ist aus dem Stand der Technik noch nicht bekannt. Es kann beispielsweise auf dem für das bekannte 2'-Hydroxy-Analogon angegebenen Syntheseweg hergestellt werden, indem man anstelle des p-Methoxybenzyllithiums p-Methylbenzyllithium einsetzt. Das so erhaltene Zwischenprodukt wird aus einer Isopropanol-Diethylether-Mischung als Hydrobromid kristallisiert, das bei 227°C unter Zersetzung schmilzt.

b) Umsetzung zur Titelverbindung

Ausgehend von 1,51 g (5 mMol) (±)-2',5,9,9-Tetramethyl-6,7-benzomorphan und 0,68 g (5,5 mMol) (R)-2-Methoxypropionsäurechlorid erhält man analog Beispiel 1 ein Gemisch der zu erwartenden diastereomeren Basen, die - wie dort beschrieben - als Hydrochloride kristallisiert und getrennt werden. Man erhält dabei die Titelverbindung in einer Ausbeute von 0,7 g (nach Umkristallisieren 0,4 g = 47,3 % d. Th.) mit einem Schmelzpunkt von 212°C (Zers.) und einem spezifischen Drehwert von $[\alpha]_D^{25}$ = -110,0° (c = 1, $CH_3OH$).

4.1.33 Beispiel 33
(+)-1S,5R,2"S)-2-(2-Methoxypropyl)-2',5,9,9-Tetramethyl-6,7-benzomorphan Hydrochlorid

Ausgehend von 1,51 g (5 mMol) (±)-2',5,9,9-Tetramethyl-6,7-benzomorphan und 0,68 g (5,5 mMol) (S)-2-Methoxypropionsäurechlorid erhält man analog Beispiel 32 die Titelverbindung in einer Ausbeute von 0,4 g (47,3 % d. Th.) mit einem Schmelzpunkt von 212°C (Zers.) und einem spezifischen Drehwert von $[\alpha]_D^{25}$ = +111,8° (c = 1, $CH_3OH$).

4.1.34 Beispiel 34
(-)-(1R,5S,2"R)-2'-Amino-2-(2-methoxypropyl)-5,9,9-trimethyl-6,7-benzomorphan Dihydrochlorid

a) Herstellung der Ausgangsverbindung

Das hier und in weiteren Beispielen benötigte (±)-2'-Nitro-5,9,9-trimethyl-6,7-benzomorphan ist aus dem Stand der Technik noch nicht bekannt. Man kann es z.B. durch Nitrierung des (±)-5,9,9-Trimethyl-6,7-benzomorphans (Beispiel 30) in Analogie zu einer Vorschrift von E.L. May und E.M. Fry [J. Org. Chem. 22 (1957) 1366] herstellen. Die resultierende Zwischenverbindung wird aus einer Ethanol-Ether-Mischung als Hydrochlorid kristallisiert, das bei 289°C (Zers.) schmilzt.

b) Acylierung

2,97 g (0,01 Mol) (±)-2'-Nitro-5,9,9-trimethyl-6,7-benzomorphan Hydrochlorid werden in Gegenwart von 3,04 g (0,03 Mol) Triethylamin analog Beispiel 1 mit 1,35 g (0,011 Mol) (R)-2-Methoxypropionsäurechlorid acyliert. Nach Aufarbeitung erhält man 3,5 g (rund 100 % d. Th.) des Zwischenproduktes vom Typ (3).

c) Katalytische Hydrierung der 2'-Nitro- zur 2'-Amino-Gruppe

3,5 g des aus Verfahrensschritt b) resultierenden Zwischenproduktes werden in 70 ml Methanol gelöst und in Gegenwart von 0,4 g Palladium auf Kohle (Pd-Gehalt: 5 %) bei 20°C unter einem Wasserstoffdruck von 5 bar hydriert. Die Wasserstoffaufnahme kommt nach 4 Stunden - nach dem Verbrauch der berechneten Menge - zum Stillstand. Nach dem Abfiltrieren des Katalysators wird die Reaktionsmischung eingedampft, zuletzt bei 80°C und unter vollem Wasserstrahlvakuum. Rückstand: 3,1 g (rund 100 % d. Th.).

d) Reduktion mit Lithiumaluminiumhydrid

Der oben erhaltene Eindampfungsrückstand (3,1 g) wird analog Beispiel 1 mit 1,3 g Lithiumaliminiumumhydrid (Lithiumtetrahydridoalanat) reduziert. Das dabei erhaltene Gemisch der beiden zu erwartenden diastereomeren Basen (2,7 g) wird analog Beispiel 2 durch Säulenchromatographie an 500 g Kieselgel getrennt. Die langsamer laufende Substanz (0,7 g, $R_f$ = 0,65) wird analog Beispiel 2 (aber mit zwei Äquivalenten Salzsäure) als Dihydrochlorid kristallisiert. Man erhält die Titelverbindung in einer Ausbeute von 0,95 g (50,6 % d. Th.) und mit einem Schmelzpunkt von 260°C (Zers.) sowie einem spezifischen Drehwert von $[\alpha]_D^{25}$ = -93,4° (c = 1, $CH_3OH$).

4.1.35 Beispiel 35
(+)-(1S,5R,2"R)-2'-Amino-2-(2-methoxypropyl)-5,9,9-trimethyl-6,7-benzomorphan Dimethansulfonat

0.9 g der in Beispiel 34 abgetrennten, schneller laufenden Substanz ($R_f$ = 0,70) werden aus Methanol-Diethylether-Mischung analog Beispiel 28 (jedoch mit 2 Äquivalenten Methansulfonsäure) kristallisiert. Man erhält die Titelverbindung in einer Ausbeute von 0,48 g (19,4 % d. Th.) mit einem Schmelzpunkt von 189°C (Zers.) und einem spezifischen Drehwert von $[\alpha]_D^{25}$ = +55,6° (c = 1, $CH_3OH$).

4.1.36 Beispiel 36
(+)-(1S,5R,2"S)-2'-Amino-2-(2-methoxypropyl)-5,9,9-trimethyl-6,7-benzomorphan Hydrochlorid

Ausgehend von 2,97 g (0,01 Mol) (±)-2'-Nitro-5,9,9-trimethyl-6,7-benzomorphan Hydrochlorid, 3,04 g (0,03 Mol) Triethylamin und 1,35 g (0,011 Mol) (S)-2-Methoxypropionsäurechlorid erhält man 3,5 g (rund 100 % d. Th.) des betreffenden Acylierungsproduktes, das - wie dort beschrieben - katalytisch hydriert (woraus 3,2 g Hydrierungsprodukt resultieren) und anschließend mit Lithiumaluminiumhydrid (Lithiumtetrahydridoalanat) reduziert wird. Das Reduktionsprodukt (2,8 g), das aus einer Mischung der zu erwartenden diasteromeren Basen besteht, wird unter Erwärmen in 10 ml Isopropanol gelöst. Über einen Zeitraum von ca. 12. Stunden kristallisiert beim Stehen im Kühlschrank die chromatographisch langsamer laufende Substanz ($R_f$ = 0,65) als Base (0,75 g = 49,0 % d. Th.) mit einem Schmelzpunkt von 142°C. Davon wurden 0,3 g analog Beispiel 32 als Dihydrochlorid kristallisiert. Man erhält die Titelverbindung (0,37 g) mit einem Schmelzpunkt von 265°C (Zers.) und einen spezifischen Drehwert von $[\alpha]_D^{25}$ = +92,5° (c = 1, $CH_3OH$).

4.1.37 Beispiel 37
(-)-(1R,5S,2"S)-2'-Amino-2-(2-methoxypropyl)-5,9,9-trimethyl-6,7-benzomorphan Dihydrochlorid

Die Isopropanol-Mutterlauge von Beispiel 34 wird eingedampft und der Rückstand (2,0 g) an 300 g Kieselgel in Analogie zu Beispiel 2 chromatographiert. Das dabei neben weiteren 0,3 g der langsamer laufenden Substanz ($R_f$ = 0,65, Beispiel 34) schneller laufende Diastereomere (1,2 g, $R_f$ = 0,70) wird analog Beispiel 32 als Dihydrochlorid kristallisiert. Man erhält dabei die Titelverbindung in einer Ausbeute von 1,48 g (78,8 % d. Th.) mit einem Schmelzpunkt von 259°C (Zers.) und einem spezifischen Drehwert von $[\alpha]_D^{25}$ = -68,4° (c = 1, $CH_3OH$).

4.1.38 Beispiel 38
(±)-2'-Hydroxy-2-(2-methoxyisobutyl)-5,9,9-trimethyl-6,7-benzomorphan Hydrobromid

Ausgehend von 1,16 g (0,005 Mol) (±)-2'-Hydroxy-5,9,9-trimethyl-6,7-benzomorphan und 0,75 g (0,055 Mol) 2-Methoxyisobuttersäurechlorid erhält man analog Beispiel 1 zunächst die Basenform der Titelverbindung (1,1 g). Diese wird in 5 ml Methanol gelöst und die Lösung nach dem Ansäuern mit HBr (62 %ig) mit Diethylether bis eben zur Trübung versetzt. Beim Stehen über einen Zeitraum von ca. 12 h im Kühlschrank kristallisiert die Titelverbindung, die abgesaugt, mit einer Ethanol-Ether-Mischung gewaschen und bei 80°C getrocknet wird. Ausbeute 1,1 g (55,2 % d. Th.), Schmelzpunkt 232°C (Zers.). Eine aus Methanol-Ether-Mischung umkristallisierte Probe schmilzt bei 240°C (Zers.).

4.1.39 Beispiel 39
(-)-(1R,5S,2"R)-2'-Hydroxy-2-(2-methoxybutyl)-5,9,9-trimethyl-6,7-benzomorphan Hydrochlorid

a) Herstellung der Ausgangsverbindung

Das in diesem und in weiteren Beispielen benötigte (R)-2-Methoxy-buttersäurechlorid kann z.B. aus der bekannten (R)-2-Methoxybuttersäure [N.K. Kochetkov, A.M. Liknosherstov und V.N. Kulakov, Tetrahedron 25 (1969) 2313] und Thionylchlorid bei Raumtemperatur hergestellt werden. Das nach dem Entfernen des überschüssigen Thionylchlorids verbleibende Säurechlorid wird ohne weitere Reinigung in der nächsten Reaktionsstufe eingesetzt.

b) Umsetzung zur Titelverbindung

Ausgehend von 3,47 g (0,015 Mol) (±)-2'-Hydroxy-5,9,9-trimethyl-6,7-benzomorphan und 2,46 g (0,018 Mol) (R)-2-Methoxybuttersäurechlorid erhält man analog Beispiel 1 die Titelverbindung in einer Ausbeute von 1,8 g (67,8 % d. Th.) mit einem Schmelzpunkt von 246°C (Zers.) und einem spezifischen Drehwert von $[\alpha]_D^{25}$ = -118,4° (c = 1, $CH_3OH$).

4.1.40 Beispiel 40
(+)-(1S,5R,2"R)-2'-Hydroxy-2-(2-methoxybutyl)-5,9,9-trimethyl-6,7-benzomorphan Hydrochlorid

Die in Beispiel 39 anfallende Mutterlauge wird eingedampft. Aus dem Rückstand gewinnt man analog Beispiel 2 die Titelverbindung in einer Ausbeute von 1,3 g (48,9 % d. Th.) mit einem Schmelzpunkt von 228°C (Zers.) und einem spezifischen Drehwert von $[\alpha]_D^{25}$ = +79,9° (c = 1, $CH_3OH$).

4.1.41 Beispiel 41
(-)-(1R,5S,2"S)-2'-Hydroxy-2-(2-methoxybutyl)-5,9,9-trimethyl-6,7-benzomorphan Hydrochlorid

a) Herstellung der Ausgangsverbindung

Das in diesem und in weiteren Beispielen benötigte (S)-2-Methoxybuttersäurechlorid kann z.B. aus der bekannten (S)-2-Methoxy-buttersäure [Tetrahedron 25 (1969) 2322] analog Beispiel 39 hergestellt werden.

b) Umsetzung zur Titelverbindung

Ausgehend von 3,47 g (0,015 Mol) (±)-2'-Hydroxy-5,9,9-trimethyl-6,7-benzomorphan und 2,46 g (0,018 Mol) (S)-2-Methoxybuttersäurechlorid erhält man analog Beispiel 1 die Titelverbindung in einer Ausbeute von 1,6 g (60,3 % d. Th.) mit einem Schmelzpunkt von 246°C (Zers.) und einem spezifischen Drehwert von $[\alpha]_D^{25}$ = +119,2° (c = 1, $CH_3OH$).

4.1.42 Beispiel 42
(+)-(1S,5R,2"S)-2'-Hydroxy-2-(2-methoxybutyl)-5,9,9-trimethyl-6,7-benzomorphan Hydrochlorid

Die in Beispiel 41 anfallende Mutterlauge wird eingedampft. Aus dem Rückstand gewinnt man analog Beispiel 2 die Titelverbindung in einer Ausbeute von 0,6 g (22,6 % d. Th.) mit einem Schmelzpunkt von 228°C (Zers.) und einem spezifischen Drehwert von $[\alpha]_D^{25}$ = -71,7° (c = 1, $CH_3OH$).

4.1.43 Beispiel 43
(-)-(1R,5S,2"R)-2-(2-Benzyloxypropyl)-2'-hydroxy-5,9,9-trimethyl-6,7-benzomorphan Hydrochlorid

a) Herstellung der Ausgangsverbindung

Das hier und in weiteren Beispielen verwendete (R)-2-Benzyloxypropionsäurechlorid kann z.B. auf folgendem Weg hergestellt werden:
Käuflicher (R)-(+)-Milchsäure-isobutylester wird in Gegenwart von Silberoxid mit Benzylbromid O-benzyliert. Die anschließende Verseifung mit Natronlauge ergibt (R)-2-Benzyloxy-propionsäure mit einem spezifischen Drehwert von $[\alpha]_D^{25}$ = +79,5° (c = 1, $CH_3OH$).
Daraus erhält man das entsprechende Säurechlorid analog Beispiel 39.

b) Umsetzung zur Titelverbindung

Ausgehend von 3,47 g (0,015 Mol) (±)-2'-Hydroxy-5,9,9-trimethyl-6,7-benzomorphan und 3,3 g (0,0165 Mol) (R)-2-Benzyloxypropionsäurechlorid erhält man analog Beispiel 1 ein Gemisch der zu erwartenden Diastereomeren, die - wie dort beschrieben - durch Kristallisieren der entsprechenden Hydrochloride getrennt werden. Man gewinnt die Titelverbindung in einer Ausbeute von 2,39 g (76,6 % d. Th.) mit einem Schmelzpunkt von 250°C (Zers.) und einem spezifischen Drehwert von $[\alpha]_D^{25}$ = -119,3° (c = 1, $CH_3OH$).

4.1.44 Beispiel 44
(+)-(1S,5R,2"S)-2-(2-Benzyloxypropyl)-2'-hydroxy-5,9,9-trimethyl-6,7-benzomorphan Hydrochlorid

a) Herstellung der Ausgangsverbindung

Das hier und in weiteren Beispielen verwendete (S)-2-Benzyloxypropionsäurechlorid kann z.B. analog Beispiel 43 aus der (S)-2-Benzyloxypropionsäure (spezifischer Drehwert: $[\alpha]_D^{25}$ = -78,5° (c = 1, $CH_3OH$)) hergestellt werden.

b) Umsetzung zur Titelverbindung

Ausgehend von 3,47 g (0,015 Mol) (±)-2'-Hydroxy-5,9,9-trimethyl-6,7-benzomorphan und 3,3 g (0,0165 Mol) (S)-2-Benzyloxypropionsäurechlorid erhält man analog Beispiel 43 die Titelverbindung in einer Ausbeute von 2,56 g (82,1 % d. Th.) mit einem Schmelzpunkt von 250°C (Zers.) und einem spezifischen Drehwert von $[\alpha]_D^{25}$ = +119,5° (c = 1, $CH_3OH$).

4.1.45 Beispiel 45
(-)-(1R,5S,2"R)-2'-Hydroxy-2-(2-methylthiopropyl)-5,9,9-trimethyl-6,7-benzomorphan Hydrochlorid

a) Herstellung der Ausgangsverbindung

Das hier und in weiteren Beispielen benötigte (R)-2-Methylthiopropionsäurechlorid kann z.B. analog Beispiel 39 aus der bekannten (R)-2-Methylthio-propionsäure (L.N. Owen und M.B. Rahman J. Chem. Soc. [c] 1971, 2432] hergestellt werden.

b) Umsetzung zur Titelverbindung

1,62 g (0,007 Mol) (±)-2'-Hydroxy-5,9,9-trimethyl-6,7-benzomorphan werden analog Beispiel 1 mit 1,07 g (7,7 mMol) (R)-2-Methylthiopropionsäurechlorid umgesetzt. Nach Reduktion des dabei entstandenen Reaktionsproduktes mit Lithiumaluminiumhydrid ($LiAlH_4$) erhält man ein Gemisch der zu erwartenden Diastereomeren, die durch Kristallisation ihrer Nitrate getrennt werden. Die aus Ethanol nach Ansäuern mit 65 %-iger Salpetersäure und Versetzen mit Diethylether bis zur Trübung auskristallisierende Substanz wird nach Stehen im Kühlschrank über einen Zeitraum von ca. 12 h abgesaugt, mit einer Ethanol-Ether-Mischung dann mit Ether gewaschen und bei 80°C getrocknet. Das Nitrat (0,67 g) wird aus siedendem Ethanol umkristallisiert (0,52 g), anschließend in die freie Base überführt und schließlich analog Beispiel 2 als Hydrochlorid kristallisiert. Man erhält die Titelverbindung in einer Ausbeute von 0,43 g (34,4 % d. Th.) mit einem Schmelzpunkt von 265°C (Zers.) und einem spezifischen Drehwert von $[\alpha]_D^{25}$ = -135,1° (c = 0,5 $CH_3OH$).

4.1.46 Beispiel 46
(+)-(1S,5R,2"R)-2'-Hydroxy-2-(2-methylthiopropyl)-5,9,9-trimethyl-6,7-benzomorphan Hydrochlorid

Die nach Beispiel 45 erhaltene Mutterlauge der ersten Nitrat-Kristallisation wird eingedampft und der Nitrat-Rückstand in die freie Base überführt. Diese (1 g) wird analog Beispiel 55 über 20 g Aluminiumoxid filtriert und dann als Hydrochlorid kristallisiert (Ethanol-Ether). Dieses (0,29 g) wird aus einer Mischung aus 2 ml Methanol und Diethylether umkristallisiert. Man erhält die Titelverbindung in einer Ausbeute von 0,21 g (16,8 % d. Th.) mit einem Schmelzpunkt von 238°C (Zers.) und einem spezifischen Drehwert von $[\alpha]_D^{25}$ = +73,0° (c = 0,5, $CH_3OH$).

4.1.47 Beispiel 47
(-)-(1R,5S,2"S)-2'-Hydroxy-2-(2-methylthiopropyl-5,9,9-trimethyl-6,7-benzomorphan Hydrochlorid

a) Herstellung der Ausgangsverbindung

Das hier und in weiteren Beispielen benötigte (S)-2-Methylthiopropionsäurechlorid kann z.B. aus der bekannten (S)-2-Methylthio-propionsäure [J. Chem. Soc. 1971, 2437] analog Beispiel 39 hergestellt werden.

b) Umsetzung zur Titelverbindung

Ausgehend von 1,62 g (0,007 Mol) (±)-2'-Hydroxy-5,9,9-trimethyl-6,7-benzomorphan erhält man analog Beispiel 45 die Titelverbindung in einer Ausbeute von 0,42 g (33,6 % d. Th.) mit einem Schmelzpunkt von 265°C (Zers.) und einem spezifischen Drehwert von $[\alpha]_D^{25}$ = +135,1° (c = 0,5, $CH_3OH$).

4.1.48 Beispiel 48
(-)-(1R,5S,2"S)-2'-Hydroxy-2-(2-methylthiopropyl-5,9,9-trimethyl-6,7-benzomorphan Hydrochlorid

Ausgehend von der Nitrat-Mutterlauge aus Beispiel 47 erhält man analog Beispiel 46 die Titelverbindung in einer Ausbeute von 0,34 g (27,2 % d. Th.) mit einem Schmelzpunkt von 238°C (Zers.) und einem spezifischen Drehwert von $[\alpha]_D^{25}$ = -73,9° (c = 0,5, $CH_3OH$).

4.1.49 Beispiel 49
(+)-(1S,5R,2"S)-2-(2-Allyloxypropyl)-2'-hydroxy-5,9,9-trimethyl-6,7-benzomorphan Hydrochlorid

a) Herstellung der Ausgangsverbindung

Das hier verwendete (S)-(-)-2-Allyloxypropionsäurechlorid kann z.B. auf folgendem Wege hergestellt werden: Käuflicher (S)-(-)-Milchsäureethylester wird in Gegenwart von Silberoxid mit Allylbromid O-allyliert. Die anschließende Verseifung mit Natronlauge ergibt (S)-2-Allyloxy-propionsäure mit einem Siedepunkt von 110 - 112°C bei einem Druck von 15 mbar und einem spezifischen Drehwert von $[\alpha]_D^{25}$ = 78,4° (c = 1, $CH_3OH$). Daraus erhält man das entsprechende Säurechlorid analog Beispiel 39.

b) Umsetzung zur Titelverbindung

Ausgehend von 3,47 g (0,015 Mol) (±)-2'-Hydroxy-5,9,9-trimethyl-6,7-benzomorphan und 3,3 g (0,0165 Mol) (S)-2-Allyloxy-propionsäurechlorid erhält man analog Beispiel 1 ein Gemisch der beiden zu erwartenden Diastereomeren, die - wie dort beschrieben - durch Krisallisation der entsprechenden Hydrochloride getrennt werden. Man gewinnt die Titelverbindung in einer Ausbeute von 1,4 g (51,0 % d. Th.) mit einem Schmelzpunkt von 245°C (Zers.) und einem spezifischen Drehwert von $[\alpha]_D^{25}$ = +120° (c = 1, $CH_3OH$).

4.1.50 Beispiel 50
(-)-(1S,5R,2"S)-2-(2-Allyloxypropyl)-2'-hydroxy-5,9,9-trimethyl-6,7-benzomorphan Hydrochlorid

Die in Beispiel 49 angefallenen Mutterlaugen werden eingedampft. Aus dem Rückstand erhält man analog Beispiel 2 die Titelverbindung in einer Ausbeute von 0,62 g (22,6 % d. Th.) mit einem Schmelzpunkt von 213°C und einem spezifischen Drehwert von $[\alpha]_D^{25}$ = -38,6° (c = 1, $CH_3OH$).

4.1.51 Beispiel 51
(-)-(1R,5S,2"R)-2'-Hydroxy-2-(2-phenoxypropyl)-5,9,9-trimethyl-6,7-benzomorphan Hydrochlorid

2,31 g (0,010 Mol) (±)-2'-Hydroxy-5,9,9-trimethyl-6,7-benzomorphan und 2,03 g (0,011 Mol) (R)-2-Phenoxypropionsäurechlorid (hergestellt - analog Beispiel 39 - aus der bekannten Säure [CA 72, 12917d] werden analog Beispiel 1 umgesetzt. Man erhält ein Gemisch der beiden zu erwartenden Diastereomeren, die - wie dort beschrieben - durch Kristallisation der entsprechenden Hydrochloride getrennt werden. Man gewinnt auf diese Weise die Titelverbindung in einer Ausbeute von 1,6 g (79,6 % d. Th.) mit einem Schmelzpunkt von 279°C (Zers.) und einem spezifischen Drehwert von $[\alpha]_D^{25}$ = -142,9° (c = 1, $CH_3OH$).

4.1.52 Beispiel 52
(+)-(1S,5R,2"S)-2'-Hydroxy-2-(2-phenoxypropyl)-5,9,9-trimethyl-6,7-benzomorphan Hydrochlorid

Arbeitet man analog Beispiel 51 mit (S)-2-Phenoxy-propionsäurechlorid, so erhält man die Titelverbindung in einer Ausbeute von 1,6 g und einem spezifischen Drehwert von $[\alpha]_D^{25}$ = +143,3°C (c = $CH_3OH$).

4.1.53 Beispiel 53
(-)-(1R,5S,2"R)-3'-Hydroxy-2-(2-methoxypropyl)-5,9,9,2'-tetramethyl-6,7-benzomorphan Hydrochlorid

a) Ausgangsverbindung

Das hier und in weiteren Beispielen benötigte (+/-)-3'-Hydroxy-5,9,9,2'-tetramethyl-6,7-benzomorphan ist aus dem Stand der Technik noch nicht bekannt. Es kann beispielsweise auf dem für das bekannte 2'-Hydroxy-Derivat [DOS 20 27 077; CA 74 (1971), 125482x] angegebenen Syntheseweg hergestellt werden, indem man anstelle des p-Methoxybenzyllithiums das 3-Methoxy-4-methylbenzyllithium einsetzt. Das beim Ringschluß zum Benzomorphan-system überwiegend erhaltene 3'-Hydroxy-5,9,9,2'-tetramethyl-6,7-benzomorphan wird durch Flash-Säulenchromatographie (vergl. Bsp. 16) gereinigt.

b) Umsetzung zur Titelverbindung

Ausgehend von 2,44 g (0,010 Mol) (+/-)-3'-Hydroxy-5,9,9,2'-tetramethyl-6,7-benzomorphan und 1,6 g (0,013 Mol) (R)-2-Methoxypropionsäurechlorid erhält man analog Beispiel 1 ein Gemisch der zu erwartenden diasteriomeren Basen, die durch Säulenchromatographie an der 100-fachen Menge Kieselgel analog Beispiel 2 getrennt werden. Die langsamer

laufende Substanz ($R_f$ = 0,25) wird als Hydrochlorid auskristallisiert. Man erhält die Titelverbindung in einer Ausbeute von 1,06 g (59,9%) mit einem Schmelzpunkt von 181-182°C und einem spezifischen Drehwert von $[\alpha]_D^{25}$ = -89,5 (c = 1,$CH_3OH$).

4.1.54 Beispiel 54
(+)-(1S,5R,2"R)-3'-Hydroxy-2-(2-methoxypropyl)-5,9,9,2'-tetramethyl-6,7-benzomorphan Hydrochlorid

Die in Beispiel 53 abgetrennte, schneller laufende Substanz ($R_f$ = 0,34) wird analog Beispiel 2 als Hydrochlorid gefällt. Man erhält die Titelverbindung in einer Ausbeute von 1,24 g (70,1 %) als amorphes Pulver mit einem spezifischen Drehwert von $[\alpha]_D^{25}$ = +55,9° (c = 1, $CH_3OH$).

4.1.55 Beispiel 55
(+)-(1S,5R,2"S)-3'-Hydroxy-2-(2-methoxypropyl)-5,9,9,2'-tetramethyl-6,7-benzomorphan Hydrochlorid

Ausgehend von 2,44g (0,010 Mol) (+/-)-3'-Hydroxy-5,9,9,2'-tetramethyl-6,7-benzomorphan (siehe Beispiel 53) und 1,6 g (0,013 Mol) (S)-2-Methoxypropionsäurechlorid erhält man analog Beispiel 1 ein Gemisch der zu erwartenden diasteriomeren Basen, die durch Säulenchromatographie an der 100-fachen Menge Kieselgel analog Beispiel 2 getrennt werden. Die langsamer laufende Substanz ($R_f$ = 0,25) wird als Hydrochlorid auskristallisiert. Man erhält die Titelverbindung in einer Ausbeute von 1,48 g (83,6 %) mit einem Schmelzpunkt von 186-188°C und einem spezifischen Drehwert von $[\alpha]_D^{25}$ = +88,2° (c = 1, $CH_3OH$).

4.1.56 Beispiel 56
(-)-(1R,5S,2"S)-3'-Hydroxy-2-(2-methoxypropyl)-5,9,9,2'-tetramethyl-6,7-benzomorphan Hydrochlorid

Die in Beispiel 55 abgetrennte, schneller laufende Substanz ($R_f$ = 0,34) wird analog Beispiel 2 als Hydrochlorid gefällt. Man erhält die Titelverbindung in einer Ausbeute von 1,40 g (79,1 %) als amorphes Pulver mit einem spezifischen Drehwert von $[\alpha]_D^{25}$ = -56,8° (c = 1, $CH_3OH$).

4.1.57 Beispiel 57
(-)-(1R,5S,2"R)-2'-Hydroxy-2-(2-methoxypropyl)-5,9,9,3'-tetramethyl-6,7-benzomorphan Methansulfonat

a) Ausgangsverbindung

Das hier und in weiteren Beispielen benötigte (+/-)-2'-Hydroxy-5,9,9,3'-tetramethyl-6,7-benzomorphan ist aus dem Stand der Technik noch nicht bekannt. Es kann beispielsweise auf dem für das bekannte 2'-Hydroxy-Derivat [DOS 20 27 077; CA 74 (1971), 125482x] angegebenen Syntheseweg hergestellt werden, indem man anstelle des p-Methoxybenzyllithiums das 4-Methoxy-3-methylbenzyllithium einsetzt. Das beim Ringschluß zum Benzomorphansystem überwiegend erhaltene 2'-Hydroxy-5,9,9,3'-tetramethyl-6,7-benzomorphan wird durch Flash-Säulenchromatographie (vergl. Bsp. 16) gereinigt.

b) Umsetzung zur Titelverbindung

Ausgehend von 2,44 g (0,010 Mol) (+/-)-2'-Hydroxy-5,9,9,3'-tetramethyl-6,7-benzomorphan und 1,6 g (0,013 Mol) (R)-2-Methoxypropionsäurechlorid erhält man analog Beispiel 1 ein Gemisch der zu erwartenden diasteriomeren Basen, die durch Säulenchromatographie an der 100-fachen Menge Kieselgel analog Beispiel 2 getrennt werden. Die langsamer laufende Substanz ($R_f$ = 0,26) wird als Methansulfonat auskristallisiert. Man erhält die Titelverbindung in einer Ausbeute von 1,64 g (79,3 %) mit einem Schmelzpunkt von 266-268°C und einem spezifischen Drehwert von $[\alpha]_D^{25}$ = -95,4° (c=1, $CH_3OH$).

4.1.58 Beispiel 58
(+)-(1S,5R,2"R)-2'-Hydroxy-2-(2-methoxypropyl)-5,9,9,3'-tetramethyl-6,7-benzomorphan Hydrochlorid

Die in Beispiel 57 abgetrennte, schneller laufende Substanz ($R_f$ = 0,29) wird analog Beispiel 2 als Hydrochlorid gefällt. Man erhält die Titelverbindung in einer Ausbeute von 1,60 g (90,4 %) als amorphes Pulver mit einem spezifischen Drehwert von $[\alpha]_D^{25}$ = +73,4° (c = 1, $CH_3OH$).

4.1.59 Beispiel 59
(+)-(1S,5R,2"S)-2'-Hydroxy-2-(2-methoxypropyl)-5,9,9,3'-tetramethyl-6,7-benzomorphan Methansulfonat

Ausgehend von 2,44 g (0,010 Mol) (+/-)-2'-Hydroxy-5,9,9,3'-tetramethyl-6,7-benzomorphan (siehe Beispiel 57) und 1,6 g (0,013 Mol) (S)-2-Methoxypropionsäurechlorid erhält man analog Beispiel 1 ein Gemisch der zu erwartenden diasteriomeren Basen, die durch Säulenchromatographie an der 100-fachen Menge Kieselgel analog Beispiel 2 getrennt werden. Die langsamer laufende Substanz ($R_f$ = 0,26) wird als Methansulfonat auskristallisiert. Man erhält die Titelverbindung in einer Ausbeute von 1,56 g (88,1 %) mit einem Schmelzpunkt von 265-267°C und einem spezifischen Drehwert von $[\alpha]_D^{25}$ = +96,9° (c = 1, $CH_3OH$).

4.1.60 Beispiel 60
(-)-(1R,5S,2"S)-2'-Hydroxy-2-(2-methoxypropyl)-5,9,9,3'-tetramethyl-6,7-benzomorphan Hydrochlorid

Die in Beispiel 59 abgetrennte, schneller laufende Substanz ($R_f$ = 0,29) wird analog Beispiel 2 als Hydrochlorid gefällt. Man erhält die Titelverbindung in einer Ausbeute von 1,36 g (76,8 %) als amorphes Pulver mit einem spezifischen Drehwert von $[\alpha]_D^{25}$ = -74,9° (c = 1, $CH_3OH$).

4.1.61 Beispiel 61
(-)-(1R,5S,2"R)-2',3'-Dihydroxy-2-(2-methoxypropyl)-5,9,9-trimethyl-6,7-benzomorphan Hydrochlorid

a) Ausgangsverbindung

Das hier und in weiteren Beispiel benötigte (+/-)-2',3'-Dihydroxy-5,9,9-trimethyl-6,7-benzomorphan ist aus dem Stand der Technik noch nicht bekannt. Es kann beispielsweise auf dem für das bekannte 2'-Hydroxy-Derivat [DOS 20 27 077; CA 74 (1971), 125482x] angegebenen Syntheseweg hergestellt werden, indem man anstelle des p-Methoxybenzyllithiums das 3,4-Dimethoxybenzyllithium einsetzt. Das beim Ringschluß zum Benzomorphansystem überwiegend erhaltene 2',3'-Dihydroxy-5,9,9-trimethyl-6,7-benzomorphan wird durch Flash-Säulenchromatographie (vergl. Bsp. 16) gereinigt.

b) Umsetzung zur Titelverbindung

Ausgehend von 8,51 g (0,03 Mol) (+/-)-2',3'-Dihydroxy-5,9,9-trimethyl-6,7-benzomorphan Hydrochlorid und 4,1 g (0,033 Mol) (R)-2-Methoxypropionsäurechlorid erhält man analog Beispiel 1 ein Gemisch der zu erwartenden diasteriomeren Basen, die durch Säulenchromatographie an der 100-fachen Menge Kieselgel analog Beispiel 2 getrennt werden. Die langsamer laufende Substanz ($R_f$ = 0,21) wird als Hydrochlorid auskristallisiert.
Man erhält die Titelverbindung in einer Ausbeute von 2,54 g (47,6 %) mit eiem Schmelzpunkt von 167°C (Zers.) und einem spezifischen Drehwert von $[\alpha]_D^{25}$ = -98,6° (c = 0,5; $CH_3OH$).

4.1.62 Beispiel 62
(+)-(1S,5R,2"R)-2',3'-Dihydroxy-2-(2-methoxypropyl)-5,9,9-trimethyl-6,7-benzomorphan Hydrochlorid

Die in Beispiel 61 abgetrennt, schneller laufende Substanz ($R_f$ = 0,29) wird analog Beispiel 2 als Hydrochlorid gefällt. Man erhält die Titelverbindung in einer Ausbeute von 2,28 g (42,7 %) mit einem Schmelzpunkt von 251°C (Zers.) und mit einem spezifischen Drehwert von $[\alpha]_D^{25}$ = +64,3° (c = 0,5; $CH_3OH$).

4.2. Verbindungen (1) nach Verfahren 3.2

4.2.1 Beispiel 63
(±)-2'-Hydroxy-2-(2-methoxyethyl)-5,9,9-trimethyl-6,7-benzomorphan Hydrobromid

0,6 g (0,003 Mol) (±)-2'-Hydroxy-5,9,9-trimethyl-6,7-benzomorphan, 0,72 g (0,0075 Mol) 2-Methoxyethylchlorid, 0,11 g (0,0075 Mol) Natriumiodid und 0,95 g (0,0113 Mol) Natriumhydrogencarbonat werden in 10 ml absolutem Dimethylformamid 23 Stunden bei 95°C gerührt. Anschließend wird das Reaktionsgemisch im Rotationsverdampfer eingedampft, zuletzt bei 95°C und vollem Wasserstrahlvakuum. Der Rückstand wird mit 25 ml Wasser und 25 ml Dichlormethan geschüttelt und die abgetrennte wäßrige Phase noch einmal mit 10 ml Dichlormethan extrahiert. Die vereinigten Extrakte werden mit Wasser gewaschen, mit Natriumsulfat getrocknet und nach dem Abfiltrieren des Trockenmittels - wie oben beschrieben - i. Vak. eingeengt. Der aus der freien Base der Titelverbindung bestehende Eindampfungsrückstand wird analog Beispiel 38 als Hydrobromid kristallisiert. Man erhält die Titelverbindung in einer Ausbeute von 0,48 g (43,5 % d. Th.) mit einem Schmelzpunkt von 247°C (Zers.).

4.2.2 Beispiel 64
(-)-(1R,5S,2"R)-2'-Hydroxy-2-(2-methoxypropyl)-5,9,9-trimethyl-6,7-benzomorphan Hydrochlorid

a) Herstellung des Alkylierungsmittels

Durch Reduktion der aus dem Stand der Technik bekannten (+)-(R)-2-Methoxy-propionsäure mit Lithiumaluminiumhydrid ($LiAlH_4$) erhält man das (-)-(R)-2-Methoxy-propanol mit einem Siedepunkt von 70°C bei einem Druck von 76 mbar und einem spezifischen Drehwert von $[\alpha]_D^{25}$ = -15,8° (c = 100). Analog erhält man aus der ebenfalls bekannten (-)-(S)-2-Methoxy-propionsäure das entsprechende (+)-(S)-2-Methoxy-propanol mit einem Siedepunkt von 70°C bei einem Druck von 76 mbar und einem Drehwert von $[\alpha]_D^{25}$ = +16,5° (c = 100).

Durch Umsetzung von (-)-(R)-2-Methoxypropanol mit p-Toluolsulfonsäurechlorid in Pyridin analog R.S. Tipson [J. Org. Chem. 9 (1944) 235)] erhält man das (+)-(R)-2-Methoxypropyl-p-toluolsulfonat in Form eines gelblichen Öles mit einem spezifischen Drehwert von $[\alpha]_D^{25}$ = + 3,7° (C = 100) und auf analoge Weise aus (+)-(S)-2-Methoxy-propanol das entsprechende (-)-(S)-2-Methoxypropyl-p-toluolsulfonat mit einem Drehwert von $[\alpha]_D^{25}$ = -3,7° (c = 100).

b) Umsetzung zur Titelverbindung

1,16 g (5 mMol) (±)-2'-Hydroxy-5,9,9-trimethyl-6,7-benzomorphan (Beispiel 1) werden mit 1,83 g (7,5 mMol) (R)-2-Methoxypropyl-p-toluolsulfonat, 1,26 g (0,015 Mol) Natriumhydrogencarbonat und 1,12 g (7,5 mMol) Natriumiodid in einer Mischung aus 15 ml absolutem Dimethylformamid und 25 ml absolutem Tetrahydrofuran 60 Stunden unter Rühren auf Rückflußtemperatur erwärmt. Anschließend wird analog Beispiel 63 aufgearbeitet. Man erhält ein Gemisch der beiden zu erwarenden diastereomeren Basen (2 g), das nach Beispiel 1 auch auf anderem Wege hergestellt werden kann. Man trennt in der dort beschriebenen Weise durch Kristallisieren der Hydrochloride und erhält die Titelverbindung in einer Ausbeute von 0,55 g (64,7 % d. Th.) mit einem Schmelzpunkt von 264°C (Zers.) und einem spezifischen Drehwert von $[\alpha]_D^{25}$ = -118,2° (c = 1, $CH_3OH$). Aus der Mutterlauge kann analog Beispiel 2 das (+)-(1S,5R,2"R)-Diastereomere gewonnen werden.

4.2.3 Beispiel 65
(-)-(1R,5S,2"R)-2-(2-Methoxypropyl)-2'-nitro-5,9,9-trimethyl-6,7-benzomorphan Hydrochlorid

2,97 g (0,010 Mol) (±)-2'-Nitro-5,9,9-trimethyl-6,7-benzomorphan Hydrochlorid (Beispiel 34) werden analog Beispiel 64 mit 2,68 g (0,011 Mol) (R)-2-Methoxypropyl-p-toluolsulfonat, 1,65 g (0,011 Mol) Natriumiodid und 2,1 g (0,025 Mol) Natriumhydrogencarbonat umgesetzt. Als Reaktionsprodukt isoliert man - wie dort beschrieben - zunächst ein Gemisch der beiden zu erwartenden diastereomeren Basen (3,2 g). Zur Abtrennung von Nebenprodukten wird dieses in 64 ml Dichlormethan gelöst und die Lösung über eine Säule mit 64 g Aluminiumoxid (Aktivitätsstufe III - neutral - nach Brockmann) filtriert. Nach dem Eluieren mit weiteren 128 ml Dichlormethan werden die vereinigten Eluate im Rotationsverdampfer eingedampft - zuletzt bei 80°C und vollem Wasserstrahlvakuum. Der Rückstand (2,4 g) wird in 10 ml Methanol gelöst und die Lösung nach Ansäuern mit 2,5 N ethanolischer Salzsäure mit Diethylether bis eben zu beginnender Trübung versetzt. Es kristallisiert die Titelverbindung, die nach 3 Tagen Stehen bei Raumtemperatur abgesaugt, mit Ethanol-Ether-Mischung (1:1), dann mit Ether gewaschen und bei 80°C getrocknet wird. Man erhält die Titelverbindung in einer Ausbeute von 0,84 g (45,5 % d. Th.) mit einem Schmelzpunkt von 226°C (Zers.) und einem spezifischen Drehwert von $[\alpha]_D^{25}$ = -120° (c = 1, $CH_3OH$). Aus der Mutterlauge kann gewünschtenfalls die stereoisomere (+)-(1S,5R,2"R)-Verbindung gewonnen werden.

4.2.4 Beispiel 66
(+)-(1S,5R,2"S)-2-(2-Methoxypropyl)-2'-nitro-5,9,9-trimethyl-6,7-benzomorphan Hydrochlorid

Ausgehend von 2,97 g (0,010 Mol) (±)-2'-Nitro-5,9,9-trimethyl-6,7-benzomorphan Hydrochlorid und 2,68 g (0,011 Mol) (S)-2-Methoxypropyl-p-toluolsulfonat erhält man analog Beispiel 65 die Titelverbindung in einer Ausbeute von 0,96 g (52,0 % d. Th.) mit einem Schmelzpunkt von 226°C (Zers.) und einem spezifischen Drehwert von $[\alpha]_D^{25}$ = +121° (c = $CH_3OH$). Aus der Mutterlauge kann analog Beispiel 2 die stereoisomere (-)-(1R,5S,2"S)-Form gewonnen werden.

4.2.5 Beispiel 67
(-)-(1R,5S,2"R)-2-(2-Methoxypropyl)-3'-nitro-5,9,9-trimethyl-6,7-benzomorphan

a) Herstellung der Ausgangsverbindung

Das hier und in weiteren Beispielen benötigte (±)-3'-Nitro-5,9,9-trimethyl-6,7-benzomorphan entsteht neben dem (±)-2'-Nitro-Isomeren bei der Nitrierung von 5,9,9-Trimethyl-6,7-benzomorphan (Beispiel 34) in einem Verhältnis von ca.

1 : 3. Man gewinnt es aus der Mutterlauge des als Hydrochlorid kristallisierten 2'-Nitro-Isomeren über die zunächst hergestellte Base. Diese wird als Oxalat (Schmelzpunkt 158°C, (Zers.)) aus der zehnfachen Menge Methanol kristallisiert. Das Oxalat wird in die Base zurücküberführt (nicht kristallisiert), die für die nachfolgend beschriebene Umsetzung verwendet wird. Eine Probe der Base, kristallisiert als Methansulfonat, schmilzt bei 242°C (Zers.).

b) Umsetzung zur Titelverbindung

5,2 g (0,02 Mol) (±)-3'-Nitro-5,9,9-trimethyl-6,7-benzomorphan werden analog Beispiel 65 mit 5,36 g (0,022 Mol) (R)-2-Methoxypropyl-p-toluolsulfonat umgesetzt. Als Reaktionsprodukt erhält man ein Gemisch der beiden zu erwartenden steroisomeren Basen (8 g), das - wie in Beispiel 65 beschrieben - durch Filtrieren über Aluminiumoxid gereinigt wird (4,7 g gereinigtes Basengemisch). Im Dünnschichtchromatogramm (Kieselgel 60, Toluol-Essigester 85:15, 3 mal entwickeln) weisen die Diastereomeren $R_f$-Werte von jeweils 0,81 und 0,85 auf. Eine Trennung von 1 g Diasteroemeren-Gemisch auf 300 g Kieselgel auf dem Wege der Säulenchromatographie liefert die Titelverbindung (100 mg) mit einem $R_f$-Wert von 0,81 als bräunliches sehr zähflüssiges Öl, das nicht kristallisiert.

4.2.6 Beispiel 68
(+)-(1S,5R,2"R)-2-(2-Methoxypropyl)-3'-nitro-5,9,9-trimethyl-6,7-benzomorphan

Bei der in Beispiel 67 beschriebenen Säulenchromatographie des Diasteromerengemisches wurden außer der dort isolierten (-)-(1R,5S,2"R)-Form ($R_f$ = 0,81) auch 100 mg der (+)-(1S,5R,2"S)-Form ($R_f$ = 0,85) als bräunliches, sehr zähflüssiges Öl gewonnen, das nicht kristallisiert.

4.2.7 Beispiel 69
(-)-(1R,5S,2"R)-2'-Chlor-2-(2-methoxypropyl)-5,9,9-trimethyl-6,7-benzomorphan Hydrochlorid

a) Herstellung der Ausgangsverbindung

Das hier und in weiteren Beispielen benötigte (±)-2'-Chlor-5,9,9-trimethyl-6,7-benzomorphan kann z.B. aus dem 2'-Nitro-Analogon (Beispiel 34) hergestellt werden, indem man letzteres zur 2'-Amino-Verbindung hydriert (Dihydrochlorid: Schmelzpunkt 283°C, (Zers.)) und dieses durch Sandmeyer-Reaktion in die gewünschte 2'-Chlor-Verbindung überführt (Hydrobromid: Schmelzpunkt > 310°C, (Zers.).

b) Umsetzung zur Titelverbindung

3,3 g (0,010 Mol) (±)-2'-Chlor-5,9,9-trimethyl-6,7-benzomorphan Hydrobromid und 2,68 g (0,011 Mol) (R)-2-Methoxypropyl-p-toluolsulfonat werden analog Beispiel 65 zu einem Gemisch der beiden zu erwartenden diastereomeren Basen umgesetzt und - wie dort beschrieben - durch Filtration über Aluminiumoxid gereingt. Das gereinigte Diastereromerengemisch wird analog Beispiel 1 durch Kristallisation der Hydrochloride getrennt. Man erhält die Titelverbindung in einer Ausbeute von 0,27 g (15,1 % d. Th.) mit einem Schmelzpunkt von 287°C (Zers.) und einem Drehwert von $[\alpha]_D^{25}$ = -102,6° (c = 1, $CH_3OH$).

4.2.8 Beispiel 70
(+)-(1S,5R,2"R)-2'-Chlor-2-(2-methoxypropyl)-5,9,9-trimethyl-6,7-benzomorphan Hydrochlorid

Die Mutterlauge von Beispiel 69 werden eingedampft und der Rückstand mit der eben benötigten Menge siedenden Methanols gelöst. Nach Zusatz von Diethylether bis zu eben beginnender Trübung und gleichzeitiger Kristallisation erhält man die Titelverbindung, die - nach Stehen über einen Zeitraum von ca. 12 Stunden bei Raumtemperatur - abgesaugt, mit Ethanol-Ether 1:1, dann mit Ether gewaschen und bei 80°C getrocknet wird. Ausbeute 0,56 g (31,3 % d. Th.), Schmelzpunkt: 289°C, spezifischer Drehwert: $[\alpha]_D^{25}$ = +67,9°.

4.2.9 Beispiel 71
(+)-(1S,5R,2"S)-2'-Chlor-2-(2-methoxypropyl)-5,9,9-trimethyl-6,7-benzomorphan Hydrochlorid

Ausgehend von 3,3 g (0,010 Mol) (±)-2'-Chlor-5,9,9-trimethyl-6,7-benzomorphan und 2,68 g (0,011 Mol) (S)-2-Methoxypropyl-p-toluolsulfonat erhält man analog Beispiel 69 die Titelverbindung in einer Ausbeute von 0,41 g (22,9 % d. Th.) mit einem Schmelzpunkt von 288°C (Zers.) und einem spezifischen Drehwert von $[\alpha]_D^{25}$ = +101,7° (c = 1, $CH_3OH$).

4.2.10 Beispiel 72
(-)-(1R,5S,2"S)-2'-Chlor-2-(2-methoxypropyl)-5,9,9-trimethyl-6,7-benzomorphan Hydrochlorid

Aus den Mutterlaugen von Beispiel 71 erhält man analog Beispiel 70 die Titelverbindung in einer Ausbeute von 0,75 g (41,9 % d. Th.) mit einem Schmelzpunkt von 288°C und einem spezifischen Drehwert von $[\alpha]_D^{25}$ = -68,6° (c = 1, $CH_3OH$).

4.3. Verbindungen (1) nach Verfahren 3.3

4.3.1 Beispiel 73
(-)-(1R,5S,2"S)-2'-Methoxy-2-(2-methoxypropyl)-5,9,9-trimethyl-6,7-benzomorphan Methansulfonat

0,68 g (2 mMol) (-)-(1R,5S,2"S)-2'-Hydroxy-2-(2-methoxypropyl)-5,9,9-trimethyl-6,7-benzomorphan Hydrochlorid (Beispiel 4) werden in 20 ml absolutem Dimethylformamid unter Rühren und Eiskühlung mit 0,13 g 77 %-iger Natriumhydrid-Suspension in Paraffin (4,2 mMol) versetzt. Nach 30 Minuten Rühren werden 0,31 g (2,2 mMol) Methyliodid, gelöst in 5 ml Dimethylformamid, innerhalb von 5 Minuten zugetropft. Anschließend wird 3 Stunden bei Raumtemperatur weitergerührt und dann im Rotationsverdampfer, zuletzt bei 95°C und vollem Wasserstrahlvakuum, eingedampft. Der Rückstand wird mit 25 ml Dichlormethan und 25 ml Wasser geschüttelt. Die abgetrennte wäßrige Phase wird noch einmal mit 15 ml Dichlormethan extrahiert. Nach dem Waschen mit 15 ml Wasser werden die vereinigten Extrakte eingedampft und der Rückstand analog Beispiel 28 als Methansulfonat kristallisiert. Man erhält die Titelverbindung in einer Ausbeute von 0,38 g) (46,0 %) mit einem Schmelzpunkt von 150-152°C (Zers.) und einem spezifischen Drehwert von $[\alpha]_D^{25}$ = -68,7° (c = 1, $CH_3OH$).

4.3.2 Beispiel 74
(+)-(1S,5R,2"R)-2'-Methoxy-2-(2-methoxypropyl)-5,9,9-trimethyl-6,7-benzomorphan Methansulfonat

Ausgehend von 0,68 g (2 mMol) (+)-(1S,5R,2"R)-2'-Hydroxy-2-(2-methoxypropyl)-5,9,9-trimethyl-6,7-benzomorphan Hydrochlorid (Beispiel 2) erhält man analog Beispiel 73 die Titelverbindung in einer Ausbeute von 0,38 g (46,0 % d. Th.) mit einem Schmelzpunkt von 150-152°C (Zers.) und einem spezifischen Drehwert von $[\alpha]_D^{25}$ = +67,2° (c = 1,$CH_3OH$).

4.3.3 Beispiel 75
(-)-(1R,5S,2"R)-2'-Ethoxy-2-(2-methoxypropyl)-5,9,9-trimethyl-6,7-benzomorphan Methansulfonat

Ausgehend von 0,34 g (1 mMol) (-)-(1R,5S,2"R)-2'-Hydroxy-2-(2-methoxypropyl)-5,9,9-trimethyl-6,7-benzomorphan Hydrochlorid (Beispiel 1) und 0,17 g (0,0011 Mol) Ethyliodid erhält man analog Beispiel 73 die Titelverbindung in einer Ausbeute von 0,30 g (70,2 % d. Th.) mit einem Schmelzpunkt von 146-148°C (Zers.) und einem spezifischen Drehwert von $[\alpha]_D^{25}$ = -93,4° (c = 1, $CH_3OH$).

4.3.4 Beispiel 76
(-)-(1R,5S,2"R)-2'-Acetoxy-2-(2-methoxypropyl)-5,9,9-trimethyl-6,7-benzomorphan Methansulfonat

0,68 g (2 mMol) (-)-(1R,5S,2"R)-2'-Hydroxy-2-(2-methoxypropyl)-5,9,9-trimethyl-6,7-benzomorphan Hydrochlorid (Beispiel 1) werden in 15 ml absolutem Dichlormethan gelöst. Nach Zugabe von 0,51 g (5 mMol) Triethylamin wird die Lösung bei Raumtemperatur unter Rühren innerhalb von ca. 10 Minuten mit 0,19 g (2,4 mMol) Acetylchlorid, gelöst in 5 ml Dichlormethan, versetzt. Anschließend wird noch 2 Stunden bei Raumtemperatur weitergerührt. Dann wird dreimal mit je 10 ml Eiswasser gewaschen, mit Natriumsulfat getrocknet und nach dem Abfiltrieren des Trockenmittels eingedampft (Rotationsverdampfer, zuletzt bei einer Temperatur von 80°C und bei vollem Wasserstrahlvakuum). Der Rückstand, der aus der freien Base der Titelverbindung besteht (0,6 g, 1,74 mMol) wird in 3 ml Ethanol gelöst und durch Zugabe von Methansulfonsäure (0,16 g, (1,74 mMol) in 3 ml Ethanol) eben gerade bis pH 4 angesäuert. Nach Versetzen mit Diethylether bis eben zur Trübung kristallisiert die Titelverbindung, die - nach dem Stehen über einen Zeitraum von ca. 12 h im Kühlschrank - abgesaugt, mit Ethanol-Ether 1:2 gewaschen und bei 80°C getrocknet wird. Ausbeute 0,7 g (79,3 % d. Th.), Schmelzpunkt: 183-184°C (Zers.), $[\alpha]_D^{25}$ = -83,9° (c = 1, $CH_3OH$).

4.3.5 Beispiel 77
(+)-(1S,5R,2"S)-2'-Acetoxy-2-(2-methoxypropyl)-5,9,9-trimethyl-6,7-benzomorphan Methansulfonat

Ausgehend von 0,68 g (0,002 Mol) (+)-(1S,5R,2"S)-2'-Hydroxy-2-(2-methoxypropyl)-5,9,9-trimethyl-6,7-benzomorphan Hydrochlorid (Beispiel 3) erhält man in Analogie zu Beispiel 76 die Titelverbindung in einer Ausbeute von 0,6 g

(68,0 % d. Th.) mit einem Schmelzpunkt von 183-184°C (Zers.) und einem spezifischen Drehwert von $[\alpha]_D^{25}$ = +82,8° (c = 1, $CH_3OH$).

4.3.6 Beispiel 78
(-)-(1R,5S,2"S)-2'-Acetoxy-2-(2-methoxypropyl)-5,9,9-trimethyl-6,7-benzomorphan Methansulfonat

Ausgehend von 0,68 g (0,002 Mol) (-)-(1R,5S,2"S)-2'-Hydroxy-2-(2-methoxypropyl)-5,9,9-trimethyl-6,7-benzomorphan Hydrochlorid (Beispiel 4) erhält man analog Beispiel 76 die Titelverbindung in einer Ausbeute von 0,58 g (65,7 % d. Th.) mit einem Schmelzpunkt von 183°C (Zers.) und einem spezifischen Drehwert von $[\alpha]_D^{25}$ = -57,1° (c = 1, $CH_3OH$).

4.3.7 Beispiel 79
(+)-(1S,5R,2"R)-2'-Acetoxy-2-(2-methoxypropyl)-5,9,9-trimethyl-6,7-benzomorphan Methansulfonat

Ausgehend von 0,68 g (2 mMol) (+)-(1S,5R,2"R)-2'-Hydroxy-2-(2-methoxypropyl)-5,9,9-trimethyl-6,7-benzomorphan Hydrochlorid (Beispiel 2) erhält man analog Beispiel 76 die Titelverbindung in einer Ausbeute von 0,67 g (75,9 % d. Th.) mit einem Schmelzpunkt von 183°C und einem spezifischen Drehwert $[\alpha]_D^{25}$ = +58,2° (c = 1, $CH_3OH$).

4.3.8 Beispiel 80
(-)-(1R,5S,2"R)-2-(2-Methoxypropyl)-2'-propionoxy-5,9,9-trimethyl-6,7-benzomorphan Methansulfonat

Ausgehend von 0,34 g (1 mMol) (-)-(1R,5S,2"R)-2'-Hydroxy-2-(2-methoxypropyl)-5,9,9-trimethyl-6,7-benzomorphan Hydrochlorid (Beispiel 1) und 0,15 g (1,1 mMol) Propionsäureanhydrid erhält man analog Beispiel 76 die Titelverbindung in einer Ausbeute von 0,21 g (46,1 % d. Th.) mit einem Schmelzpunkt von 145°C (Zers.) und einem spezifischen Drehwert von $[\alpha]_D^{25}$ = -78,3° (c = 1, $CH_3OH$).

4.3.9 Beispiel 81
(-)-(1R,5S,2"R)-2-(2-Methoxypropyl)-5,9,9-trimethyl-6,7-benzomorphan Hydrochlorid

a) (-)-(1R,5S,2"R)-2-(2-Methoxypropyl)-2'-(1-phenyl-5-tetrazolyloxy)-5,9,9-trimethyl-6,7-benzomorphan Hydrochlorid

1,36 g (4 mMol) (-)-(1R,5S,2"R)-2'-Hydroxy-2-(2-methoxypropyl)-5,9,9-trimethyl-6,7-benzomorphan Hydrochlorid (Beispiel 1) werden mit 0,79 g (4,4 mMol) 5-Chlor-1-phenyltetrazol und 1,36 g (0,01 Mol) trockenem, fein pulverisiertem Kaliumcarbonat in 78 ml absolutem Aceton 6 Tage unter Rühren auf Rückflußtemperatur erwärmt. Anschließend wird die Lösung eingedampft und der Rückstand mit 50 ml Wasser versetzt. Man extrahiert zweimal mit je 25 ml Dichlormethan und wäscht die vereinigten Extrakte mit 15 ml Wasser. Nach dem Trocknen mit Natriumsulfat und Abfiltrieren des Trockenmittels wird die Reaktionsmischung im Rotationsverdampfer eingedampft, zuletzt bei 80°C und vollem Wasserstrahlvakuum. Der Rückstand wird mit 5 ml Ethanol gelöst und nach Ansäuern mit ethanolischer Salzsäure mit Diethylether bis eben zur Trübung versetzt. Es kristallisiert das Zwischenprodukt, das - nach Stehen über einen Zeitraum von ca. 12 h im Kühlschrank - abgesaugt, mit einer Ethanol-Ether-Mischung (1:1), dann mit Ether gewaschen und bei 80°C getrocknet wird. Ausbeute 1,67 g (86,3 % d. Th.), Schmelzpunkt 241°C (Zers.).

b) Hydrierung des Zwischenproduktes zur Titelverbindung

Das Zwischenprodukt (1,67 g, 3,5 mMol) wird in Gegenwart von 0,7 g 10 %-iger Palladium-Kohle in 70 ml Eisessig unter einem Wasserstoffdruck von 5 bar und bei Raumtemperatur bis zur vollständigen Umsetzung hydriert (6 - 10 Stunden). Dann wird vom Katalysator abgesaugt, das Filtrat eingedampft und der Rückstand - nach dem Versetzen mit 50 ml Wasser und überschüssigem Ammoniak - mit Dichlormethan (2 mal je 25 ml) extrahiert. Die vereinigten Extrakte werden mit Wasser gewaschen, mit Natriumsulfat getrocknet und nach dem Abfiltrieren des Trockenmittels im Rotationsverdampfer, zuletzt bei 80°C und vollem Wasserstrahlvakuum, eingedampft. Der Rückstand wird analog Beispiel 2 als Hydrochlorid kristallisiert. Man erhält die Titelverbindung in einer Gesamtausbeute von 0,5 g (38,5 % d. Th.) mit einem Schmelzpunkt von 225°C (Zers.) und einem spezifischen Drehwert von $[\alpha]_D^{25}$ = -101,5° (c = 1 $CH_3OH$).

4.3.10 Beispiel 82
(+)-(1S,5R,2"S)-2-(2-Methoxypropyl)-5,9,9-trimethyl-6,7-benzomorphan Hydrochlorid

Ausgehend von 1,36 g (0,004 Mol) (+)-(1S,5R,2"S)-2'-Hydroxy-2-(2-methoxypropyl)-5,9,9-trimethyl-6,7-benzomorphan Hydrochlorid (Beispiel 3) erhält man analog Beispiel 81 über das entsprechende Zwischenprodukt (1,65 g, 85,1 % dh. Th., Schmelzpunkt 241° (Zers.)) die Titelverbindung in einer Gesamtausbeute von 0,64 g (49,4 % d. Th.) mit einem Schmelzpunkt von 225°C (Zers.) und einem spezifischen Drehwert von $[\alpha]_D^{25}$ = +106,9° (c = 1, $CH_3OH$).

4.3.11 Beispiel 83
(-)-(1R,5S,2"S)-2-(2-Methoxypropyl)-5,9,9-trimethyl-6,7-benzomorphan Oxalat

Ausgehend von 1,36 g (4 mMol) (-)-(1R,5S,2"S)-2'-Hydroxy-2-(2-methoxypropyl)-5,9,9-trimethyl-6,7-benzomorphan Hydrochlorid (Beispiel 4) erhält man analog Beispiel 81 über das entsprechende Zwischenprodukt (1,73 g, 89,4 % d. Th., Schmelzpunkt 218,5°C (Zers.)) die Titelverbindung in Form der freien Base (0,7 g). Diese wird mit 2 ml Isopropanol gelöst und mit der entsprechenden Molmenge Oxalsäure (0,22 g) versetzt. Es kristallisiert die Titelverbindung während man nach und nach unter Rühren 50 ml Diethylether zufügt. Nach dem Aufbewahren über einen Zeitraum von ca. 12 h im Kühlschrank wird abgesaugt, mit Isopropanol-Ether, dann mit Ether gewaschen und bei 80°C getrocknet. Man erhält die Titelverbindung in einer Gesamtausbeute von 0,8 g (53,0 % d. Th.) mit einem Schmelzpunkt von 135°C (Zers.) und einem spezifischen Drehwert von $[\alpha]_D^{25}$ = -60,0° (c = 1, $CH_3OH$).

4.3.12 Beispiel 84
(+)-(1S,5R,2"R)-2-(2-Methoxypropyl)-5,9,9-trimethyl-6,7-benzormorphan Oxalat

Ausgehend von 1,36 g (4 mMol) (+)-(1S,5R,2"R)-2'-Hydroxy-2-(2-methoxypropyl)-5,9,9-trimethyl-6,7-benzomorphan Hydrochlorid (Beispiel 2) erhält man analog Beispiel 81 über das entsprechende Zwischenprodukt (1,65 g, 85,1 % d. Th., Schmelzpunkt 217°C (Zers.)) die Base der Titelverbindung, die analog Beispiel 83 als Oxalat kristallisiert wird. Gesamtausbeute 0,6 g (39,7 % d. Th.), Schmelzpunkt: 135°C (Zers.), spezifischer Drehwert: $[\alpha]_D^{25}$ = + 60,2° (c = 1, $CH_3OH$).

4.3.13 Beispiel 85
(-)-(1R,5S,2"R)-2'-Acetamido-2-(2-methoxypropyl)-5,9,9-trimethyl-6,7-benzomorphan Hydrochlorid

0,70 g (1,87 mMol) (-)-(1R,5S,2"R)-2'-Amino-2-(2-methoxypropyl)-5,9,9-trimethyl-6,7-benzomorphan Dihydrochlorid (Beispiel 34) werden in 20 ml absolutem Dichlormethan gelöst, mit 0,76 g (7,4 mMol) Triethylamin und danach unter Rühren bei Raumtemperatur tropfenweise mit 0,17 g (2,1 mMol) Acetylchlorid, gelöst in 5 ml Dichlormethan, versetzt. Anschließend wird 1 Stunde auf Rückflußtemperatur erwärmt, danach abgekühlt, dreimal mit je 10 ml Wasser gewaschen, mit Natriumsulfat getrocknet und nach dem Abfiltrieren des Trockenmittels im Rotationsverdampfer, zuletzt bei 80°C und vollem Wasserstrahlvakuum, eingedampft. Der Rückstand wird in 2 ml Methanol gelöst und die Lösung nach dem Ansäuern mit 2,5 N ethanolischer Salzsäure mit Diehtylether bis eben zu beginnender Trübung versetzt. Das sich ausscheidende Kristallisat wird - nach dem Stehen über einen Zeitraum von ca. 12 h im Kühlschrank - abgesaugt, mit einer Ethanol-Ether-Mischung (1:1), dann mit Ether gewaschen und bei 80°C getrocknet. Man erhält die Titelverbindung in einer Ausbeute von 0,49 g (68,8 % d. Th.) mit einem Schmelzpunkt von 252°C (Zers.) und einem spezifischen Drehwert von $[\alpha]_D^{25}$ = -108,2° (c = 1, $CH_3OH$).

4.3.14 Beispiel 86
(+)-(1S,5R,2"S)-2'-Acetamido-2-(2-methoxypropyl)-5,9,9-trimethyl-6,7-benzomorphan Hydrochlorid

Ausgehend von 1,07 g (2,58 mMol) (+)-(1S,5R,2"S)-2-Amino-2-(2-methoxypropyl)-5,9,9-trimethyl-6,7-benzomorphan Hydrochlorid (Beispiel 36) erhält man analog Beispiel 85 die Titelverbindung in einer Ausbeute von 0,73 g (74,3 % d. d. Th.) mit einem Schmelzpunkt von 249°C (Zers.) und einem spezifischen Drehwert von $[\alpha]_D^{25}$ = +109,5° (c = 1, $CH_3OH$).

4.3.15 Beispiel 87
(-)-(1R,5S,2"S)-2'-Acetamido-2-(2-methoxypropyl)-5,9,9-trimethyl-6,7-benzomorphan

1,0 g (0,0026 Mol) (-)-(1R,5S,2"S)-2'-Amino-2-(2-methoxypropyl)-5,9,9-trimethyl-6,7-benzomorphan Dihydrochlorid werden analog Beispiel 85 umgesetzt. Das Reaktionsprodukt wird - wie dort beschrieben - isoliert und aus 0,5 ml Isopropanol und 5 ml Petrolether kristallisiert. Man erhält die Titelverbindung in einer Ausbeute von 0,7 g (76,4 % d. Th.) mit einem Schmelzpunkt von 128°C und einem spezifischen Drehwert von $[\alpha]_D^{25}$ = -89,3° (c = 1, $CH_3OH$ - 1 N HCl 1:1).

4.3.16 Beispiel 88
(+)-(1S,5R,2"R)-2'-Acetamido-2-(2-methoxypropyl)-5,9,9-trimethyl-6,7-benzomorphan

Ausgehend von 1,11 g (2,5 mMol) (+)-(1S,5R,2"R)-2'-Amino-2-(2-methoxypropyl)-5,9,9-trimethyl-6,7-benzomorphan Dimethansulfonat (Beispiel 35) erhält man analog Beispiel 87 die Titelverbindung in einer Ausbeute von 0,37 g (47,7 % d. Th.) mit einem Schmelzpunkt von 129°C und einem spezifischen Drehwert von $[\alpha]_D^{25}$ = +89,9° (c = 1, $CH_3OH$ - 1 N HCl 1:1).

4.3.17 Beispiel 89
(-)-(1R,5S,2"R)-3'-Brom-2'-hydroxy-2-(2-methoxypropyl)-5,9,9-trimethyl-6,7-benzomorphan Hydrochlorid

0,85 g (2,5 mMol) (-)-(1R,5S,2"R)-2'-Hydroxy-2-(2-methoxypropyl)-5,9,9-trimethyl-6,7-benzomorphan Hydrochlorid (Beispiel 1) werden in 30 ml Eisessig suspendiert. Die Suspension wird unter Rühren bei Raumtemperatur mit 0,15 ml (0,47 g, 2,9 mMol) Brom versetzt. Nach 2 Stunden Rühren wird im Rotationsverdampfer, zuletzt bei 80°C und vollem Wasserstrahlvakuum, eingedampft. Der Rückstand wird unter Schütteln mit 25 ml Wasser und 25 ml Dichlormethan mit Ammoniak im Überschuß versetzt. Die abgetrennte wäßrige Phase wird noch einmal mit 15 ml Dichlormethan extrahiert, die vereinigten Extrakte mit 15 ml Wasser gewaschen, mit Natriumsulfat getrocknet und nach dem Abfiltrieren des Trockenmittels - wie oben beschrieben - eingedampft. Der Rückstand (1 g) wird über eine Säule mit 250 g Kieselgel - analog Beispiel 2 - unter Verwendung einer Mischung Dichlormethan-Methanol-konz. Ammoniak 90:10:0,5 chromatographisch gereinigt. Die Eluate mit der reinen Substanz werden wie oben eingedampft und der Rückstand analog Beispiel 2 als Hydrochlorid kristallisiert. Man erhält die Titelverbindung in einer Ausbeute von 0,75 g (65,8 % d. Th.) mit einem Schmelzpunkt von 245°C (Zers.) und einem spezifischen einem spezifischen Drehwert von $[\alpha]_D^{25}$ = -82,5° (c = 1, $CH_3OH$).

4.3.18 Beispiel 90
(+)-(1R,5R,2"R)-2'-Hydroxy-1'-nitro-2-(2-methoxypropyl)-5,9,9-trimethyl-6,7-benzomorphan Hydrochlorid

0,85 g (2,5 mMol) (-)-(1R,5S,,2"R)-2'-Hydroxy-2-(2-methoxypropyl)-5,9,9-trimethyl-6,7-benzomorphan Hydrochlorid werden bei 20°C in 30 ml Eisessig suspendiert und unter Rühren mit 0,18 ml (0,25 g) 65 %-iger Salpetersäure (2,6 mMol $HNO_3$) versetzt. Innerhalb von ca. 10 Minuten entsteht eine klare, gelbe Lösung, die sich nach weiteren 5 Minuten braun färbt und sich unter Ausscheiden einer kristallinen Verbindung zu trüben beginnt. Nach einer Reaktionszeit von insgesamt einer Stunde wird abgesaugt und mit einer Eisessig-Diethylether-Mischung (1:1), dann mit Ether gewaschen und bei 80°C getrocknet. Die Kristalle (0,35 g) werden in einer heißen Methanol-Wasser-Mischung (17,5 ml + 1,75 ml) gelöst und die Lösung mit Diethylether (40 ml) versetzt. Die auskristallisierende Substanz wird nach Kühlen im Eisbad abgesaugt, mit Ether gewaschen und bei 80°C getrocknet. Man erhält die Titelverbindung in einer Ausbeute von 0,32 g (33,3 % d. Th.) mit einem Schmelzpunkt von > 245°C (unter allmählicher Braunfärbung und Zersetzung); spezifischer Drehwert $[\alpha]_D^{25}$ = +87,0° (c = 1, $CH_3OH$).

4.3.19 Beispiel 91
(-)-(1R,5S,2"R)-2'-Hydroxy-2-(2-methoxypropyl)-3'-nitro-5,9,9-trimethyl-6,7-benzomorphan Hydrochlorid

Bei der in Beispiel 90 beschriebenen Nitrierung entstehen aus der Ausgangsverbindung ($R_f$ = 0,57) das oben beschriebene 1'-Nitro-Derivat ($R_f$ = 0,45), das isomere 3'-Nitro-Derivat ($R_f$ = 0,72) und das 1',3'-Dinitro-Derivat ($R_f$ = 0,10) (DC: Kieselgel 60, Chloroform-Methanol-konz. Ammoniak 90:10:0,5). Die nach dem Absaugen der 1'-Nitro-Verbindung verbleibende Mutterlauge (Beispiel 90) wird eingedampft und der Rückstand in die freien Basen überführt. Diese werden in 10 ml Dichlormethan gelöst und die gelbe Lösung über 25 g Aluminiumoxid (Aktivität III, neutral) filtriert. Man eluiert mit Dichlormethan bis das Eluat nicht mehr intensiv gelb gefärbt ist und dampft dann das Eluat ein. Der Rückstand (0,35 g) wird in 2,5 ml Ethanol gelöst und die Lösung nach dem Ansäuern mit ethanolischer Salzsäure mit Diethylether bis eben zur Trübung versetzt. Nach dem Stehen über einen Zeitraum von ca. 12 h im Kühlschrank wird abgesaugt, mit einer Ethanol-Ether-Mischung (1:1) gewaschen und bei einer Temperatur von 80°C getrocknet. Man erhält die Titelverbindung in einer Ausbeute von 0,40 g (41,6 % d. Th.) mit einem Schmelzpunkt von 229-230°C (Zers.) und einem spezifischen Drehwert von $[\alpha]_D^{25}$ = -110,1° (c = 1, $CH_3OH$).

4.3.20 Beispiel 92
(+)-(1R,5R,2"R)-1',3'-Dinitro-2'-hydroxy-2-(2-methoxypropyl)-5,9,9-trimethyl-6,7-benzomorphan Hydrochlorid

1,52 g (5 mMol) (-)-(1R,5S,2"R)-2'-Hydroxy-2-(2-methoxypropyl)-5,9,9-trimethyl-6,7-benzomorphan (Beispiel 1) werden in 3 ml Eisessig gelöst und die Lösung unter Rühren bei 3-4°C innerhalb von 1,5 Stunden mit einer Mischung von 3,4 ml Eisessig und 5,42 ml 100 %-iger Salpetersäure versetzt. Anschließend rührt man noch 3 Stunden bei Raumtemperatur nach. Dann wird die gelbe Reaktionsmischung in 50 g Eiswasser gegossen, die Lösung ammoniakalisch gestellt und 3 mal mit je 20 ml Dichlormethan extrahiert. Nach Waschen der vereinigten Extrakte mit Wasser und Trocknen über Natriumsulfat sowie Abfiltrieren des Trockenmittels wird die Reaktionsmischung im Rotationsverdamper, zuletzt bei 80°C und vollem Wasserstrahlvakuum, eingedampft. Der Rückstand wird aus einer Ethanol-Ether-Mischung kristallisiert. Man erhält die Titelverbindung in einer Ausbeute von 1,0 g (46,5 % d. Th.) mit einem Schmelzpunkt von > 300°C (Zers.) und einem spezifischen Drehwert von $[\alpha]_D^{25}$ = +27,9° (c = 1, $CH_3OH$).

4.3.21 Beispiel 93
(-)-(1R,5R,2"R)-1'-Amino-2'-hydroxy-2-(2-methoxypropyl)-5,9,9-trimethyl-6,7-benzomorphan Hydrochlorid

0,35 g (1,0 mMol) (+)-(1R,5R,2"R)-2'-Hydroxy-2-(2-methoxypropyl)-1'-nitro-6,7-benzomorphan Hydrochlorid (Beispiel 90) werden in 40 ml Methanol in Gegenwart von 0,1 g Palladium-Kohle (5 % Pd) 4 Stunden unter einem Wasserstoffdruck von 5 bar bei Raumtemperatur hydriert. Nach dem Abfiltrieren des Katalysators das Reaktionsgemisch wird im Rotationsverdampfer, zuletzt bei 80°C und vollem Wasserstrahlvakuum, eingedampft und der Rückstand aus 10 ml Ethanol kristallisiert. Nach dem Abkühlen im Eisbad werden die Kristalle abgesaugt, mit einer Ethanol-Ether-Mischung gewaschen und bei 80°C getrocknet. Man erhält die Titelverbindung in einer Ausbeute von 0,17 g (52,7 % d. Th.) mit einem Schmelzpunkt von 282°C (nach langsamer Zersetzung ab 250°C) und einem spezifischen Drehwert von $[\alpha]_D^{25}$ = -128,3° (c = 1, $CH_3OH$).

4.3.22 Beispiel 94
(-)-(1R,5S,2"R)-3'-Amino-2'-hydroxy-2-(2-methoxypropyl)-5,9,9-trimethyl-6,7-benzomorphan Hydrochlorid

Ausgehend von 0,39 g (1,0 mMol) (-)-(1R,5S,2"R)-2'-Hydroxy-2-(2-methoxypropyl)-3'-nitro-5,9,9-trimethyl-6,7-benzomorphan Hydrochlorid (Beispiel 91) erhält man analog Beispiel 93 die Titelverbindung in einer Ausbeute von 0,27 g (76,1 % d. Th.) mit einem Schmelzpunkt von 268°C (Zers.) und einem spezifischen Drehwert von $[\alpha]_D^{25}$ = -99,7° (c = 0,5, $CH_3OH$).

4.3.23 Beispiel 95
(-)-(1R,5R,2"R)-1',3'-Diamino-2'-hydroxy-2-(2-methoxypropyl)-5,9,9-trimethyl-6,7-benzomorphan Hydrochlorid

Ausgehend von 0,70 g (1,63 mMol) (+)-(1R,5R,2"R)-1',3'-Dinitro-2'-hydroxy-2-(2-methoxypropyl)-5,9,9-trimethyl-6,7-benzomorphan Hydrochlorid (Beispiel 92) erhält man analog Beispiel 93 die Titelverbindung in einer Ausbeute von 0,20 g (33,2 % d. Th.) mit einem Schmelzpunkt von 228°C (Zers.) und einem spezifischen Drehwert von $[\alpha]_D^{25}$ = -111,7° (c = 0,5, $CH_3OH$).

4.3.24 Beispiel 96
(-)-(1R,5S,2"R)-3'-Amino-2-(2-methoxypropyl)-5,9,9-trimethyl-6,7-benzomorphan

Das nach Beispiel 67 erhaltene Diastereomerengemisch aus (-)-(1R,5S,2"R)- und (+)-(1S,5R,2"R)-2-(2-Methoxypropyl)-3'-nitro-5,9,9-trimethyl-6,7-benzomorphan (3,7 g) wird in 75 ml Methanol gelöst und in Gegenwart von 0,8 g Palladium-Kohle (5 % Pd) unter einem Wasserstoffdruck von 5 bar und einer Temperatur von 20°C hydriert, bis die Wasserstoff-Aufnahme zum Stillstand kommt (ca. 3 Stunden). Im Dünnschichtchromatogramm (Kieselgel 60, Chloroform-Methanol-konz. Ammoniak 90:10:0,5) erkennt man die vollständige Umsetzung der 3'-Nitro-Verbindungen ($R_f$ = 0,73) zu den entsprechenden 3'-Amino-Verbindungen ($R_f$ = 0,61). Nach dem Abfiltrieren des Katalysators wird das Reaktionsgemisch im Rotationsverdampfer, zuletzt bei 80°C und vollem Wasserstrahlvakuum, eingedampft. Der Rückstand (3,1 g) wird analog Beispiel 2 der Säulenchromatographie an 600 g Kieselgel unterworfen. Die Trennung der diastereomeren 3'-Amino-Verbindungen wird dünnschichtchromatographisch verfolgt (Kieselgel 60, Chloroform-Methanol-konz. Ammoniak 95:5:0,1, 2 x entwickeln). Man erhält die Titelverbindung mit dem kleineren $R_f$-Wert von 0,63 in einer Ausbeute von 0,95 g als Eindampfungsrückstand der betreffenden Eluate mit einem spezifischen Drehwert von $[\alpha]_D^{25}$ = -39,8° (c = 1, $CH_3OH$), der bislang allen Kirstallisationsversuchen widerstand.

4.3.25 Beispiel 97
(+)-(1S,5R,2"R)-3'-Amino-2-(2-methoxypropyl)-5,9,9-trimethyl-6,7-benzomorphan

Bei der oben beschriebenen Diastereomerentrennung durch Säulenchromatographie (Beispiel 96) erhält man als Substanz mit dem größeren $R_f$-Wert von 0,69 die Titelverbindung als Eindampfungsrückstand der betreffenden Eluate (1,0 g, spezifischer Drehwert: $[\alpha]_D^{25}$ = +104° (c = 1, $CH_3OH$)), der bislang allen Kristallisationsversuchen widerstand.

4.3.26 Beispiel 98
(+)-(1S,5R,2"S)-3'-Amino-2-(2-methoxypropyl)-5,9,9-trimethyl-6,7-benzomorphan

a) 3'-Nitro-Vorstufe (Diastereomerengemisch)

Ausgehend von 5,2 g (0,02 Mol) (±)-3'-Nitro-5,9,9-trimethyl-6,7-benzomorphan und 5,36 g (0,022 Mol) (S)-2-Methoxypropyl-p-toluol-sulfonat erhält man in Analogie zu Beispiel 67 ein Gemisch der beiden zu erwartenden diastereomeren Basen, welches durch Filtration über Aluminiumoxid gereinigt wird, woraus 4,7 g gereinigtes Basengemisch resultieren.

b) 3'-Amino-Verbindungen (Diastereomerengemisch)

Die 3'-Nitro-Vorstufe (4,7 g gereinigtes Diastereomerengemisch) wird analog Beispiel 96 zu den entsprechenden 3'-Amino-Verbindungen hydriert (3,7 g Diastereomerengemisch).

c) Säulenchromatographische Abtrennung der Titelverbindung

Das Gemisch der diastereomeren 3'-Amino-Verbindungen (3,7 g) wird analog Beispiel 96 mittels Säulenchromatographie an Kieselgel getrennt. Man erhält die Titelverbindung mit dem kleineren $R_f$-Wert von 0,63 in einer Ausbeute von 1,0 g als Eindampfungsrückstand der betreffenden Eluate mit einem spezifischen Drehwert von $[\alpha]_D^{25}$ = +40,5° (c = 1, $CH_3OH$), der bislang allen Kristallisationsversuchen widerstand.

4.3.27 Beispiel 99
(-)-(1R,5S,2"S)-3'-Amino-2-(2-methoxypropyl)-5,9,9-trimethyl-6,7-benzomorphan

Bei der oben beschriebenen Säulenchromatographie (Beispiel 98) erhält man als zweite Substanz mit dem größeren $R_f$-Wert von 0,69 die Titelverbindung als Eindampfungsrückstand der betreffenden Eluate 1,1 g, spezifischer Drehwert: $[\alpha]_D^{25}$ = -105° (c = 1, $CH_3OH$), der bislang nicht kristallisierte.

4.3.28 Beispiel 100
(-)-(1R,5S,2"R)-3'-Acetamido-2-(2-methoxy-propyl)-5,9,9-trimethyl-6,7-benzomorphan

0,95 g (3,14 mMol) (-)-(1R,5S,2"R)-3'-Amino-2-(2-methoxypropyl)-5,9,9-trimethyl-6,7-benzomorphan (Beispiel 96) werden analog Beispiel 85 acetyliert. Das Acetylierungsprodukt wird durch Filtration über Aluminiumoxid analog Beispiel 65 gereinigt. Im Dünnschichtchromatogramm (Kieselgel 60, Chloroform-Methanol-konz. Ammoniak 90:10:0,5) hat die Ausgangsverbindung einen $R_f$-Wert von 0,64 und das Acetylierungsprodukt einen $R_f$-Wert von 0,57. Der Eindampfungsrückstand (0,69 g), der sich bislang allen Kristallisationsversuchen widersetzte, weist einen spezifischen Drehwert von $[\alpha]_D^{25}$ = -53,6° (c = 1, $CH_3OH$) auf.

4.3.29 Beispiel 101
(+)-(1S,5R,2"R)-3'Acetamido-2-(2-methoxypropyl)-5,9,9-trimethyl-6,7-benzomorphan Oxalat

Ausgehend von 1,0 g (3,31 mMol) (+)-(1S,5R,2"R)-3'-Amino-2-(2-methoxypropyl)-5,9,9-trimethyl-6,7-benzomorphan (Beispiel 97) erhält man analog Beispiel 100 die Titelverbindung. Der Eindampfungsrückstand der über Aluminiumoxid gereinigten Substanz (0,5 g) wird analog Beispiel 83 in Form des Oxalates kristallisiert. Man erhält die Titelverbindung in einer Ausbeute von 0,4 g (27,8 % d. Th.) mit einem Schmelzpunkt von 148°C (Zers.) und einem spezifischen Drehwert von $[\alpha]_D^{25}$ = +39,3° (c = 1, $CH_3OH$).

4.3.30 Beispiel 102
(+)-(1S,5R,2"S)-3'-Acetamido-2-(2-methoxypropyl)-5,9,9-trimethyl-6,7-benzomorphan

Ausgehend von 1,0 g (3,31 mMol) (+)-(1S,5R,2"S)-3'-Amino-2-(2-methoxypropyl)-5,9,9-trimethyl-6,7-benzomorphan (Beispiel 98) erhält man analog Beispiel 100 die Titelverbindung als nicht kristallisierenden Eindampfungsrückstand (0,6 g, 41,7 % d. Th.) mit einem $R_f$-Wert von 0,57 und einem Drehwert von $[\alpha]_D^{25}$ = +52,8° (c = 1, $CH_3OH$).

4.3.31 Beispiel 103
(-)-(1R,5S,2"S)-3'Acetamido-2-(2-methoxypropyl)-5,9,9-trimethyl-6,7-benzomorphan Oxalat

Ausgehend von 0,95 g (3,14 mMol) (-)-(1R,5S,2"S)-3'-Amino-2-(2-methoxypropyl)-5,9,9-trimethyl-6,7-benzomorphan (Beispiel 99) erhält man analog Beispiel 101 die Titelverbindung in einer Ausbeute von 0,7 g (51,1 %) mit einem Schmelzpunkt von 148°C (Zers.) und einem spezifischen Drehwert von $[\alpha]_D^{25}$ = -39,2° (c = 1, $CH_3OH$).

4.3.32 Beispiel 104
(-)-(1R,5S,2"R)-3'-Methoxy-2-(2-methoxypropyl)-5,9,9,2'-tetramethyl-6,7-benzomorphan Hydrochlorid

Ausgehend von 0,71 g (2 mMol) (-)-(1R,5S,2"R)-3'-Hydroxy-2-(2-methoxypropyl)-5,9,9,2'-tetramethyl-6,7-benzomorphan Hydrochlorid (Beispiel 53) erhält man analog Beispiel 73 die Titelverbindung in einer Ausbeute von 0,38 g (52,0 %) mit einem Schmelzpunkt von 221-224°C und einem spezifischen Drehwert von $[\alpha]_D^{25}$ = -82,6° (c = 1, $CH_3OH$).

4.3.33 Beispiel 105
(+)-(1S,5R,2"R)-3'-Methoxy-2-(2-methoxypropyl)-5,9,9,2'-tetramethyl-6,7-benzomorphan Hydrochlorid

Ausgehend von 0,71 g (2 mMol) (+)-(1S,5R,2"R)-3'-Hydroxy-2-(2-methoxypropyl)-5,9,9,2'-tetramethyl-6,7-benzomorphan Hydrochlorid (Beipiel 54) erhält man analog Beispiel 73 die Titelverbindung in einer Ausbeute von 0,44 g (59,8 %) als amorphes Pulver und einem spezifischen Drehwert von $[\alpha]_D^{25}$ = +42,2° (c = 1, $CH_3OH$).

4.3.34 Beispiel 106
(+)-(1S,5R,2"S)-3'-Methoxy-2-(2-methoxypropyl)-5,9,9,2'-tetramethyl-6,7-benzomorphan Hydrochlorid

Ausgehend von 0,71 g (2 mMol) (+)-(1S,5R,2"S)-3'-Hydroxy-2-(2-methoxypropyl)-5,9,9,2'-tetramethyl-6,7-benzomorphan Hydrochlorid (Beispiel 55) erhält man analog Beispiel 73 die Titelverbindung in einer Ausbeute von 0,40 g (54,7 %) mit einem Schmelzpunkt von 221-224°C und einem spezifischen Drehwert von $[\alpha]_D^{25}$ = +81,7° (c = 1, $CH_3OH$).

4.3.35 Beispiel 107
(-)-(1R,5S,2"S)-3'-Methoxy-2-(2-methoxypropyl)-5,9,9,2'-tetramethyl-6,7-benzomorphan Hydrochlorid

Ausgehend von 0,71 g (2 mMol) (-)-(1R,5S,2"S)-3'-Hydroxy-2-(2-methoxypropyl)-5,9,9,2'-tetramethyl-6,7-benzomorphan Hydrochlorid (Beispiel 56) erhält man analog Beispiel 73 die Titelverbindung in einer Ausbeute von 0,42 g (56,1 %) als amorphes Pulver und einem spezifischen Drehwert von $[\alpha]$ = -41,0° (c = 1, $CH_3OH$).

4.3.36 Beispiel 108
(-)-(1R,5S,2"R)-2'-Methoxy-2-(2-methoxypropyl)-5,9,9,3'-tetramethyl-6,7-benzomorphan Hydrochlorid

Ausgehend von 0,71 g (2 mMol) (-)-(1R,5S,2"R)-2'-Hydroxy-2-(2-methoxypropyl)-5,9,9,3'-tetramethyl-6,7-benzomorphan Hydrochlorid (Beispiel 57) erhält man analog Beispiel 73 die Titelverbindung in einer Ausbeute von 0,36 g (49,3 %) mit einem Schmelzpunkt von 224-226°C und einem spezifischen Drehwert von $[\alpha]_D^{25}$ = -112,2° (c = 1, $CH_3OH$).

4.3.37 Beispiel 109
(+)-(1S,5R,2"R)-2'-Methoxy-2-(2-methoxypropyl)-5,9,9,3'-tetramethyl-6,7-benzomorphan Hydrochlorid

Ausgehend von 0,71 g (2 mMol) (+)-(1S,5R,2"R)-2'-Hydroxy-2-(2-methoxypropyl)-5,9,9,3'-tetramethyl-6,7-benzomorphan Hydrochlorid (Beispiel 58) erhält man analog Beispiel 73 die Titelverbindung in einer Ausbeute von 0,39 g (53,0 %) als amorphes Pulver und einem spezifischen Drehwert von $[\alpha]_D^{25}$ = +72,4° (c =1, $CH_3OH$).

4.3.38 Beispiel 110
(+)-(1S,5R,2"S)-2'-Methoxy-2-(2-methoxypropyl)-5,9,9,3'-tetramethyl-6,7-benzomorphan Hydrochlorid

Ausgehend von 0,64 g (2 mMol) (+)-(1S,5R,2"S)-2'-Hydroxy-2-(2-methoxypropyl)-5,9,9,3'-tetramethyl-6,7-benzomorphan (Beispiel 59) erhält man analog Beispiel 73 die Titelverbindung in einer Ausbeute von 0,45 g (61,5 %) mit einem Schmelzpunkt von 228-229°C und einem spezifischen Drehwert von $[\alpha]_D^{25}$ = +111,5° (c = 1, $CH_3OH$).

4.3.39 Beispiel 111
(-)-(1R,5S,2"S)-2'-Methoxy-2-(2-methoxypropyl)-5,9,9,3'-tetramethyl-6,7-benzomorphan Hydrochlorid

Ausgehend von 0,71 g (2 mMol) (-)-(1R,5S,2"S)-2'-Hydroxy-2-(2-methoxypropyl)-5,9,9,3'-tetramethyl-6,7-benzomorphan Hydrochlorid (Beispiel 60) erhält man analog Beispiel 73 die Titelverbindung in einer Ausbeute von 0,40 g (53,4 %) als amorphes Pulver und einem spezifischen Drehwert von $[\alpha]_D^{25}$ = -71,8° (c = 1, $CH_3OH$).

4.3.40 Beispiel 112
(-)-(1R,5S,2"R)-3'-Hydroxy-2'-methoxy-2-(2-methoxypropyl)-5,9,9-trimethyl-6,7-benzomorphan Hydrobromid

Ausgehend von 3,56 g (10 mMol) (-)-(1R,5S,2"R)-2',3'-Dihydroxy-2-(2-methoxypropyl)-5,9,9-trimethyl-6,7-benzomorphan Hydrochlorid (Beispiel 61) erhält man analog Beispiel 73, jedoch mit zwei Equivalenten Natriumhydrid ein Gemisch der beiden isomeren Monomethoxy-Derivate und des Dimethoxy-Derivates, die durch Säulenchromatographie an der 100-fachen Menge Kieselgel analog Beispiel 2 getrennt werden [Laufmittel: Dichlormethan-Isopropanol konz. Ammoniak 97:3:03]. Die als mittlere Substanz laufende Verbindung ($R_f$ = 0,73) wird als Hydrobromid kristallisiert. Man erhält die Titelverbindung in einer Ausbeute von 0,62 g (15,0 %) mit einem Schmelzpunkt von 212°C (Zers.) und einem spezifischen Drehwert von $[\alpha]_D^{25}$ = -78,4° (c = 0,5; $CH_3OH$).

4.3.41 Beispiel 113
(-)-(1R,5S,2"R)-2'-Hydroxy-3'-methoxy-2-(2-methoxypropyl)-5,9,9-trimethyl-6,7-benzomorphan Hydrobromid

Die in Beispiel 112 abgetrennte, langsamer laufende Substanz ($R_f$ = 0,68) wird als Hydrobromid gefällt. Man erhält die Titelverbindung in einer Ausbeute von 0,88 g (21,2 %) mit einem Schmelzpunkt von 235°C (Zers.) und mit einem spezifischen Drehwert von $[\alpha]_D^{25}$ = -93,1° (c = 0,5; $CH_3OH$).

4.3.42 Beispiel 114
(-)-(1R,5S,2"R)-2',3'-Dimethoxy-2-(2-methoxypropyl)-5,9,9-trimethyl-6,7-benzomorphan Hydrochlorid

Die in Beispiel 112 abgetrennte, schneller laufende Substanz ($R_f$ = 0,86) wird als Hydrochlorid gefällt. Man erhält die Titelverbindung in einer Ausbeute von 0,87 g (22,7 %) mit einem Schmelzpunkt von 193°C (Zers.) und mit einem spezifischen Drehwert von $[\alpha]_D^{25}$ = -93,2° (c = 0,5; $CH_3OH$).

4.4 Verbindungen (1) nach speziellen Verfahren

4.4.1 Beispiel 115
(-)-(1R,5S,2"R)-2'-Hydroxy-2-(2-hydroxypropyl)-5,9,9-trimethyl-6,7-benzomorphan Hydrochlorid

1,5 g (3,60 mMol) (-)-(1R,5S,2"R)-2-(2-Benzyloxypropyl)-2'-hydroxy-5,9,9-trimethyl-6,7-benzomorphan Hydrochlorid (Beispiel 43) werden in 75 ml Methanol gelöst und in Gegenwart von Palladiumhydroxid auf Aktivkohle (0,6 g feuchter "Pearlman-Katalysator") bei einem Wasserstoffdruck von 5 bar und einer Temperatur von 70°C bis zur vollständigen Umsetzung hydriert (ca. 6,2 Stunden). Danach wird der Katalysator abfiltriert und das Filtrat im Rotationsverdampfer, zuletzt bei 80°C und vollem Wasserstrahlvakuum, eingedampft. Der Rückstand wird in 10 ml Methanol gelöst und die Lösung mit 30 ml Diethylether versetzt. Das sich ausscheidende Kristallisat wird nach Stehen über einen Zeitraum von ca. 12 h bei Raumtemperatur abgesaugt, zunächst mit einer Ethanol-Ether-Mischung (1:1), dann mit Ether gewaschen und bei 80°C getrocknet. Man erhält die Titelverbindung in einer Ausbeute von 1,0 g (85,5 % d. Th.) mit einem Schmelzpunkt von 276°C (Zers.) und einem spezifischen Drehwert von $[\alpha]_D^{25}$ = -127,4° (c = 1, $CH_3OH$).

4.4.2 Beispiel 116
(+)-(1S,5R,2"S)-2'-Hydroxy-2-(2-hydroxypropyl)-5,9,9-trimethyl-6,7-benzomorphan Hydrochlorid

Ausgehend von 1,5 g (3,6 mMol) (+)-(1S,5R,2"S)-2-(2-Benzyloxypropyl)-2'-hydroxy-5,9,9-trimethyl-6,7-benzomorphan Hydrochlorid (Beispiel 44) erhält man analog Beispiel 115 die Titelverbindung in einer Ausbeute von 1,0 g (85,5 % d. Th.) mit einem Schmelzpunkt von 276°C (Zers.) und einem spezifischen Drehwert von $[\alpha]_D^{25}$ = +126,8° (c = 1, $CH_3OH$).

4.4.3 Beispiel 117
(-)-(1R,5S,2"S)-2'-Hydroxy-2-(2-hydroxypropyl)-5,9,9-trimethyl-6,7-benzomorphan Hydrochlorid

Das nach Beispiel 44 in der Mutterlauge verbleibende Diasteremere (3,17 g Eindampfungsrückstand) wird analog Beispiel 115 hydriert. Man erhält die Titelverbindung in einer Ausbeute von 1,5 g (56,2 % d. Th.) mit einem Schmelzpunkt von 247°C (Zers.) und einem spezifischen Drehwert von $[\alpha]_D^{25}$ = -86,8° (c = 1, $CH_3OH$).

4.4.4 Beispiel 118
(+)-(1S,5R,2"R)-2'-Hydroxy-2-(2-hydroxypropyl)-5,9,9-trimethy1-6,7-benzomorphan Hydrochlorid

Das nach Beispiel 43 in der Mutterlauge verbleibende Diastereomere (3,17 g Eindampfungsrückstand) wird analog Beispiel 115 hydriert. Man erhält die Titelverbindung in einer Ausbeute von 1,5 g (56,2 % d. Th.) mit einem Schmelzpunkt von 247°C (Zers.) und einem spezifischen Drehwert von $[\alpha]_D^{25}$ = +86,4° (c = 1, $CH_3OH$).

4.4.5 Beispiel 119
(+)-(1R/S,5S/R,2"S)-2'-Fluor-2-(2-methoxypropyl)-5,9,9-trimethyl-6,7-benzomorphan Oxalat

a) Ausgangsverbindung: (1R/S,5S/R,2"S)-2'-Fluor-2-(2-methoxypropionyl)-5,9,9-trimethyl-6,7-benzomorphan (Diastereomerengemisch)

5,94 g (0,02 Mol) (±)-2'-Nitro-5,9,9-trimethyl-6,7-benzomorphan Hydrochlorid werden analog Beispiel 32 mit 2,68 g (0,22 Mol) (S)-2-Methoxypropionsäurechlorid acyliert. Das so erhaltene Acylierungsprodukt (7,0 g) wird - wie dort beschrieben - zum entsprechenden 2'-Amino-Analogon hydriert. Letzeres wird - mit aus dem Stand der Technik bekannten Verfahren [T.L. Fletcher und M.J. Namkung, Chem. and Ind. 1961, 179] - zum entsprechenden 2'-Fluor Derivat umgesetzt (5,7 g). Dabei resultiert ein Gemisch der zu erwartenden Diastereomeren, die sich im Dünnschichtchromatogramm nicht auftrennen lassen ($R_f$ = 0,73, Kieselgel, Chloroform-Methanol-konz. Ammoniak 90:10:0,5).

b) Umsetzung zur Titelverbindung

1,58 g (5 mMol) der Vorstufe werden analog Beispiel 32 mit Lithiumaluminiumhydrid ($LiAlH_4$) zur Titelverbindung reduziert und - wie dort beschrieben - durch Säulenchromatographie an Kieselgel gereinigt. Der Eindampfungsrückstand der Eluate mit der gereinigten Substanz (0,4 g, $R_f$ = 0,78) wird analog Beispiel 83 als Oxalat kristallisiert. Man erhält die Titelverbindung in einer Ausbeute von 0,35 g (17,7 % d. Th.) mit einem Schmelzpunkt von 165°C (Zers.) und mit einem Drehwert von $[\alpha]_D^{25}$ = +9,7° (c = 1, $CH_3OH$). Die Substanz besteht aus einem Gemisch der zu erwartenden Diastereomeren ((1R,5S,2"S)- und (1S,5R2"S)-Verbindung) in einem Verhältnis von ca. 1:1 ($^1$H-NMR-spektroskopisch ermittelt).

4.4.6 Beispiel 120
(-)-(1R/S,5S/R,2"R)-2'-Fluor-2-(2-methoxypropyl)-5,9,9-trimethyl-6,7-benzomorphan Oxalat

a) Ausgangsverbindung: (1R/S,5S/R,2"R)-2'-Fluor-2-(2-methoxypropionyl)-5,9,9-trimethyl-6,7-benzomorphan (Diastereomerengemisch)

Ausgehend von 2,97 g (0,01 Mol) (±)-2'-Nitro-5,9,9-trimethyl-6,7-benzomorphan Hydrochlorid erhält man unter Verwendung von 1,34 g (0,011 Mol) (R)-2-Methoxypropionsäurechlorid ein Gemisch der zu erwartenden diastereomeren 2'-Fluor-2-(2-methoxypropionyl)-5,9,9-trimethyl-6,7-benzomorphane (Reaktionsfolge analog Beispiel 119), die sich dünnschichtchromatographisch nicht trennen lassen ($R_f$ = 0,73, Kieselgel, Chloroform-Methanol-konz. Ammoniak 90:10:0,5), Ausbeute: 2,9 g.

b) Umsetzung zur Titelverbindung

2,9 g (9,2 mMol) der obigen Vorstufe werden analog Beispiel 119 mit Lithiumaluminiumhydrid zur Titelverbindung reduziert. Das Reaktionsprodukt wird wie dort beschrieben gereinigt. Der Eindampfungsrückstand der reinen Fraktionen (0,9 g) wird analog Beispiel 98 als Oxalat kristallisiert. Man erhält die Titelverbindung in einer Ausbeute von 0,88 g (22,3 % d. Th.) mit einem Schmelzpunkt von 169°C (Zers.) und einem spezifischen Drehwert von $[\alpha]_D^{25}$ = -9,5° (c = 1, $CH_3OH$) als Gemisch der zu erwartenden Diastereomeren ((1R,5S,2"R)- und (1S,5R,2"R)-Verbindung).

**Patentansprüche**

1. Benzomorphane der allgemeinen Formel I

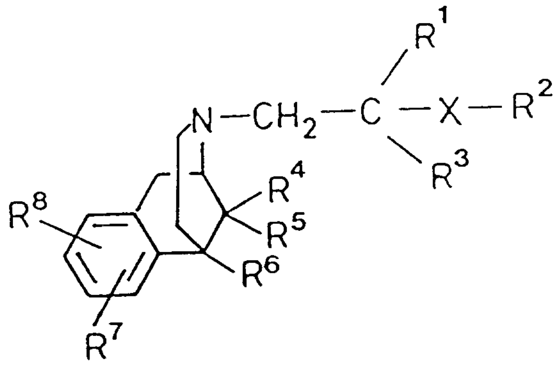

(I)

worin

X Sauerstoff oder Schwefel;

$R^1$ $C_1$-$C_8$-Alkyl, $C_3$-$C_6$-Alkenyl, $C_3$-$C_6$-Alkinyl, einen aromatischen Rest mit 6 bis 10 Kohlenstoffatomen - auch in Zusammensetzungen - wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;

$R^2$ Wasserstoff, $C_1$-$C_8$-Alkyl, $C_3$-$C_6$-Alkenyl, $C_3$-$C_6$-Alkinyl, einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann; einen über eine Alkylenkette gebundenen Arylrest mit 7 bis 14 Kohlenstoffatomen, wobei der Aromat mit einer oder mehreren Niederalkylgruppe(n), Alkoxygruppe(n), Nitrogruppe(n), Aminogruppe(n) und/oder einein oder mehreren Halogenatome(n) - untereinander gleich oder verschieden - substituiert sein kann;

$R^3$ Wasserstoff, $C_1$-$C_6$-Alkyl;

$R^4$ $C_1$-$C_8$-Alkyl;

$R^5$ $C_1$-$C_8$-Alkyl;

$R^6$ $C_1$-$C_8$-Alkyl, einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;

$R^7$ und $R^8$ unabhängig voneinander
Wasserstoff, $C_1$-$C_8$-Alkyl, Halogen,
-OH, $C_1$-$C_8$-Alkoxy,
einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann,
-CN, $-NO_2$,
$NH_2$, -NH($C_1$-$C_8$-Alkyl),
-N($C_1$-$C_8$-Alkyl)$_2$, wobei die Alkylreste gleich oder verschieden sein können,
-NH-Acyl oder -N-Acyl-($C_1$-$C_8$-Alkyl), worin Acyl für Benzoyl oder für einen Alkylcarbonylrest mit einem

geradkettigen oder verzweigten Niederalkylrest mit 1 bis 6 Kohlenstoffatom(en), wobei der Alkylrest gegebenenfalls mit einem oder mehreren Halogenatom(en), die untereinander gleich oder verschieden sein können, substituiert sein kann,

bedeuten können, deren Stereoisomere sowie deren Säureadditionssalze, mit der Maßgabe, daß - im Falle

X Sauerstoff;
$R^1$ $C_1$-$C_3$-Alkyl, $C_3$-Alkenyl,
$C_3$-Alkinyl;
$R^2$ Wasserstoff, $C_1$-$C_4$-Alkyl, $C_3$- und $C_4$-Alkenyl;
$R^3$ Wasserstoff, $C_1$-$C_3$-Alkyl;
$R^4$ Methyl;
$R^5$ Methyl;
$R^6$ $C_1$-$C_4$-Alkyl oder Phenyl

und einer der beiden Substituenten $R^7$ oder $R^8$ Wasserstoff bedeuten - der verbleibende Substituent $R^7$ oder $R^8$ in der 2'-Position nicht die Bedeutung von Wasserstoff, Hydroxy, $C_1$-$C_3$-Alkoxy, O-Acyl haben darf.

**2.** Benzomorphane gemäß Anspruch 1
wobei

X Sauerstoff, Schwefel;

$R^1$ Methyl, Ethyl, Propyl, Isopropyl, Phenyl;

$R^2$ Methyl, Ethyl, Propyl, Isopropyl, Allyl, Propargyl, Phenyl, Benzyl;

$R^3$ Wasserstoff, $C_1$-$C_4$-Alkyl;

$R^4$ Methyl, Ethyl, Propyl, Isopropyl;

$R^5$ Methyl, Ethyl, Propyl, Isopropyl;

$R^6$ Methyl, Ethyl, Propyl, Isopropyl, Phenyl;

$R^7$ Fluor, Chlor, Hydroxy, Niederalkyl,
$C_1$-$C_3$-Alkoxy,
einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann;

$R^8$ Wasserstoff, Niederalkyl, Hydroxy oder $C_1$-$C_8$-Alkoxy

bedeuten können, deren Stereoisomere sowie deren Säureadditionssalze.

**3.** Benzomorphane gemäß Anspruch 1
wobei

X Sauerstoff,

$R^1$ Methyl, Ethyl;

$R^2$ Methyl; Ethyl;

$R^3$ Wasserstoff;

$R^4$ Methyl, Ethyl;

$R^5$ Methyl, Ethyl;

$R^6$ Methyl, Ethyl;

$R^7$ Hydroxy, Methyl, Methoxy,
einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann;

$R^8$ Wasserstoff, Methyl, Ethyl, Hydroxy oder $C_1$-$C_3$-Alkoxy

bedeuten können, deren Stereoisomere sowie deren Säureadditionssalze.

**4.** Benzomorphane gemäß Anspruch 1
wobei

X Sauerstoff,

$R^1$ Methyl;

$R^2$ Methyl;

$R^3$ Wasserstoff;

$R^4$ Methyl;

$R^5$ Methyl;

$R^6$ Methyl;

$R^7$ Hydroxy, Methyl, Methoxy, Acetoxy;

$R^8$ Wasserstoff, Methyl, Hydroxy oder $C_1$-$C_3$-Alkoxy

bedeuten können, deren Stereoisomere sowie deren Säureadditionssalze.

**5.** Benzomorphane gemäß Anspruch 1
wobei

X Sauerstoff;

$R^1$ Methyl;

$R^2$ Methyl;

$R^3$ Wasserstoff,

$R^4$ Methyl;

$R^5$ Methyl;

$R^6$ Methyl;

$R^7$ Hydroxy, Methyl, Methoxy, Acetoxy;

$R^8$ Wasserstoff, Methyl, Hydroxy, Methoxy oder Ethoxy

bedeuten können und wobei sich der Substituent $R^7$ in 3'-Stellung und der Substituent $R^8$ in 2'-Stellung befindet und das 2"-Kohlenstoffatom eine R-Konfiguration aufweist sowie deren Säureadditionssalze.

6. (-)-(1R,5S,2"R)-3'-Hydroxy-2-(2-methoxypropyl)-5,9,9-trimethyl-6,7-benzomorphan und dessen Säureadditionssalze.

7. Pharmazeutische Zubereitung, gekennzeichnet durch den Gehalt an einer Verbindung nach einem der Ansprüche 1 bis 6 und deren Säureadditionssalze neben üblichen Hilfs- und Trägerstoffen.

8. Verwendung von Verbindungen nach einem der Ansprüche 1 bis 6 als Arneimittel.

9. Verwendung von Verbindungen der allgemeinen Formel I worin

X Sauerstoff oder Schwefel;

$R^1$ $C_1$-$C_8$-Alkyl, $C_3$-$C_6$-Alkenyl, $C_3$-$C_6$-Alkinyl, einen aromatischen Rest mit 6 bis 10 Kohlenstoffatomen - auch in Zusammensetzungen-, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;

$R^2$ Wasserstoff, $C_1$-$C_8$-Alkyl, $C_3$-$C_6$-Alkenyl, $C_3$-$C_6$-Alkinyl, einen aromatischen Rest mit 6 bis 10 Kohlenstoffatomen - auch in Zusammensetzungen-, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann, einen über eine Alkylenkette gebundenen Arylrest mit 7 bis 14 Kohlenstoffatomen, wobei der Aromat mit einer oder mehreren Niederalkylgruppe(n), Alkoxygruppe(n), Nitrogruppe(n), Aminogruppe(n) und/oder einem oder mehreren Halogenatome(n) - untereinander gleich oder verschieden - substituiert sein kann,

$R^3$ Wasserstoff, $C_1$-$C_6$-Alkyl;

$R^4$ $C_1$-$C_8$-Alkyl;

$R^5$ $C_1$-$C_8$-Alkyl;

$R^6$ $C_1$-$C_8$-Alkyl, einen aromatischen Rest mit 6 bis 10 Kohlenstoffatomen - auch in Zusammensetzungen, wobei der Aromat mit einer oder mehreren Niederalkylgruppe(n), Alkoxygruppe(n), Nitrogruppe(n), Aminogruppe(n) und/oder einem oder mehreren Halogenatomen - untereinander gleich oder verschieden - substituiert sein kann;

$R^7$ und $R^8$ unabhängig voneinander Wasserstoff, $C_1$-$C_8$-Alkyl, Halogen, -OH, $C_1$-$C_8$-Alkoxy, einen O-Benzoyl oder O-Alkylcarbonylrest mit einem geradkettigen oder verzweigten Niederalkylrest mit 1 bis 6 Kohlenstoffatomen, wobei der Alkylrest gegebenenfalls mit einem oder mehreren Halogenatom(en), die untereinander gleich oder verschieden sein können, substituiert sein kann, -CN, $-NO_2$, $NH_2$, -NH($C_1$-$C_8$-Alkyl), -N($C_1$-$C_8$-Alkyl)$_2$ wobei die Alkylreste gleich oder verschieden sein können, -NH-Acyl oder -N-Acyl-($C_1$-$C_8$-Alkyl), worin Acyl für Benzoyl oder für einen Alkylcarbonylrest mit einem geradkettigen oder verzweigten Niederalkylrest mit 1 bis 6 Kohlenstoffatom(en), wobei der Alkylrest gegebenenfalls mit einem oder mehreren Halogenatom(en), die untereinander gleich oder verschieden sein können, substituiert sein kann,

bedeuten können, deren Stereoisomere sowie deren Säureadditionssalze zur Herstellung eines Medikamentes zur therapeutischen Behandlung von Gehirnischämien verschiedener Genese, Epilepsie und neurodegenerativen Erkrankungen.

**10.** Verfahren zum Herstellen von Verbindungen der allgemeinen Formel I

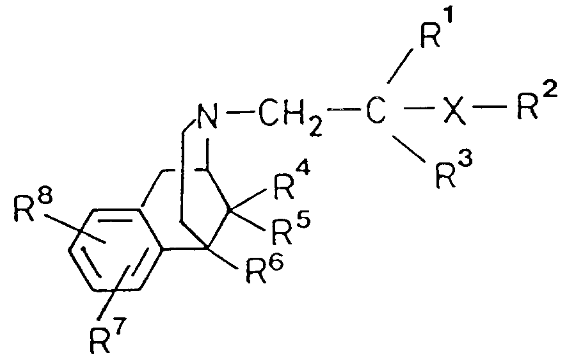

(I)

in der die Substituenten $R^1$ bis $R^8$ die in Anspruch 1 angegebene Bedeutung haben, dadurch gekennzeichnet, daß man entweder

a) die Benzophormane der allgemeinen Formel II - in der $R^4$, $R^5$ und $R^6$ die angegebene Bedeutung haben und $R^9$ und $R^{10}$ entweder die Bedeutung von $R^7$ und $R^8$ haben oder in diese auf an sich bekannte Weise überführbare Substituenten verkörpern - auf an sich bekannte Weise in inerten Lösungsmitteln - gegebenenfalls - in Gegenwart eines säurebindenden Agenzes - mit Carbonsäurederivaten der allgemeinen Formel 4, worin Y eine durch eine sekundäre Aminogruppe substituierbare Austrittsgruppe aus der Gruppe Halogen, Hydroxy, OC(O)Alkyl, O-Acyl, $OSO_2$Alkyl und $OSO_2$Aryl verkörpert und $R^1$, $R^2$ und $R^3$ sowie X

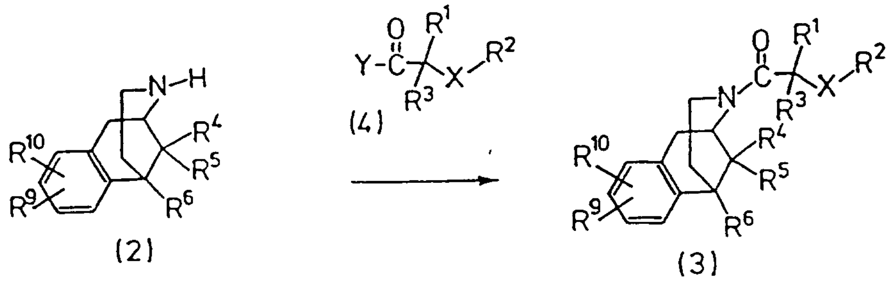

(2) (4) (3)

die angegebene Bedeutung besitzen, umsetzt und das resultierende Säureamid 3 anschließend in einem inerten Lösungsmittel auf an sich bekannte Weise zum Amin der allgemeinen Formel 6 reduziert

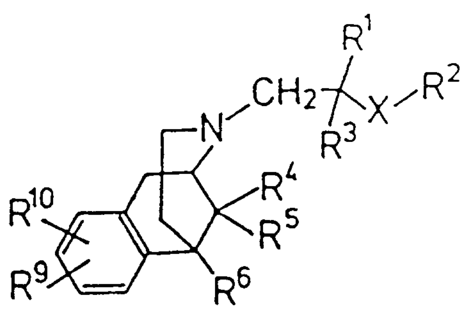

(6)

und gegebenenfalls die Substituenten $R^9$ und $R^{10}$ in $R^7$ und $R^8$ überführt und das Produkt (1) aus dem Reaktionsgemisch isoliert

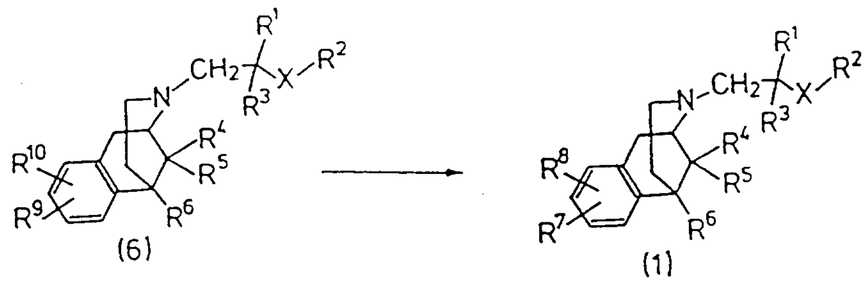

(6) (1)

oder

b) Benzomorphane der allgemeinen Formel 2

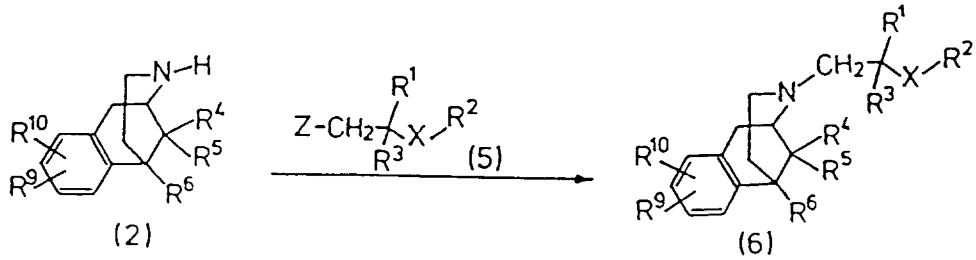

(2) (5) (6)

- in der $R^4$, $R^5$ und $R^6$ die angegebene Bedeutung haben und $R^9$ und $R^{10}$ entweder die Bedeutung von $R^7$ und $R^8$ besitzen oder in diese auf an sich bekannte Weise überführbare Substituenten verkörpern - auf an sich bekannte Weise in inerten Lösungsmitteln - gegebenenfalls in Gegenwart eines säurebindenden Agenzes - mit einem Alkylierungsmittel der allgemeinen Formel 5, worin Z eine durch eine sekundäre Aminogruppe substituierbare Austrittsgruppe aus der Gruppe Halogen - wie z.B. Cl, Br und I - oder Arylsulfonate $OSO_2$Aryl- wie z.B. Tosylat- oder Alkylsulfonate - wie z.B. Methansulfonat oder Halogenmethansulfonat oder eine Sulfatgruppe bedeutet, zum tertiären Amin vom Typ 6 umsetzt

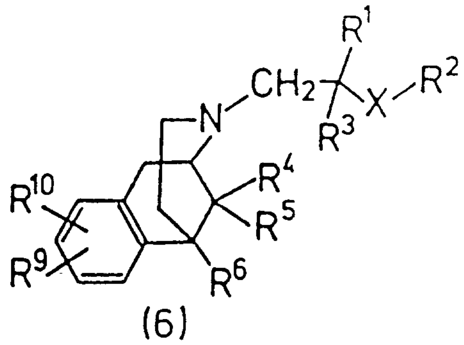

(6)

und gegebenenfalls die Substituenten $R^9$ und $R^{10}$ in $R^7$ und $R^8$ überführt und das Reaktionsprodukt isoliert.

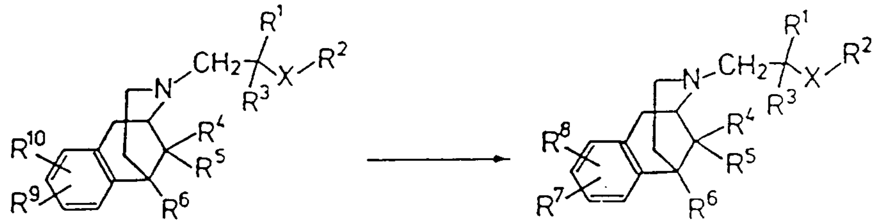

(6) (1)

## Claims

1. Benzomorphans of general formula I

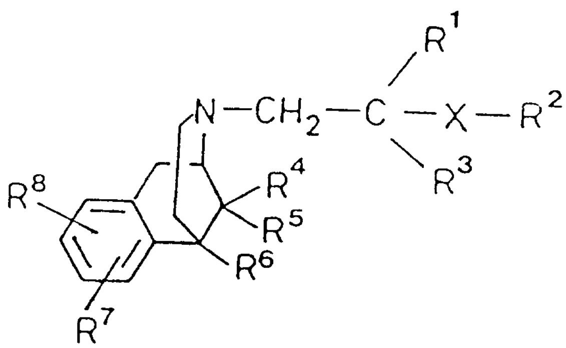

(I)

wherein

X denotes oxygen or sulphur;

$R^1$ denotes $C_{1-8}$-alkyl, $C_{3-6}$-alkenyl, $C_{3-6}$-alkynyl, an aromatic group having 6 to 10 carbon atoms, also in combinations- it being possible for the aromatic moeity to be substituted by one or more lower alkyl group(s), alkoxy group(s), nitro group(s), amino group(s) and/or one or more halogen atom(s) which may be identical to or different from one another;

$R^2$ denotes hydrogen, $C_{1-8}$-alkyl, $C_{3-6}$-alkenyl, $C_{3-6}$-alkynyl, an aromatic group having 6 to 10 carbon atoms, also in combinations, it being possible for the aromatic moeity to be substituted with one or more lower alkyl group(s), alkoxy group(s), nitro group(s), amino group(s), and/or one or more halogen atom(s), which may be identical to or different from one another;
an aryl group bound to an alkylene chain and having 7 to 14 carbon atoms, whilst the aromatic moeity may be substituted with one or more lower alkyl group(s), alkoxy group(s), nitro group(s), amino group(s) and/or one or more halogen atom(s), which may be identical to or different from one another;

$R^3$ denotes hydrogen, $C_{1-6}$-alkyl;

$R^4$ denotes $C_{1-8}$-alkyl;

$R^5$ denotes $C_{1-8}$-alkyl;

$R^6$ denotes $C_{1-8}$-alkyl, an aromatic group having 6 to 10 carbon atoms, also in combinations, it being possible for the aromatic moeity to be substituted with one or more lower alkyl group(s), alkoxy group(s), nitro group(s), amino group(s), and/or one or more halogen atom(s), which may be identical to or different from one another;

$R^7$ and $R^8$ independently of each other represent hydrogen, $C_{1-8}$-alkyl, halogen, -OH, $C_{1-8}$-alkoxy, an O-benzoyl or O-alkyl carbonyl group with one straight-chained or branched lower alkyl group having 1 to 6 carbon atoms, wherein the alkyl group may optionally be substituted with one or more halogen atom(s) which may be identical to or different from one another, -CN, $-NO_2$, $NH_2$, -NH($C_{1-8}$-alkyl), -N($C_{1-8}$-alkyl)$_2$, wherein the alkyl groups may be identical or different, -NH-acyl or -N-acyl-($C_{1-8}$-alkyl), wherein acyl represents benzoyl or an alkyl carbonyl group with a straight-chained or branched lower alkyl group having 1 to 6 carbon atoms, whilst the alkyl group may optionally be substituted with one or more halogen atom(s) which may be identical to or different from one another,

the stereoisomeres thereof as well as the acid addition salts thereof, with the proviso that if

X denotes oxygen;
$R^1$ denotes $C_{1-3}$-alkyl, $C_3$-alkenyl, $C_3$-alkynyl;
$R^2$ denotes hydrogen, $C_{1-4}$-alkyl, $C_3$- and $C_4$-alkenyl;
$R^3$ denotes hydrogen, $C_{1-3}$-alkyl;
$R^4$ denotes methyl;
$R^5$ denotes methyl;
$R^6$ denotes $C_{1-4}$-alkyl or phenyl

and one of the two substituents $R^7$ or $R^8$ denotes hydrogen, the remaining substituent $R^7$ or $R^8$ in the 2'-position must not denote hydrogen, hydroxy, $C_{1-3}$-alkoxy O-acyl.

**2.** Benzomorphans of general formula I wherein

X denotes oxygen or sulphur;

$R^1$ denotes methyl, ethyl, propyl, isopropyl, phenyl;

$R^2$ denotes methyl, ethyl, propyl, isopropyl, allyl, propargyl, phenyl, benzyl;

$R^3$ denotes hydrogen, $C_{1-4}$-alkyl;

$R^4$ denotes methyl, ethyl, propyl, isopropyl;

$R^5$ denotes methyl, ethyl, propyl, isopropyl;

$R^6$ denotes methyl, ethyl, propyl, isopropyl, phenyl;

$R^7$ denotes fluorine, chlorine, hydroxy, lower alkyl, $C_{1-3}$-alkoxy, an O-benzoyl or O-alkyl carbonyl group with one straight-chained or branched lower alkyl group having 1 to 6 carbon atoms, wherein the alkyl group may optionally be substituted with one or more halogen atom(s) which may be identical to or different from one another;

$R^8$ denotes hydrogen, lower alkyl, hydroxy or $C_{1-8}$-alkoxy

the stereoisomers and acid addition salts thereof.

**3.** Benzomorphans according to claim 1
wherein

X denotes oxygen;

$R^1$ denotes methyl, ethyl;

$R^2$ denotes methyl, ethyl;

$R^3$ denotes hydrogen;

$R^4$ denotes methyl, ethyl;

$R^5$ denotes methyl, ethyl;

$R^6$ denotes methyl, ethyl;

$R^7$ denotes hydroxy, methyl, methoxy, an O-benzoyl or O-alkyl carbonyl group with one straight-chained or branched lower alkyl group having 1 to 6 carbon atoms, wherein the alkyl group may optionally be substituted with one or more halogen atom(s) which may be identical to or different from one another;

$R^8$ denotes hydrogen, methyl, ethyl, hydroxy or $C_{1-3}$-alkoxy

the stereoisomers and acid addition salts thereof.

**4.** Benzomorphans according to claim 1 wherein

X denotes oxygen;

$R^1$ denotes methyl;

$R^2$ denotes methyl;

$R^3$ denotes hydrogen;

$R^4$ denotes methyl;

$R^5$ denotes methyl;

$R^6$ denotes methyl;

$R^7$ denotes hydroxy, methyl, methoxy, acetoxy;

$R_8$ denotes hydrogen, methyl, hydroxy or $C_{1-3}$-alkoxy

the stereoisomers and acid addition salts thereof.

**5.** Benzomorphans according to claim 1 wherein

X denotes oxygen;

$R^1$ denotes methyl;

$R^2$ denotes methyl;

$R^3$ denotes hydrogen;

$R^4$ denotes methyl;

$R^5$ denotes methyl;

$R^6$ denotes methyl;

$R^7$ denotes hydroxy, methyl, methoxy, acetoxy;

$R^8$ denotes hydrogen, methyl, hydroxy, methoxy or ethoxy,

whilst $R^7$ is in the 3'-position and $R^8$ is in the 2'-position and the 2"-carbon atom has the R-configuration, as well as the acid addition salts thereof.

6. (-)-(1R,5S,2"R)-3'-hydroxy-2-(2-methoxypropyl)-5,9,9-trimethyl-6,7-benzomorphan and the acid addition salts thereof.

7. Pharmaceutical preparations, characterised in that they contain a compound according to one of claims 1 to 6 or an acid addition salt thereof together with conventional excipients and carriers.

8. Use of compounds according to one of claims 1 to 6 in pharmaceutical compositions.

9. Use of compounds of general formula I wherein

| | |
|---|---|
| X | denotes oxygen or sulphur; |
| $R^1$ | denotes $C_{1-8}$-alkyl, $C_{3-6}$-alkenyl, $C_{3-6}$-alkynyl, an aromatic group having 6 to 10 carbon atoms, also in combinations, it being possible for the aromatic moeity to be substituted with one or more lower alkyl group(s), alkoxy group(s), nitro group(s), amino group(s), and/or one or more halogen atom(s), which may be identical to or different from one another; |
| $R^2$ | denotes hydrogen, $C_{1-8}$-alkyl, $C_{3-6}$-alkenyl, $C_{3-6}$-alkynyl, an aromatic group having 6 to 10 carbon atoms, also in combinations, it being possible for the aromatic moeity to be substituted with one or more lower alkyl group(s), alkoxy group(s), nitro group(s), amino group(s), and/or one or more halogen atom(s), which may be identical to or different from one another; an aryl group bound to an alkylene chain and having 7 to 14 carbon atoms, wherein the aromatic moeity may be substituted with one or more lower alkyl group(s), alkoxy group(s), nitro group(s), amino group(s), and/or one or more halogen atom(s), which may be identical to or different from one another. |
| $R^3$ | denotes hydrogen, $C_{1-6}$-alkyl; |
| $R^4$ | denotes $C_{1-8}$-alkyl; |
| $R^5$ | denotes $C_{1-8}$-alkyl; |
| $R^6$ | denotes $C_{1-8}$-alkyl, an aromatic group having 6 to 10 carbon atoms, also in combinations, it being possible for the aromatic moeity to be substituted with one or more lower alkyl group(s), alkoxy group(s), nitro group(s), amino group(s), and/or one or more halogen atom(s), which may be identical to or different from one another; |
| $R^7$ and $R^8$ | independently of each other represent hydrogen, $C_{1-8}$-alkyl, halogen, -OH, $C_{1-8}$-alkoxy, an O-benzoyl or O-alkylcarbonyl group with one straight-chained or branched lower alkyl group having 1 to 6 carbon atoms, wherein the alkyl group may optionally be substituted with one halogen atom(s) which may be identical to or different from one another, -CN, $-NO_2$, $NH_2$, $-NH(C_{1-8}$-alkyl), $-N(C_{1-8}$-alkyl$)_2$ whilst the alkyl groups may be identical to or different, -NH-acyl or -N-acyl-($C_1$-$C_8$-alkyl), wherein acyl is benzoyl or an alkyl carbonyl group with a straight-chained or branched lower alkyl group having 1 to 6 carbon atoms, whilst the alkyl group may optionally be substituted with one or more halogen atom(s) which may be identical to or different from one another, |

the stereoisomers and the acid addition salts thereof for preparing a medicament for the therapeutic treatment of cerebral ischaemia of various origins, epilepsy and neurodegenerative diseases.

**10.** Process for preparing compounds of general formula I

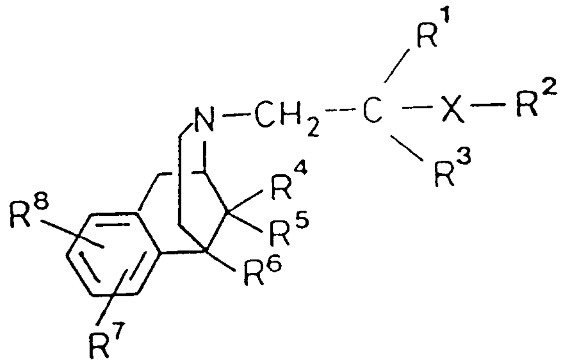

(I)

wherein the substituents $R^1$ to $R^8$ have the meanings given in claim 1, characterised in that either

a) the benzomorphans of general formula II (wherein $R^4$, $R^5$ and $R^6$ are as hereinbefore defined and $R^9$ and $R^{10}$ either have the same meaning as $R^7$ and $R^8$ or represent substituents which can be converted into the latter in known manner, in inert solvents in known manner, optionally in the presence of an acid binding agent) are reacted with carboxylic acid derivatives of general formula 4 wherein Y represents a leaving group capable of being substituted by a secondary amino group, selected from the group comprising halogen, hydroxy, OC(O)alkyl, O-acyl, $OSO_2$alkyl and $OSO_2$aryl and $R^1$, $R^2$ and $R^3$ and X are as hereinbefore defined,

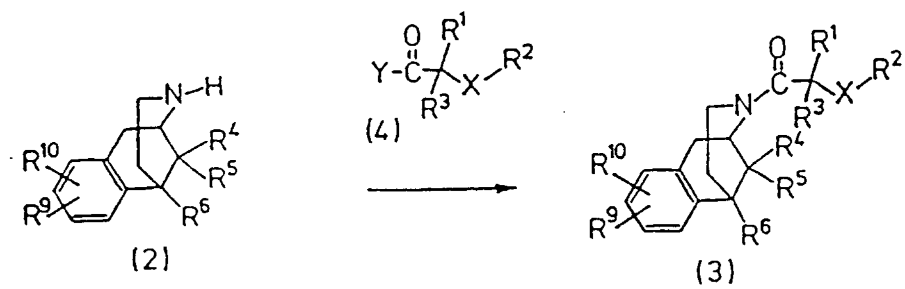

(4)

(2) (3)

and the resulting acid amide 3 is subsequently reduced in an inert solvent, in known manner, to obtain the amine of general formula 6

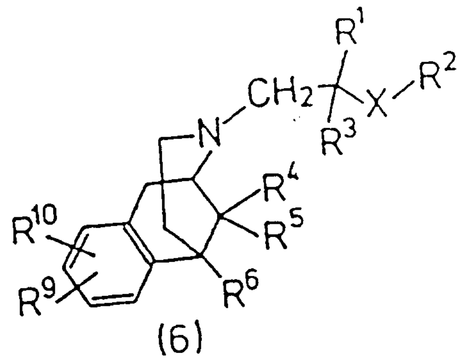

(6)

and optionally the substituents $R^9$ and $R^{10}$ are converted into $R^7$ and $R^8$ and the product (1) is isolated from the reaction mixture

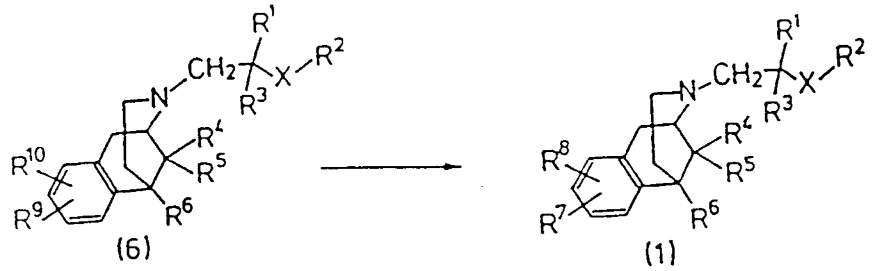

(6) (1)

or

b) benzomorphans of general formula 2

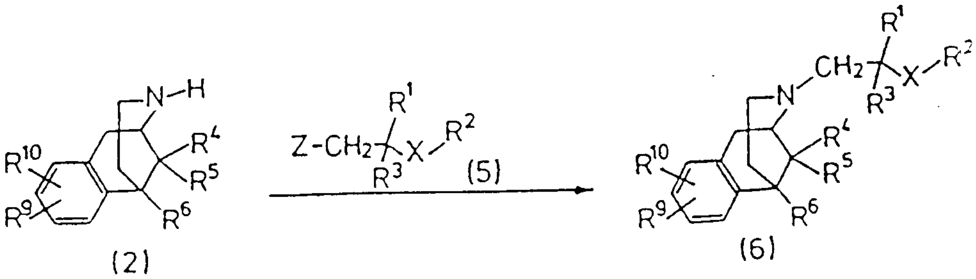

(2) (6)

wherein $R^4$, $R^5$ and $R^6$ are as hereinbefore defined and $R^9$ and $R^{10}$ either have the same meanings as $R^7$ and $R^8$ or represent substituents which can be converted into the latter in known manner, in inert solvents in known manner, optionally in the presence of an acid binding agent, are reacted with an alkylating agent of general formula 5 wherein Z represents a leaving group, which can be substituted by a secondary amino group, selected from the group comprising halogen, such as Cl, Br and I, or arylsulphonates $OSO_2$aryl, such as tosylate or alkylsulphonates, e.g. methanesulphonate or halomethanesulphonate or a sulphate group, to obtain the tertiary amine of type 6

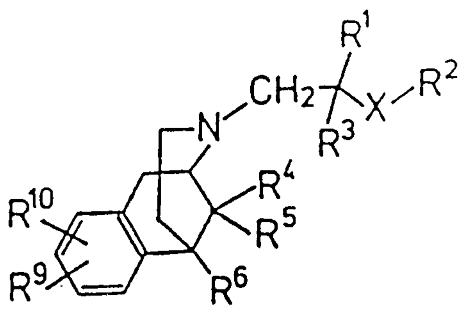

(6)

and optionally the substituents $R^9$ and $R^{10}$ are converted into $R^7$ and $R^8$ and the reaction product is isolated.

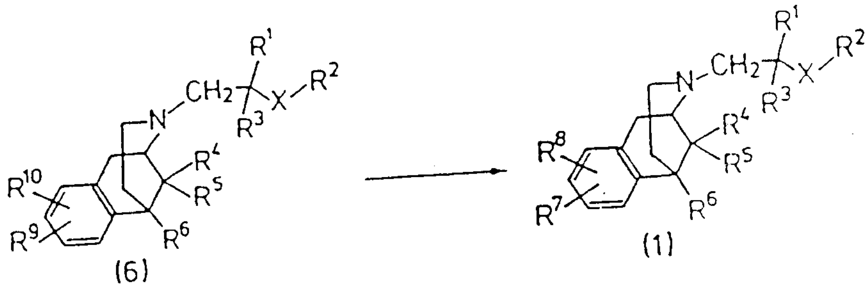

(6) (1)

## Revendications

1. Benzomorphanes de formule générale I

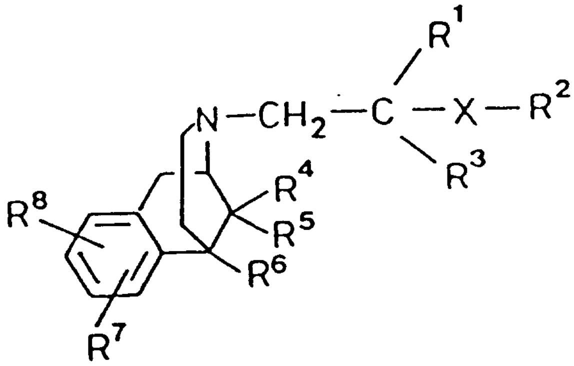

(I)

où

X peut représenter l'oxygène ou le soufre;

$R^1$ peut représenter alkyle en $C_1$-$C_8$, alcényle en $C_3$-$C_6$, alcynyle en $C_3$-$C_6$, un reste aromatique de 6 à 10 atomes de carbone, même dans des assemblages, le groupe aromatique pouvant être substitué par un ou plusieurs groupes alkyle inférieurs, alcoxy, nitro, amino et/ou un ou plusieurs atomes d'halogène, identiques ou différents;

$R^2$ peut représenter l'hydrogène, alkyle en $C_1$-$C_8$, alcényle en $C_3$-$C_6$,
alcynyle en $C_3$-$C_6$, un reste aromatique de 6 à 10 atomes de carbone, même dans des assemblages, le groupe aromatique pouvant être substitué par un ou plusieurs groupes alkyle inférieurs, alcoxy, nitro, amino et/ou un ou plusieurs atomes d'halogène, identiques ou différents,
un reste aryle de 7 à 14 atomes de carbone lié par une chaîne alkylène, le groupe aromatique pouvant être substitué par un ou plusieurs, groupes alkyle inférieurs, alcoxy, nitro, amino et/ou un ou plusieurs atomes d'halogène, identiques ou différents;

$R^3$ peut représenter l'hydrogène, alkyle en $C_1$-$C_6$;

$R^4$ peut représenter alkyle en $C_1$-$C_8$;

$R^5$ peut représenter alkyle en $C_1$-$C_8$;

$R^6$ peut représenter alkyle en $C_1$-$C_8$, un reste aromatique de 6 à 10 atomes de carbone, même dans des assemblages, le groupe aromatique pouvant être substitué par un ou plusieurs groupes alkyle inférieurs, alcoxy, nitro, amino et/ou un ou plusieurs atomes d'halogène, identiques ou différents;

$R^7$ et $R^8$ peuvent représenter indépendamment l'un de l'autre l'hydrogène, alkyle en $C_1$-$C_8$, halogène,
-OH, alcoxy en $C_1$-$C_8$,
un reste O-benzoyle ou O-alklcarbonyle ayant un reste alkyle inférieur linéaire ou ramifié de 1 à 6 atomes de carbone, le reste alkyle pouvant éventuellement être substitué par un ou plusieurs atomes d'halogène qui peuvent être identiques ou différents,
-CN, $-NO_2$,
$NH_2$, -NH(alkyle en $C_1$-$C_8$),

-N(alkyle en $C_1$-$C_8$)$_2$,
les restes alkyle pouvant être identiques ou différents,
-NH-acyle ou -N-acyl-(alkyle en $C_1$-$C_8$), où acyle peut représenter benzoyle ou un reste alkylcarbonyle ayant un reste alkyle inférieur linéaire ou ramifié de 1 à 6 atomes de carbone, le reste alkyle pouvant éventuellement être substitué par un ou plusieurs atomes d'halogène qui peuvent être identiques ou différents,

leurs stéréoisomères et leurs sels d'addition d'acide, à condition que, dans le cas où

X représente l'oxygène;
$R^1$ représente alkyle en $C_1$-$C_3$, alcényle en $C_3$, alcynyle en $C_3$;
$R^2$ représente l'hydrogène, alkyle en $C_1$-$C_4$, alcényle en $C_3$ et $C_4$;
$R^3$ représente l'hydrogène, alkyle en $C_1$-$C_3$;
$R^4$ représente méthyle;
$R^5$ représente méthyle;
$R^6$ représente alkyle en $C_1$-$C_4$ ou phényle

et l'un des deux substituants $R^7$ et $R^8$ représente l'hydrogène, le substituant $R^7$ ou $R^8$ restant ne puisse pas représenter en position 2' l'hydrogène, hydroxyle, alcoxy en $C_1$-$C_3$, O-acyle.

**2.** Benzomorphanes selon la revendication 1
où

X peut représenter l'oxygène, le soufre;
$R^1$ peut représenter méthyle, éthyle, propyle, isopropyle, phényle;
$R^2$ peut représenter méthyle, éthyle, propyle, isopropyle, allyle, propargyle, phényle, benzyle;
$R^3$ peut représenter l'hydrogène, alkyle en $C_1$-$C_4$;
$R^4$ peut représenter méthyle, éthyle, propyle, isopropyle;
$R^5$ peut représenter méthyle, éthyle, propyle, isopropyle;
$R^6$ peut représenter méthyle, éthyle, propyle, isopropyle, phényle;
$R^7$ peut représenter fluor, chlore, hydroxyle, alkyle inférieur, alcoxy en $C_1$-$C_3$,
un reste O-benzoyle ou O-alkylcarbonyle ayant un reste alkyle inférieur linéaire ou ramifié de 1 à 6 atomes de carbone, le reste alkyle pouvant éventuellement être substitué par un ou plusieurs atomes d'halogène qui peuvent être identiques ou différents;
$R^8$ peut représenter l'hydrogène, alkyle inférieur, hydroxyle ou alcoxy en $C_1$-$C_8$

leurs stéréoisomères et leurs sels d'addition d'acide.

**3.** Benzomorphanes selon la revendication 1
où

X peut représenter l'oxygène;
$R^1$ peut représenter méthyle, éthyle;
$R^2$ peut représenter méthyle, éthyle;
$R^3$ peut représenter l'hydrogène;
$R^4$ peut représenter méthyle, éthyle;
$R^5$ peut représenter méthyle, éthyle;
$R^6$ peut représenter méthyle, éthyle;
$R^7$ peut représenter hydroxyle, méthyle, méthoxy,
un reste O-benzoyle ou O-alkylcarbonyle ayant un reste alkyle inférieur linéaire ou ramifié de 1 à 6 atomes de carbone, le reste alkyle pouvant éventuellement être substitué par un ou plusieurs atomes d'halogène qui peuvent être identiques ou différents;
$R^8$ peut représenter l'hydrogène, méthyle, éthyle, hydroxyle ou alcoxy en $C_1$-$C_3$

leurs stéréoisomères et leurs sels d'addition d'acide.

**4.** Benzomorphanes selon la revendication 1
où

| | |
|---|---|
| X | peut représenter l'oxygène; |
| $R^1$ | peut représenter méthyle; |
| $R^2$ | peut représenter méthyle; |
| $R^3$ | peut représenter l'hydrogène; |
| $R^4$ | peut représenter méthyle; |
| $R^5$ | peut représenter méthyle; |
| $R^6$ | peut représenter méthyle; |
| $R^7$ | peut représenter hydroxyle, méthyle, méthoxy, acétoxy; |
| $R^8$ | peut représenter l'hydrogène, méthyle, hydroxyle ou alcoxy en $C_1$-$C_3$ |

leurs stéréoisomères et leurs sels d'addition d'acide.

5. Benzomorphanes selon la revendication 1 où

| | |
|---|---|
| X | peut représenter l'oxygène; |
| $R^1$ | peut représenter méthyle; |
| $R^2$ | peut représenter méthyle; |
| $R^3$ | peut représenter l'hydrogène; |
| $R^4$ | peut représenter méthyle; |
| $R^5$ | peut représenter méthyle; |
| $R^6$ | peut représenter méthyle; |
| $R^7$ | peut représenter hydroxyle, méthyle, méthoxy, acétoxy; |
| $R^8$ | peut représenter l'hydrogène, méthyle, hydroxyle, méthoxy ou éthoxy |

et où le substituant $R^7$ se trouve en position 3' et le substituant $R^8$ se trouve en position 2' et l'atome de carbone en 2" présente une configuration R ainsi que leurs sels d'addition d'acide.

6. (-)-(1R,5S,2"R)-3'-hydroxy-2-(2-méthoxypropyl)-5,9,9-triméthyl-6,7-benzomorphane et ses sels d'addition d'acide.

7. Préparation pharmaceutique caractérisée par la teneur en un composé selon l'une des revendications 1 à 6 et en ses sels d'addition d'acide outre des adjuvants et supports habituels.

8. Utilisation de composés selon l'une des revendications 1 à 6 comme médicaments.

9. Utilisation de composés de formule générale I où

| | |
|---|---|
| X | peut représenter l'oxygène ou le soufre; |
| $R^1$ | peut représenter alkyle en $C_1$-$C_8$, alcényle en $C_3$-$C_6$, alcynyle en $C_3$-$C_6$, un reste aromatique de 6 à 10 atomes de carbone, même dans des assemblages, le groupe aromatique pouvant être substitué par un ou plusieurs groupes alkyle inférieurs, alcoxy, nitro, amino et/ou un ou plusieurs atomes d'halogène, identiques ou différents; |
| $R^2$ | peut représenter l'hydrogène, alkyle en $C_1$-$C_8$, alcényle en $C_3$-$C_6$,<br>alcynyle en $C_3$-$C_6$, un reste aromatique de 6 à 10 atomes de carbone, même dans des assemblages, le groupe aromatique pouvant être substitué par un ou plusieurs groupes alkyle inférieurs, alcoxy, nitro, amino et/ou un ou plusieurs atomes d'halogène, identiques ou différents, un reste aryle de 7 à 14 atomes de carbone lié par une chaîne alkylène, le groupe aromatique pouvant être substitué par un ou plusieurs groupes alkyle inférieurs, alcoxy, nitro, amino et/ou un ou plusieurs atomes d'halogène, identiques ou différents; |
| $R^3$ | peut représenter l'hydrogène, alkyle en $C_1$-$C_6$; |
| $R^4$ | peut représenter alkyle en $C_1$-$C_8$; |
| $R^5$ | peut représenter alkyle en $C_1$-$C_8$; |
| $R^6$ | peut représenter alkyle en $C_1$-$C_8$, un reste aromatique de 6 à 10 atomes de carbone, même dans des assemblages, le groupe aromatique pouvant être substitué par un ou plusieurs groupes alkyle inférieurs, alcoxy, nitro, amino et/ou un ou plusieurs atomes d'halogène, identiques ou différents; |
| $R^7$ et $R^8$ | peuvent représenter indépendamment l'un de l'autre l'hydrogène, alkyle en $C_1$-$C_8$, halogène, -OH, alcoxy en $C_1$-$C_8$,<br>un reste O-benzoyle ou O-alkylcarbonyle ayant un reste alkyle inférieur linéaire ou ramifié de 1 à 6 atomes de carbone, le reste alkyle pouvant éventuellement être substitué par un ou plu- |

sieurs atomes d'halogène qui peuvent être identiques ou différents,
-CN, $-NO_2$,
$NH_2$, -NH(alkyle en $C_1$-$C_8$),
-N(alkyle en $C_1$-$C_8$)$_2$,
les restes alkyle pouvant être identiques ou différents,
-NH-acyle ou -N-acyl-(alkyle en $C_1$-$C_8$), où acyle peut représenter benzoyle ou un reste alkylcarbonyle ayant un reste alkyle inférieur linéaire ou ramifié de 1 à 6 atomes de carbone, le reste alkyle pouvant éventuellement être substitué par un ou plusieurs atomes d'halogène qui peuvent être identiques ou différents,

de leurs stéréoisomères et de leurs sels d'addition d'acide pour la préparation d'un médicament pour le traitement thérapeutique des ischémies cérébrales de genèse variable, de l'épilepsie et des maladies neurodégénératives.

**10.** Procédé de préparation de composés de formule générale I

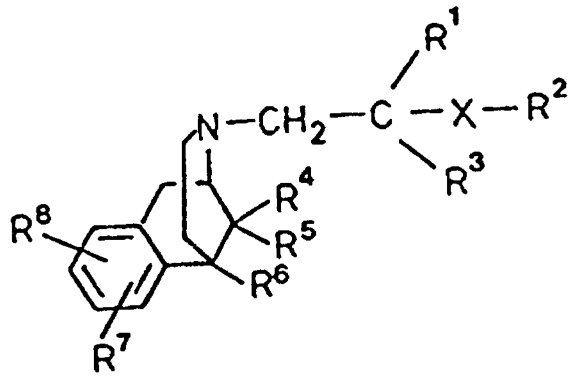

(I)

dans laquelle les substituants $R^1$ à $R^8$ ont la signification indiquée dans la revendication 1, caractérisé en ce que

a) on fait réagir les benzomorphanes de formule générale II dans laquelle $R^4$, $R^5$ et $R^6$ ont la signification indiquée et $R^9$ et $R^{10}$ ont la signification de $R^7$ et de $R^8$ ou représentent des substituants qui peuvent être convertis en ceux-ci de manière connue, de manière connue en soi dans des solvants inertes, éventuellement en présence d'un agent fixant les acides, avec des dérivés d'acide carboxylique de formule générale 4 où Y représente un groupe partant, remplaçable par un groupe amino secondaire, du groupe constitué par halogène, hydroxyle, OC(O)alkyle, O-acyle, $OSO_2$alkyle et $OSO_2$aryle et $R^1$, $R^2$ et $R^3$ ainsi que X possèdent la signification indiquée,

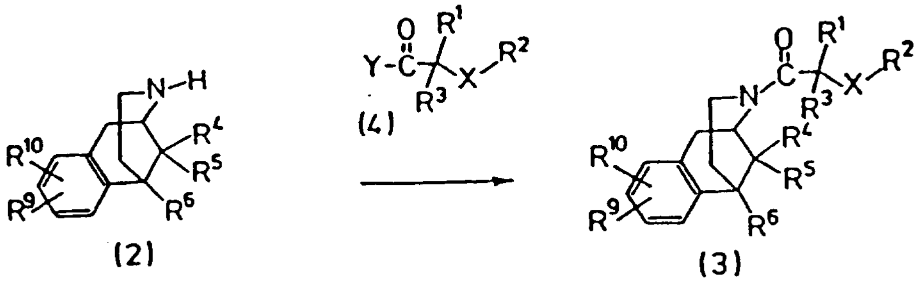

(4)

(2) (3)

puis on réduit l'amide d'acide 3 résultant dans un solvant inerte de manière connue en soi en l'amine de formule générale 6

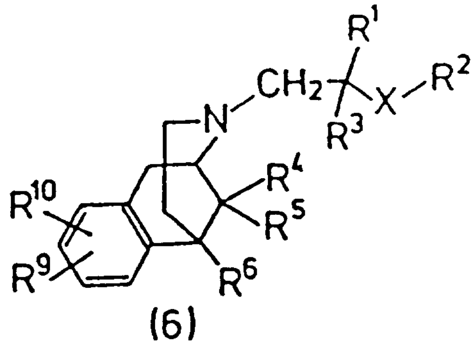

(6)

et on convertit éventuellement les substituants $R^9$ et $R^{10}$ en $R^7$ et $R^8$ et on isole le produit (1) à partir du mélange réactionnel

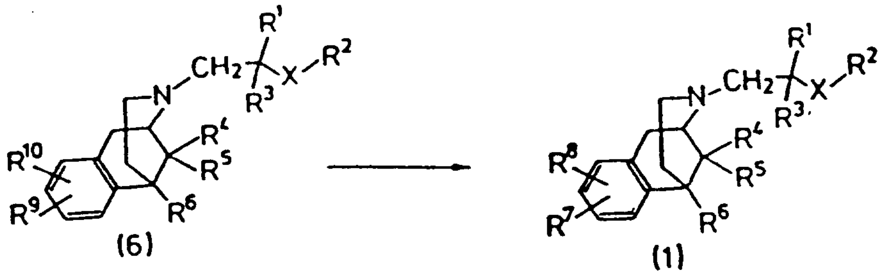

(6) (1)

ou

b) on convertit des benzomorphanes de formule générale 2

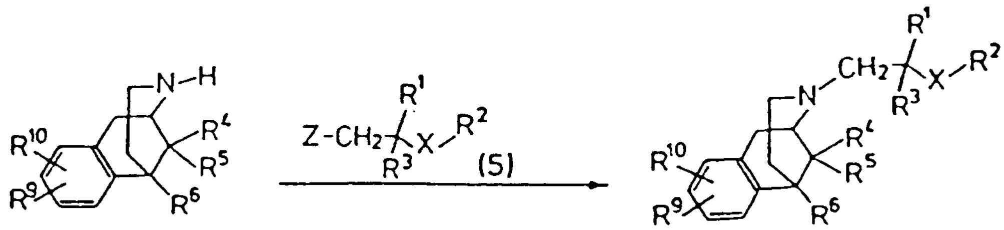


(2) (6)

dans laquelle $R^4$, $R^5$ et $R^6$ ont la signification indiquée et $R^9$ et $R^{10}$ ont la signification de $R^7$ et de $R^8$ ou représentent des substituants qui peuvent être convertis en ceux-ci de manière connue, de manière connue en soi dans des solvants inertes, éventuellement en présence d'un agent fixant les acides, avec un agent alkylant de formule générale 5 où Z représente un groupe partant, remplaçable par un groupe amino secondaire, du groupe constitué par les halogènes, comme Cl, Br et I, par exemple, ou les arylsulfonates $OSO_2$aryle comme tosylate, par exemple, ou les alkylsulfonates comme méthanesulfonate ou halogénométhanesulfonate par exemple, ou un groupe sulfate, en l'amine tertiaire de type 6

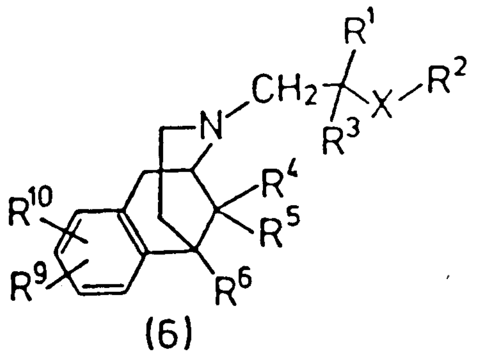

(6)

et on convertit éventuellement les substituants $R^9$ et $R^{10}$ en $R^7$ et $R^8$ et on isole le produit de la réaction.

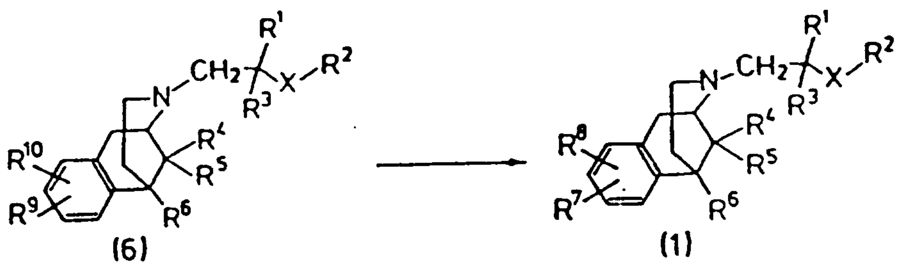

(6) (1)